# FASCICULE DE BREVET EUROPEEN

(11) **EP 2 331 510 B1**
(45) Date de publication et mention de la délivrance du brevet: **06.11.2013**
(21) Numéro de dépôt: 09748424.0
(22) Date de dépôt: 22.09.2009
(51) Int. Cl.: C07D 239/36, C07D 239/47, C07D 239/54, C07D 403/12, C07D 473/04, C07D 473/18, C07D 473/30

(54) **SUBSTRATS D' O6-ALKYLGUANINE-ADN ALKYLTRANSFERASE (AGT) ET LEUR UTILISATION DANS DES METHODES DE MARQUAGE**
O6-ALKYLGUANIN-DNA-ALKYLTRANSFERASE (AGT) SUBSTRATE UND DEREN VERWENDUNG IN VERFAHREN ZUR MARQUIERUNG
SUBSTRATES OF O6-ALKYLGUANINE-DNA ALKYLTRANSFERASE (AGT) AND THEIR USE IN METHOD OF LABELLING

(30) Priorité: 23.09.2008 FR 0856409
(43) Date de publication de la demande: 15.06.2011
(73) Titulaire: Ion Beam Applications S.A., 1348 Louvain-la-Neuve (BE)
(72) Inventeur: BOURRIER, Emmanuel, F-30200 Bagnols sur Ceze (FR); LAGET, Michel, F-30130 Carsan (FR); LAMARQUE, Laurent, F-30290 Saint Victor la Coste (FR); TINEL, Norbert, F-34400 Lunel-Viel (FR); TRINQUET, Eric, F-30130 Pont Saint Esprit (FR); BAZIN, Hervé, F-30400 Villeneuve les Avignon (FR)
(74) Mandataire: Nevant, Marc
(86) Numéro de dépôt international: PCT/FR2009/051787
(87) Numéro de publication internationale: WO 2010/034931

(56) Documents cités:
- EP-A1- 1 882 688
- WO-A-02/083937
- WO-A-2004/031405
- WO-A-2006/114409

## Description

Les effets mutagènes et cancérigènes des composés électrophiles comme la N-méthyl-N-nitrosourée sont pour l'essentiel dus à l'O⁶-alkylation des résidus guanine de l'ADN. Des mécanismes de protection contre cette alkylation de l'ADN existent chez les mammifères et les bactéries, et l'*O*⁶-alkylguanine-ADN alkyltransférase (AGT) en particulier est capable de réparer ces lésions. L'AGT transfère le groupe alkyle en position O⁶ de la guanine alkylée sur un groupe thiol d'une de ses propres cystéines, ce qui entraîne une alkylation irréversible de l'AGT. Ce transfert de groupe s'opère par une substitution nucléophile de type 2, ce qui explique pourquoi le transfert de groupes benzyle est également possible, en plus du transfert de groupes alkyle.

Dans la mesure où la surexpression de l'AGT dans les cellules tumorales est la raison principale de la résistance à des médicaments dont l'action est basée sur l'alkylation de l'ADN, comme la procarbazine, la dacarbazine, le temozolomide et la bis-2-chloroéthyl-N-nitrosourée, l'utilisation d'inhibiteurs de l'AGT a été proposée comme agent sensibilisant dans le cadre de traitements chimiothérapeutiques (Pegg et al., Prog Nucleic Acid Res Mol Biol 51:167-223, 1 995). Le brevet US 5 691 307 décrit des dérivés d'O⁶-benzylguanine portant divers substituants sur le groupe benzyle, ainsi que leur utilisation pour diminuer les niveaux d'AGT dans les cellules tumorales et ainsi augmenter leur sensibilité aux traitements basés sur l'emploi d'agents alkylants. De même, la demande de brevet WO 97/20843 divulgue d'autres composés pour cette utilisation, sous la forme de dérivés d'O⁶-benzyl et O⁶-hétérooarylméthyl-pyrimidine.

Le brevet DE 199 03 895 divulgue un dosage de l'AGT basé sur la réaction entre des dérivés biotinilés d'O⁶-alkylguanine et l'AGT, conduisant à la biotynilation de l'AGT, ce qui permet sa séparation sur des plaques recouvertes de streptavidine et sa détection, par exemple par un dosage ELISA. Cette technique est proposée pour suivre les niveaux d'AGT dans des tissus cancéreux, ainsi que pour mettre en évidence des inhibiteurs de l'AGT.

WO 01/85221 propose d'utiliser des dérivés radioactifs fluorés ou iodés d'O⁶-benzylguanine pour détecter l'AGT.

Damoiseaux et al. ChemBiochem. 4:285-287, 2001 divulguent des dérivés d'O⁶-alkylguanine incorporés dans des oligodeoxyribonuctéotides et leur utilisation comme sondes destinées à marquer l'AGT, encore une fois dans le but de mesurer les niveaux d'expression de cette enzyme dans des cellules cancéreuses afin d'améliorer l'efficacité de traitements de chimiothérapie.

La demande WO 02/083937 décrit une méthode pour détecter et manipuler une protéine d'intérêt fusionnée à l'AGT, consistant à mettre en contact cette protéine de fusion avec un substrat d'AGT portant un marqueur, ce qui permet après transfert de la molécule d'intérêt sur l'AGT, de détecter ou de manipuler cette protéine d'intérêt. Cette demande divulgue également diverses protéines de fusion comportant l'AGT, les principes généraux relatifs à la structure des substrats d'AGT, ainsi qu'une variété de marqueurs et de méthodes pour détecter ledit marqueur.

La demande WO 2004/031404 décrit des protéines de fusion particulières comprenant l'AGT et une protéine d'intérêt susceptible d'être utilisées dans la méthode décrite précédemment, des protéines de fusion marquées obtenues par cette méthode et une méthode utilisant lesdites protéines de fusion.

Les demandes WO 2004/031405 et WO 2005/085470 décrivent des substrats d'AGT particuliers portant un ou plusieurs marqueurs ainsi que leurs procédés de fabrication, ces substrats étant susceptibles d'être utilisés dans la méthode décrite dans WO 02/083937.

Des exemples de substrats d'AGT sont les dérivés de benzylguanine et benzylcytosine ci-après :

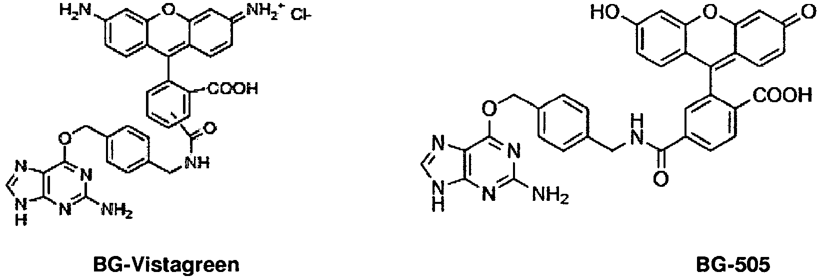

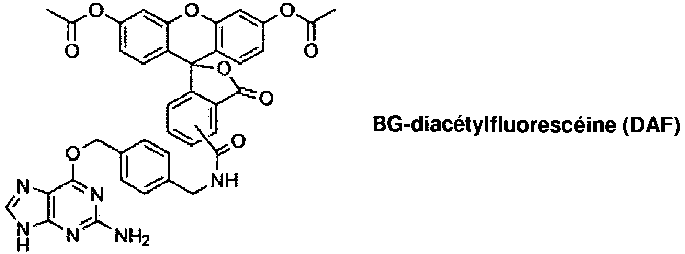

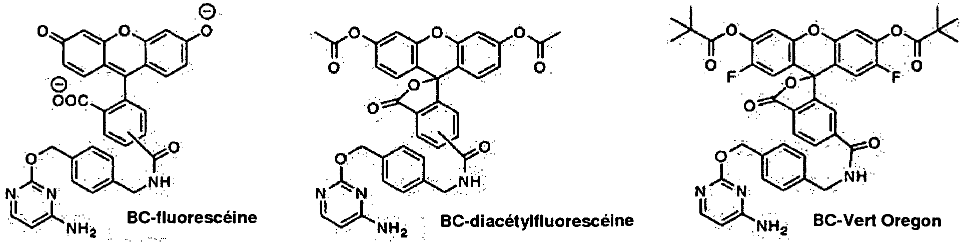

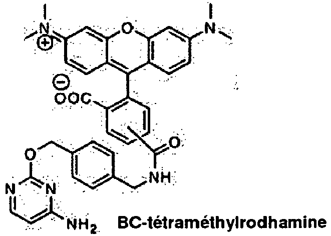

WO 2006/114409 décrit des composés de formule 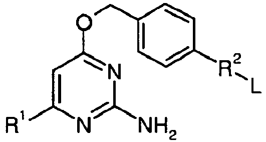 qui sont présentés comme des substrats de l'*O*⁶-alkylguanine-ADN alkyltransférase.

### DESCRIPTION :

L'invention concerne de nouveaux substrats de l'enzyme AGT ou de ses mutants, constitués d'un benzylnucléotide lié de manière covalente à une molécule d'intérêt via un bras de liaison comportant un cycle à 5 ou 6 atomes. Ces substrats sont particulièrement adaptés pour la mise en oeuvre de la technique de détection et de manipulation de protéines d'intérêt fusionnées à l'AGT, telle que décrite dans la demande de brevet WO 02/083937. Cette technique est actuellement exploitée par la société Covalys à l'aide des enzymes connues sous les dénominations commerciales « SNAP-tag » et « CLIP-tag ».

Par AGT (*O⁶-alkylguanine ADN alkyltransférase),* on entend ici l'AGT sauvage quelle que soit son origine (humaine, de souris, de rat etc..) et ses mutants fonctionnels capables de transférer une molécule d'intérêt conjuguée à un substrat benzylnucléotide sur une fonction thiol de l'enzyme. Les enzymes SNAP-tag (Juillerat et al.,Chemistry & biology, Vol. 10, 313-317 Avril 2003) et CLIP-tag (Gautier et al, Chemistry et Biology, 15, 128-136, février 2008) commercialisées par la société Covalys sont des mutants de l'AGT humaine dont les substrats sont respectivement, l'O⁶-benzylguanine (ci-après dénommée BG) et l'O²-benzylcytosine (ci-après dénommée BC). L'enzyme N-AGT (Gronemeyer et al., (Protein engineering, design & selection, vol. 19, no 7, pp 309-3016, 2006) est un autre mutant de cette enzyme dont la réactivité avec l'O⁶-benzylguanine est meilleure que celle de l'enzyme SNAP-tag.

De manière tout à fait inattendue, la réactivité des substrats de l'AGT selon l'invention avec cette enzyme AGT, et en particulier avec ses mutants SNAP-tag, CLIP-tag et N-AGT est bien meilleure que celle des substrats classiques de ces enzymes, ce qui se traduit par une cinétique de marquage de l'AGT beaucoup plus rapide. Les substrats d'AGT selon l'invention permettent ainsi d'étendre l'application de l'AGT et ses mutants à des domaines où les concentrations en substrats et les temps de marquage étaient jusqu'à présent limitant (criblage à haut débit, diagnostic, imagerie médicale,...).

Les substrats d'AGT selon l'invention sont les composés de formule (I') : 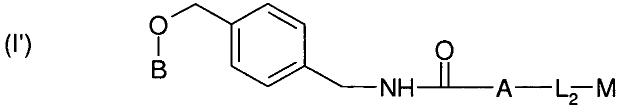 dans laquelle :
B représente un groupe de formule (II') ou (III') suivante : 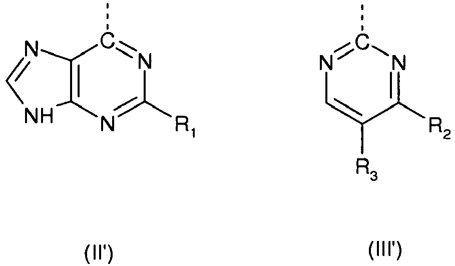 dans lesquelles les lignes pointillées représentent les liaisons avec l'oxygène de la formule (I'),
R₁ est choisi parmi : H, NH₂, un groupe OH ou un groupe oxo,
R₂ est choisi parmi : NH₂, OH ou oxo,
R₃ est choisi parmi : H ou un groupe CH₃,
L₂ est choisi parmi les groupes : 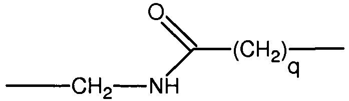 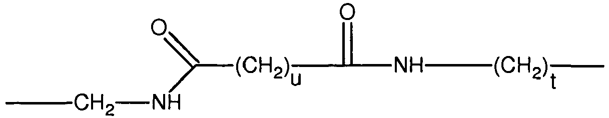 dans lesquels q, u, t sont des nombres entiers compris entre 1 et 10 ;
M est une molécule d'intérêt choisie parmi un composé fluorescent et un fluorophore, ou un groupe réactif tel que défini ci-après ;
A est choisi parmi les groupes divalents suivants :

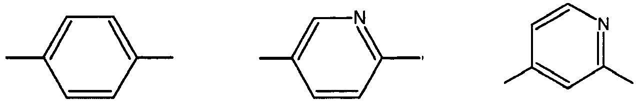

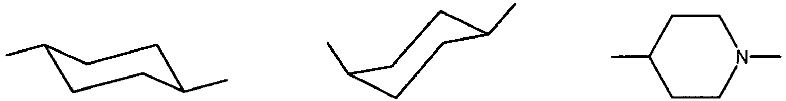

Dans le cas où R₁ est un groupe OH, la forme majoritaire du composé de formule (II') est le tautomère dans lequel R₁ est un groupe oxo et la liaison entre le carbone en position 2 et l'azote en position 3 de la purine est une liaison simple.

Dans le cas ou R₂ est un groupe OH, la forme majoritaire du composé de formule (III') est le tautomère dans lequel R₂ est un groupe oxo et la liaison entre le carbone en position 4 et l'azote en position 3 de la pyrimidine est une liaison simple.

### GROUPE A

Le groupe A est un radical divalent choisi parmi les groupes suivants :

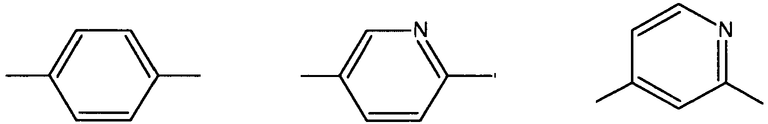

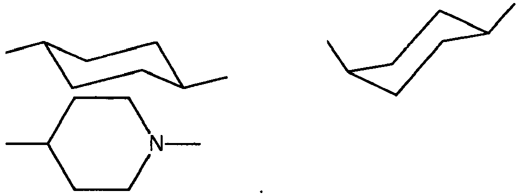

### BRAS DE LIAISON

Le bras de liaison L₂ relie la molécule d'intérêt au groupe A. La structure de ce bras de liaison est déterminée par la voie de synthèse chimique choisie pour conjuguer le groupe A avec la molécule d'intérêt.

L₂ est choisi parmi les groupes suivants : 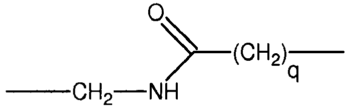 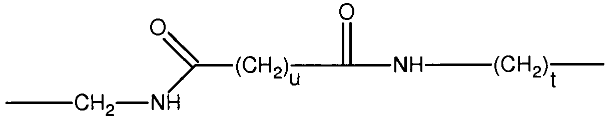 dans lesquels q, u, t sont des nombres entiers compris entre 1 et 10.

### MOLÉCULE D'INTÉRÊT

Comme mentionné plus haut, les substrats d'AGT selon l'invention sont particulièrement destinés à être utilisés pour marquer une enzyme AGT avec une molécule d'intérêt. Lorsque cette enzyme est exprimée sous forme de protéine de fusion avec une protéine d'intérêt, il va alors être possible de marquer ladite protéine avec la molécule d'intérêt porté par le substrat d'AGT.

La molécule d'intérêt peut être de nature et de fonction très diverses comme le montre la littérature sur la technologie SNAP-tag, et dépend de l'application envisagée.

Dans un mode de réalisation, la molécule d'intérêt est un marqueur susceptible de générer ou de moduler un signal mesurable, en particulier un composé luminescent ou un fluorophore.

Dans ce cas, et à titre d'exemple non-limitatif, la molécule d'intérêt peut être choisie parmi les composés luminescents ou fluorescents suivants :
- *Les fluorophores organiques,* tels que les cyanines et leurs dérivés, (en particulier celles connues sous les dénominations commerciales DY647, Cy5, DY490), la fluorescéine et ses dérivés, la coumarine et ses dérivés, la rhodamine et ses dérivés, la carbopyronine et ses dérivés, l'oxazine et ses dérivés, les AlexaFluors, le crystal violet et ses dérivés, les perylènebisimides et leurs dérivés, les squaraines, les BODIPYs, le NBD (nitrobenzoxadiazole) et ses dérivés, le DABCYL (acide 4-((4-(diméthylamino) phényl) azo) benzoique) et ses dérivés ;
- *Les fluorophores protéiques,* tels que la GFP et ses variants, les protéines fluorescentes extraites de coraux, les phycobiliprotéines, telles que la B-phycoérythrine, la R-phycoérythrine, la C-phycocyanine, les allophycocyanines, en particulier celle connue sous la dénomination XL665 ;
- *Les complexes de lanthanides fluorescents,* tels que les cryptates de lanthanides, les chélates de lanthanides (en particulier les chélates et cryptates d'europium, de terbium, de samarium, de dysprosium, de néodymium).

La molécule d'intérêt peut également être un accepteur d'énergie non-fluorescent et par exemple peut être choisie parmi les produits connus sous les dénominations commerciales ci-après : les produits QXL de la société Anaspec et en particulier les produits QXL 570, QXL 610, QXL 670 et QXL 680 ; les produits DYQ660 et DYQ661 de la société Dyomics ; les produits QSY7, QSY9 et QSY21 de la société Invitrogen ; les produits ATTO540Q, ATTO580Q, ATTO621Q de la société ATTO-TEC

De manière préférée, la molécule d'intérêt est choisie parmi les produits connus sous les dénominations commerciales ci-après : DY647; d2 ; ATTO647N; ATTO465; 5-ex-fluorescéine ; 5,6-carboxyfluorescéine ; le cryptate d'europium Py-bipy ; le complexe de terbium DOTA-TATP ; le cryptate d'europium py-bipy-tétraacide, le cryptate d'europium trisbipy, le cryptate de terbium Tb(KR), le cryptate d'europium trisbipy-pentaacide, le cryptate d'europium Py-biTATP.

De manière encore plus préférée, le fluorophore est choisi parmi les fluorophores suivants : DY647, ATTO647N, 5-ex-fluorescéine, Tb(KR) et ATTO465.

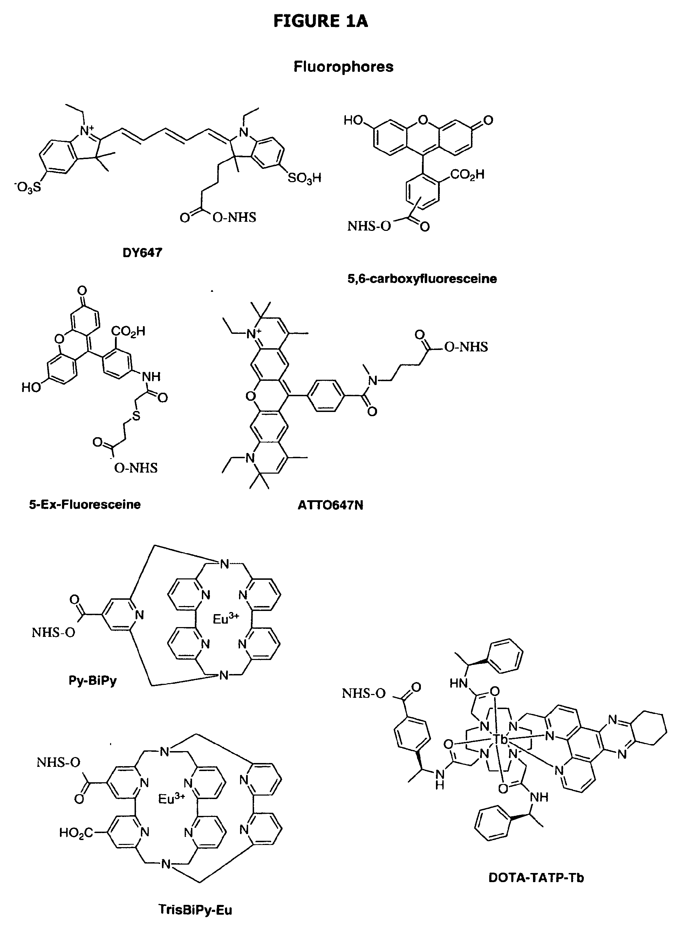

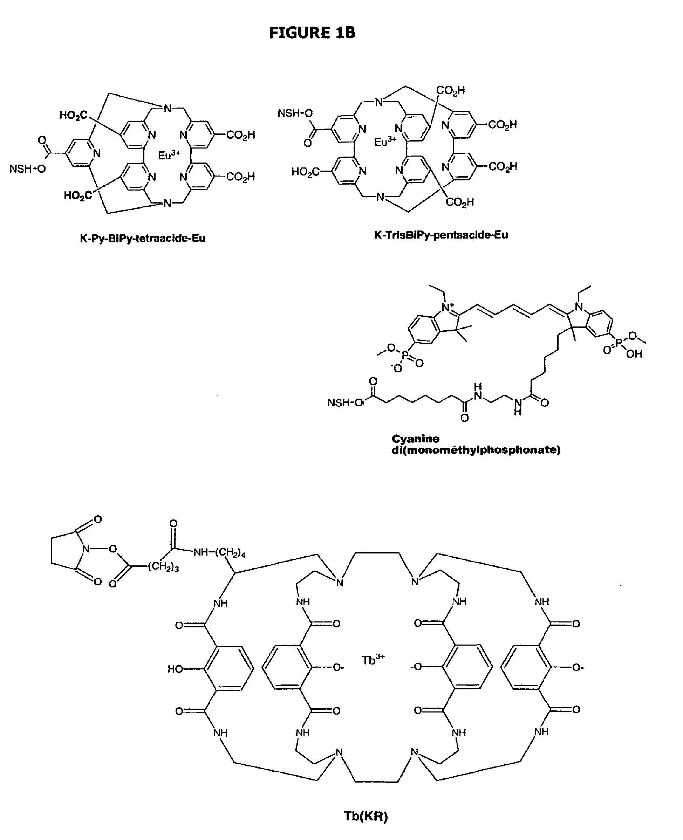

Les figures 1 A et 1 B donnent les formules de quelques fluorophores fonctionnalisés (porteurs d'un groupe réactif tel qu'un groupe NHS) dont la synthèse est connue ou bien qui sont disponibles commercialement, et pouvant être utilisés comme intermédiaires pour fabriquer les composés selon l'invention.

De nombreux fluorophores porteurs d'un groupe réactif facilitant leur couplage à d'autres molécules sont disponibles commercialement. Par exemple, les fluorophores suivants sont commerciaux : DY647-NHS, DY490-NHS (Dyomics), 5,6-carboxyfluorescéine, 5-Ex-fluorescéine (Invitrogen), ATTO647N-NHS et ATTO465-NHS (ATTO TEC), cryptate d'europium trisbipyridine (Cisbio)

La synthèse de cyanines portant divers substituants est largement décrite dans la littérature. On peut citer en particulier les cyanines et leurs procédés de synthèse décrits dans les brevets américains US 5,268,486, US 5,627,027 et le brevet Européen EP 1,322,770.

Les complexes de terre rare sont des composés connus qui sont décrits par exemple dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. D'autres chélates sont composés d'un ligand nonadenté tel que la terpyridine (EP 403 593, US 5 324 825, US 5 202 423, US 5 316 909). La terre rare est de préférence l'europium ou le terbium.

Les cryptates de terres rares et leur préparation sont quant à eux décrits notamment dans les brevets EP 0 180 492, EP 0 601 113 et la demande WO 01/96 877.

La synthèse du fluorophore Tb(KR)-NHS est décrite dans la demande de brevet WO 2008/063721.

Le DOTA-TATP-Tb et sa préparation sont décrits dans l'article de R. A. Poole et al., dans Org. BiomolChem., 2005, 3, 1013-1024.

### GROUPE REACTIF

Typiquement, le groupe réactif est un groupe, respectivement, électrophile ou nucléophile qui peut former une liaison covalente lorsqu'il est mis en présence d'un groupe nucléophile ou électrophile approprié. La réaction de conjugaison entre un composé selon l'invention comportant un groupe réactif et une molécule d'intérêt portant un groupe fonctionnel entraîne la formation d'une liaison covalente comportant un ou plusieurs atomes du groupe réactif.

Le groupement réactif est un groupe dérivé d'un des composés ci-après : un acrylamide, une amine activée (par exemple une cadavérine ou une éthylènediamine), un ester activé, un aldéhyde, un halogénure d'alkyle, un anhydride, une aniline, un azide, une aziridine, un acide carboxylique, un diazoalcane, une haloacétamide, une halotriazine, telle que la monochlorotriazine, la dichlorotriazine, une hydrazine (y compris les hydrazides), un imido ester, un isocyanate, un isothiocyanate, un maléimide, un halogénure de sulfonyle, ou un thiol, une cétone, une amine, un halogénure d'acide, un hydroxysuccinimidyl ester, un hydroxysulfosuccinimidyl ester, un azidonitrophényl, un azidophényl, une 3-(2-pyridyl dithio)-propionamide, glyoxal et en particulier les groupes de formule : 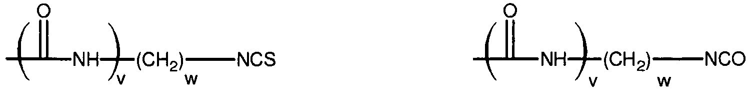 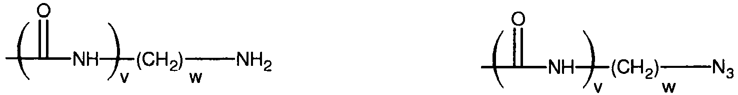 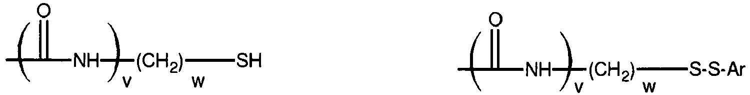 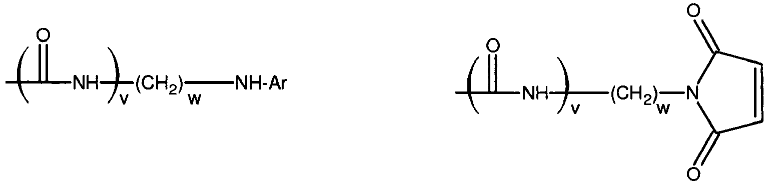 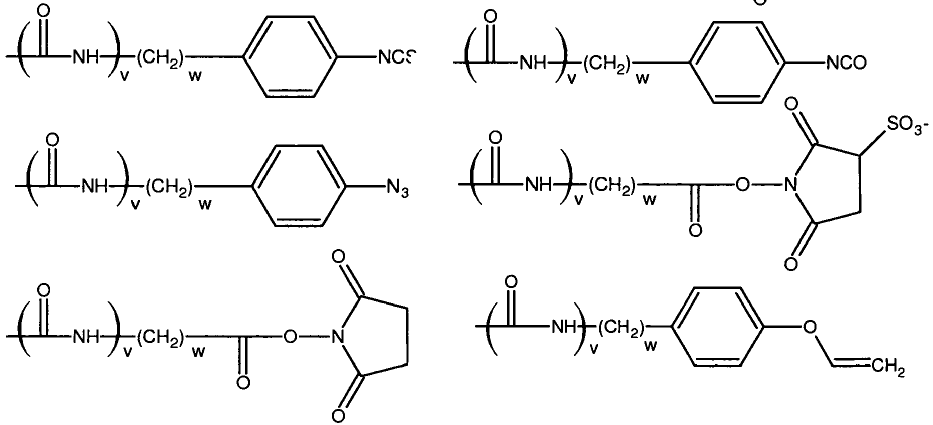 dans lesquels w varie de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène.

De manière préférée, le groupe réactif est un acide carboxylique, une amine, un succinimidyl ester d'acide carboxylique, un haloacétamide, une hydrazine, un isothiocyanate, un groupe maléimide, une amine aliphatique.

Les composés de formule (I) selon l'invention dans lesquels le groupe M est un groupe réactif sont des intermédiaires de synthèse utiles pour l'obtention des composés de formule (I) dans laquelle M est une molécule d'intérêt.

### COMPOSES PREFERES :

Les composés de formule (I') comportant un groupe de formule (III') et dont R₂ est un groupe NH₂ et R₃ est H, ainsi que ceux de formule (I') comportant un groupe de formule (II') et dont R₂ est un groupe NH₂ constituent des sous-familles préférées.

La formule (I') englobe les composés de formules suivantes, dans lesquelles M et L2 sont tels que définis précédemment. Pour chaque formule, on indique ci-après la signification du groupe A.

A représente un groupe

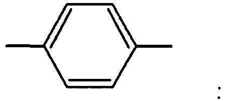

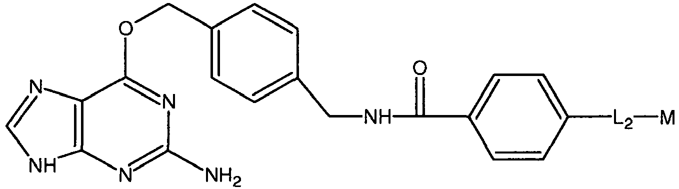

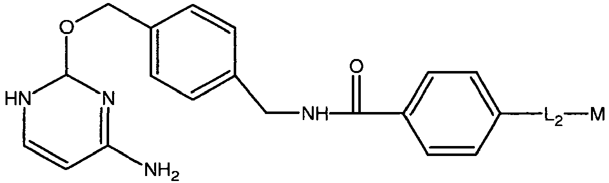

A représente un groupe

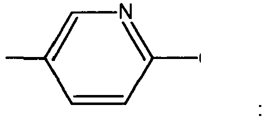

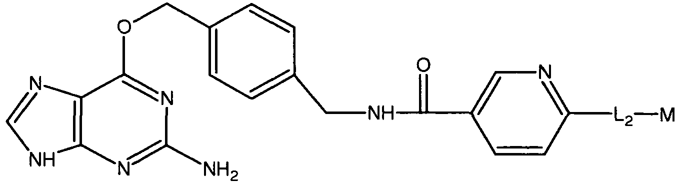

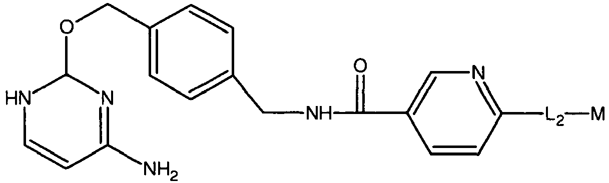

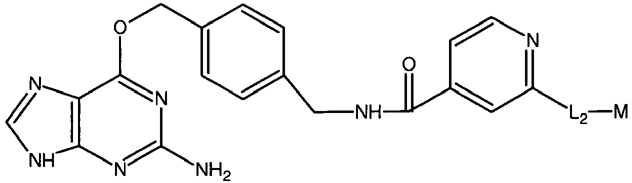

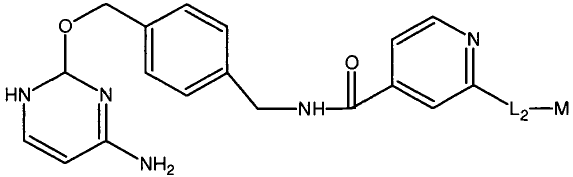

A représente un groupe

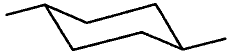

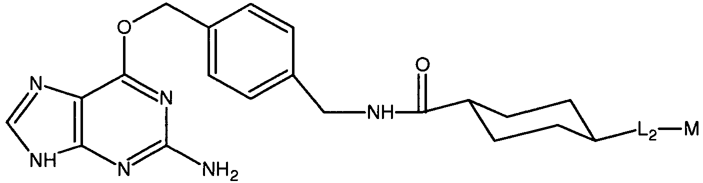

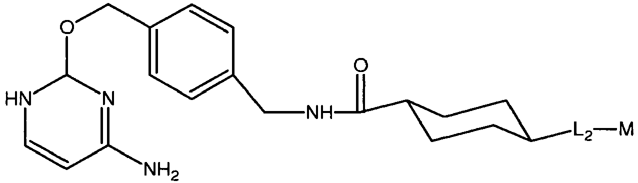

A représente un groupe

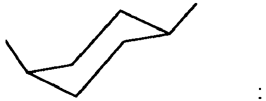

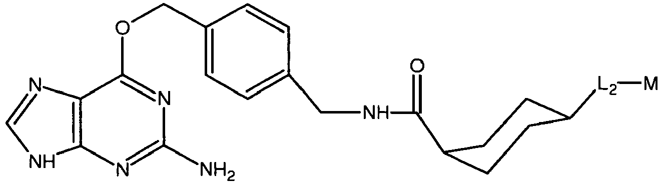

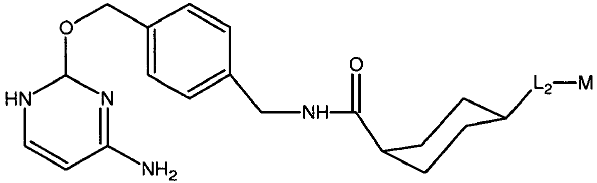

A représente un groupe

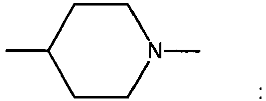

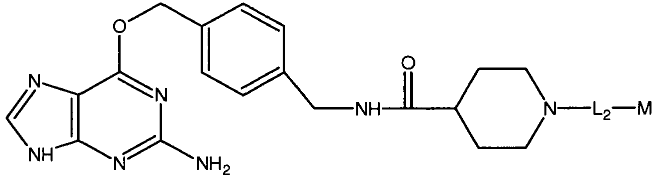

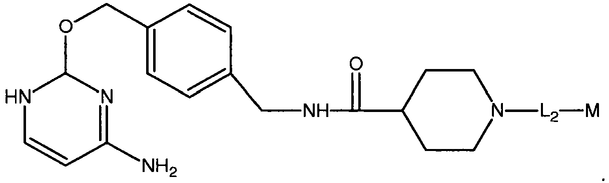

Dans la suite de la présente description, on utilisera les abréviations ci-après :
DCC : Dicyclohexylcarbodiimide
DTT : Dithiothréitol
THF : Tetrahydrofurane
DMF : Diméthylformamide anhydre
TBTU : O-(benzotriazol-1-yl)-1,1,3,3-tetramethyluronium tetrafluoroborate
TEAB : triéthylammonium bicarbonate
SPDP : N-Succinimidyl 3-[2-pyridyldithio]propionate
TCEP : hydrochlorure de tris[2-carboxéthyl]phosphine
DMSO : diméthylsulfoxyde anhydre
DIPEA : diisopropyléthylamine
TFA : acide trifluoroacétique
BG : O⁶-benzylguanine
BC : O²-benzylcytosine
Milieu DMEM : "Dulbecco's Modified Eagle's Medium" Milieu de culture cellulaire, disponible commercialement et utilisé dans de nombreuses applications.
SVF : sérum de veau fétal
Ester de NHS : Ester de N-hydroxysuccinimide
FRB : domaine de liaison de la protéine FRAP avec le complexe FKBP-rapamycine (en langue anglaise "FKBP-rapamycin binding domain of FRAP")
FRAP : protéine se liant au complexe FKBP-rapamycine (en langue anglaise, "FKBP-rapamycin binding protein")
FKBP : protéine se liant à la protéine FK506 (en langue anglaise, "FK506 binding protein")
GST : Gluthation-S-transférase
TBP : trisbipyridine. L'expression "cryptate d'europium TBP" où l'acronyme KTBP désigne un composé vendu par Cisbio, dont la formule est indiquée dans la figure 1 A
Les fluorophores suivants sont disponibles dans le commerce : DY-647-NHS (Dyomics), 5,6-carboxyfluorescéine, 5-Ex-fluorescéine (Introgen), ATTO647N-NHS (ATTO TEC), cryptate d'europium trisbipyridine (Cisbio)
La synthèse du fluorophore Tb(KR)-NHS est décrite dans la demande de brevet WO 2008/063721.

### SYNTHESES :

### I) SYNTHESES DE COMPOSES DE TYPE BENZYLGUANINE-FLUOROPHORE

Ces composés sont les composés présentés dans la partie expérimentale comme les composés de référence : ils ne comportent pas de groupe A-L₂ entre la benzylguanine et le fluorophore.

La préparation de ces composés est décrite dans la littérature et consiste à mettre en contact un dérivé BG portant un groupe réactif susceptible de réagir avec un fluorophore porteur d'un autre groupe réactif. Les schémas de synthèse ci-après sont basé sur l'utilisation de dérivés BG-NH₂ ou BG-SH, qui seront mis en contact par exemple avec un fluorophore portant un ester de N-hydroxysuccinimide ou un groupe maléimide. De tels fluorophores sont disponibles dans le commerce.

### Synthèse de BG-NH₂ :

BG-NH₂ a été synthétisé selon le protocole décrit dans l'article de Antje Keppler et al., dans Nature Biotechnology, 2003, 21, 86-89.

### Synthèse de BG-SH

Le BG-SH a été synthétisé selon la séquence décrite dans le schéma 1.

La réaction du BG-NH₂ avec SPDP (N-Succinimidyl 3-[2-pyridyldithio]propionate) suivie d'une réduction du pont disulfure avec le TCEP (hydrochlorure de tris[2-carboxéthyl]phosphine) ou de DTT conduit avec un bon rendement au BG-SH souhaité dans les conditions opératoires bien connues de l'homme du métier.

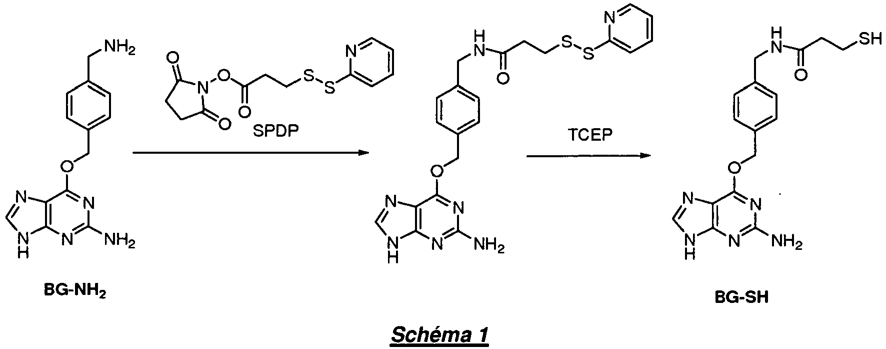

### Synthèse de BG-fluorophore et de BG-S-fluorophore

Les BG-Fluorophores et BG-S-Fluorophores ont été préparés selon les méthodes généralement utilisées pour ce type de couplage. Le BG-NH₂ réagit facilement avec un fluorophore comportant une fonction d'ester de NHS conduisant ainsi après purification par HPLC préparative au BG-fluorophore correspondant. Dans le cas où le fluorophore porte une fonction maléimide, le BG-SH est utilisé conduisant ainsi au BG-S-fluorophore (Schéma 2).

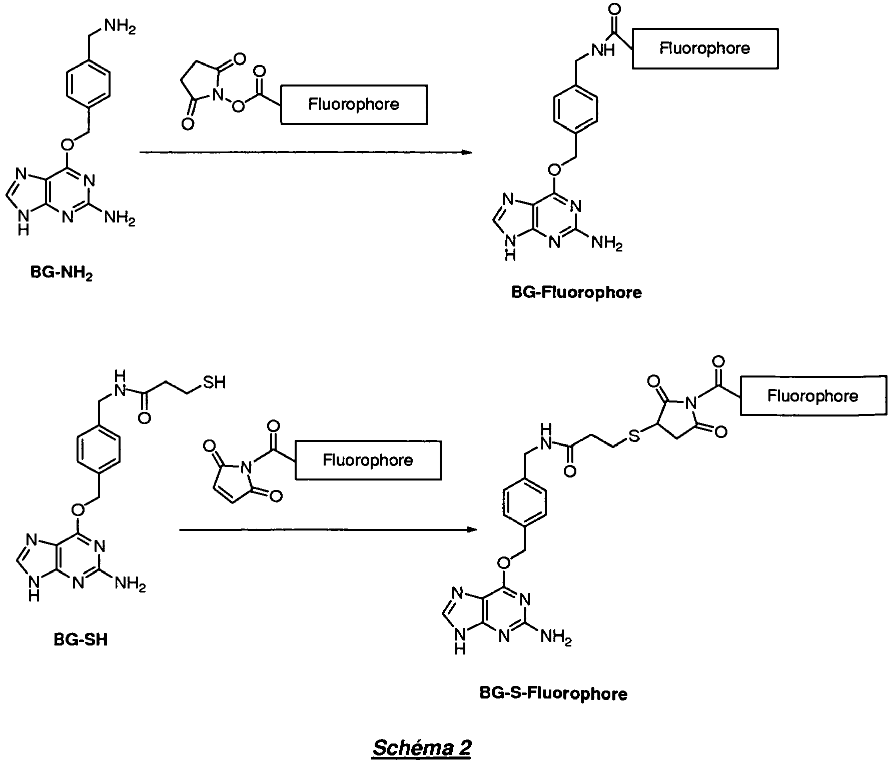

### II) SYNTHESE DE COMPOSES SELON L'INVENTION DE TYPE BENZYLGUANINE-CH₂-NH-CO-A-L2-FLUOROPHORE :

Les schémas de synthèse ci-après sont relatifs à des composés dans lesquels le groupe B est une guanine et la molécule d'intérêt est un fluorophore; la synthèse de composés comportant un autre groupe que la guanine ou une autre molécule d'intérêt qu'un fluorophore met en oeuvre les mêmes étapes ou des étapes essentiellement similaires.

La préparation de ces composés est également basée sur l'utilisation de techniques classiques de conjugaison, mettant en oeuvre divers groupes réactifs. Ces techniques classiques sont décrites par exemple dans Bioconjugate Techniques, G.T. Hermanson, Academic Press, 1996, p. 137-166.

En particulier, la synthèse de ces composés comprend les étapes essentielles suivantes :
(a) Préparation d'un groupe A porteur de deux groupes réactifs dont l'un est protégé par un groupe protecteur classique ;
(b) Activation du groupe réactif non protégé pour obtenir un groupe A porteur d'un groupe réactif protégé et d'un groupe réactif activé ;
(c) Mise en contact du produit obtenu dans l'étape précédente avec un dérivé de BG porteur d'un groupe réactif susceptible de réagir avec le groupe réactif activé porté par le groupe A, conduisant à la formation de *BG-CH₂-NH-CO-*A*-*[groupe réactif protégé] :
(d) Déprotection du groupe réactif protégé porté par le groupe A ;
(e) Mise en contact du produit obtenu dans l'étape précédente avec un fluorophore porteur d'un groupe réactif susceptible de réagir avec celui porté par le produit obtenu à l'étape précédente, conduisant au produit *BG-CH₂-NH-CO-*A*-*L2-fluorophore.

La mise en oeuvre de ce procédé très général est décrite plus en détail dans les schémas et protocoles ci-après, ainsi que dans la partie expérimentale ;

*Synthèse des groupes A préférés portant une amine protégée et une fonction carboxyle.*

### 1) Précurseurs du méthylbenzamide

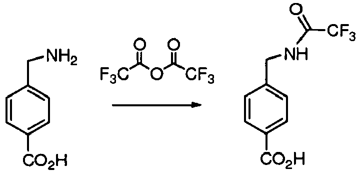

L'acide 4-aminométhylbenzoïque est protégé sous forme de trifluoroacétate permettant ainsi, dans la suite de la synthèse une déprotection douce en milieu légèrement basique.

### 2) Précurseurs des méthylnicotinamide et méthylisoniconitinamide

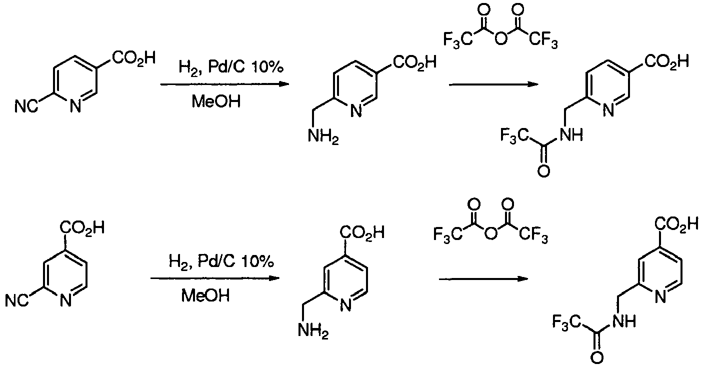

A partir des dérivés commerciaux cyanonicotinyles, les composés correspondant aminométhylnicotinyles sont obtenus par hydrogénolyse. Les groupements amino sont ensuite protégés par un groupe trifluoroacétate.

### 3) Précurseurs des trans-cyclohexylamide, cis-cydohexylamide, pipéridinylamide

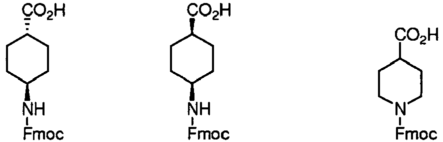

Les composés ci-dessus sont disponibles commercialement ou peuvent être préparés par des procédés bien connus de l'homme du métier.

### 4) Précurseur du carboxyméthylazidobenzamide

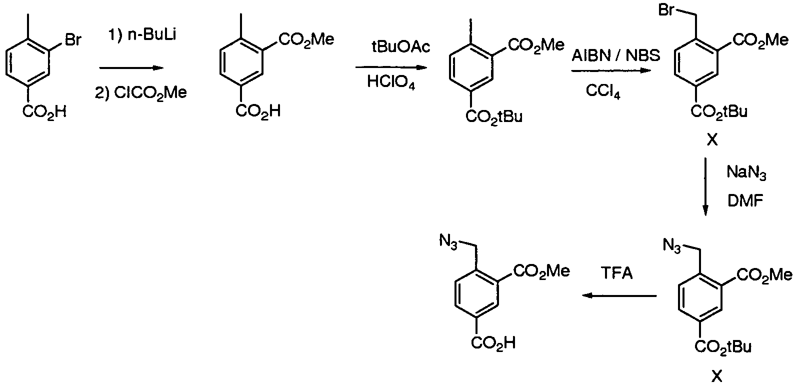

L'acide 3-bromo-4-méthylbenzoïque est converti en acide 3-carboxyméthyl-4-méthyl-benzoïque correspondant en utilisant les conditions standard généralement utilisées : échange halogène-lithium formant une espèce lithiée qui réagit avec un électrophile (diméthyle carbonate ou ClCO₂Me). La fonction acide carboxylique libre est ensuite protégée sous forme d'ester de tertiobutyle permettant d'obtenir un système de déprotection orthogonal. L'introduction du groupement azido est réalisée en deux étapes : bromation radicalaire du méthyle aromatique suivi de la substitution de l'atome de brome par l'azoture de sodium. Enfin l'ester de tertiobutyle est déprotégée en présence de TFA conduisant ainsi au précurseur souhaité.

### Couplage des groupes A portant des groupes réactifs avec une benzylguanine et un fluorophore

### 1) Fluorophores NHS

La stratégie générale de synthèse des produits selon l'invention comportant une benzylguanine et un fluorophore est présentée sur le **schéma 3** lorsque le fluorophore est utilisé sous forme d'ester de NHS. Les groupes A N-Protégés (dont la synthèse ou la source ont été présentées précédemment), sont activés soit sous forme d'anhydrides (utilisation de DCC), soit sous forme d'esters de N-hydroxysuccinimidyle (utilisation du TBTU). Ces intermédiaires réagissent rapidement avec le BG-NH₂ conduisant ainsi au produit de formule *BG-CH₂-NH-CO-*A N-protégé.

En fonction du groupement protecteur porté par l'atome d'azote du groupe A, les conditions de déprotection utilisées permettent d'obtenir le composé *BG-CH₂-NH-CO-*A*-*NH₂*.* En présence d'un fluorophore comportant une fonction ester de N-hydroxysuccinimidyle, ce composé réagit dans des conditions douces conduisant ainsi aux composés BG- *CH₂-NH-CO*-A-L₂-fluorophore.

### 2) Fluorophores Maléimide

Il est également possible d'utiliser des fluorophores portant un groupe fonctionnel maléimide. Les dérivés de BG portant le groupe *CH₂-NH-CO-*A peuvent être préparés selon la même stratégie que celle décrite pour la préparation des BG-SH à partir des BG-*CH₂*-*NH*-*CO-*A-NH₂. Ainsi la réaction de BG-*CH₂-NH-CO-*A-NH₂ avec SPDP, suivie de la réduction au TCEP permet d'accéder aux composés BG-*CH₂-NH-CO-*A-NH-CO-(CH₂)₂-SH **(Schéma 4).** Ces derniers conduisent aux BG-*CH₂-NH-CO-*A-L2-Fluorophore en présence d'un fluorophore portant un groupe maléimide.

Les composés selon l'invention sont bien sûr particulièrement adaptés au marquage de protéines de fusion comprenant une molécule d'intérêt et une enzyme AGT. Ainsi l'invention concerne également une méthode de marquage d'une protéine d'intérêt par un groupe M, ladite protéine d'intérêt étant exprimée sous forme de protéine de fusion avec une enzyme AGT, cette méthode comprenant une étape de mise en contact de ladite protéine de fusion avec un composé selon l'invention. Cette méthode est ici mise en oeuvre dans les exemples suivants pour étudier la réactivité enzymatique des composés selon l'invention avec différents variants de l'AGT, dans un modèle in vitro et dans un modèle cellulaire.

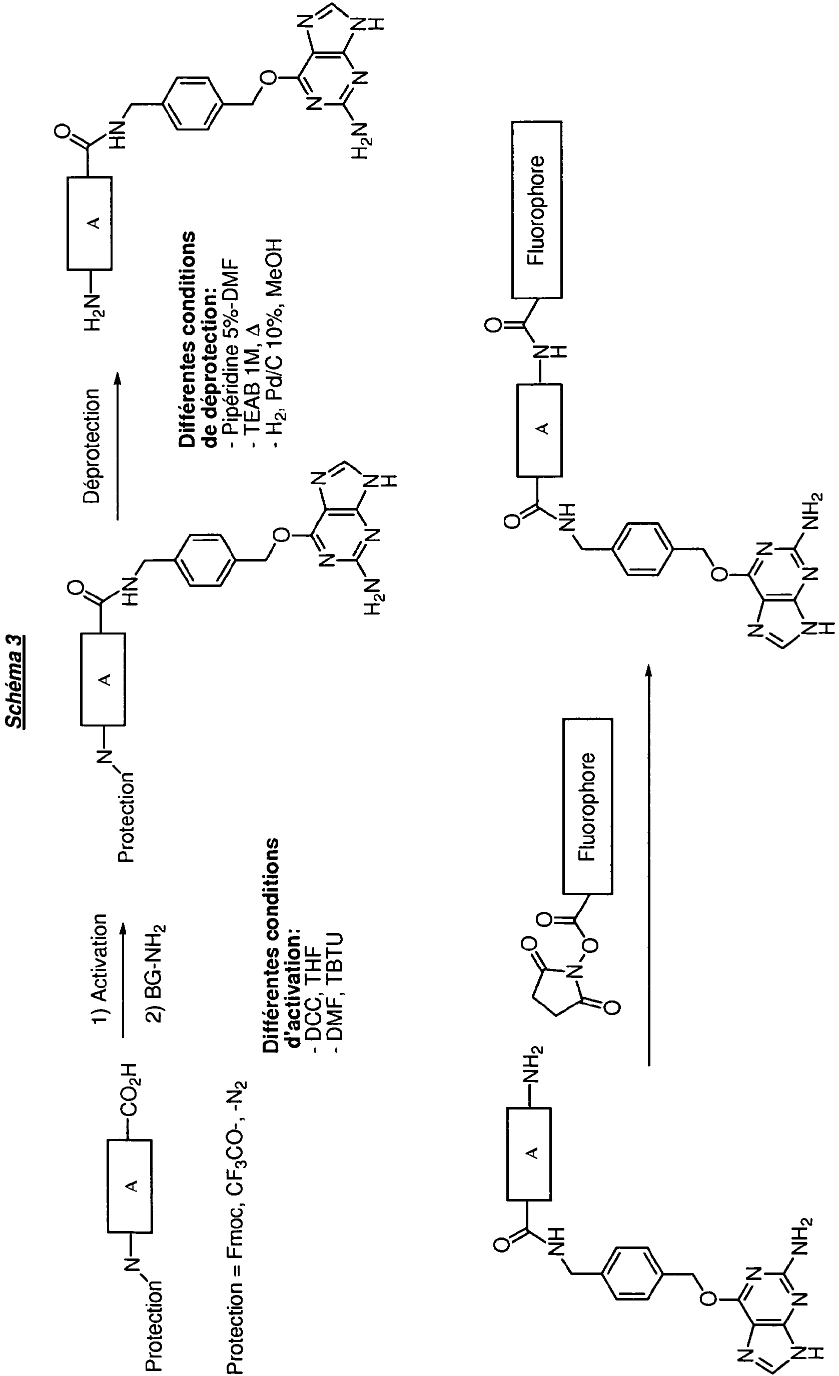

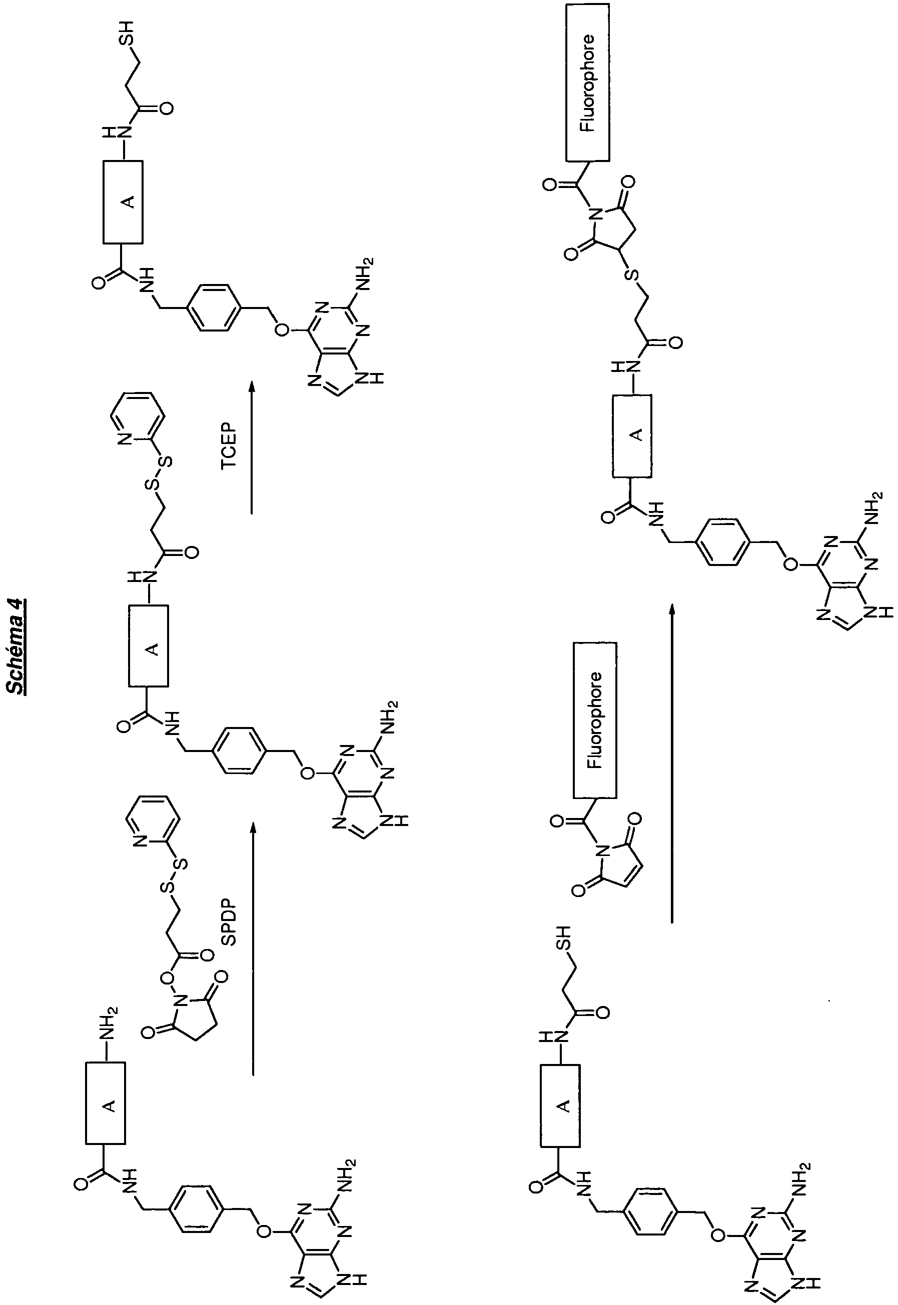

### TEST DE REACTIVITE ENZYMATIQUE

Les exemples qui suivent ont pour objet de mesurer la cinétique de transfert d'un fluorophore sur une protéine de fusion comprenant une enzyme AGT (SNAP-tag, N-AGT ou CLIP-tag), à partir d'un substrat de référence (de type benzylnucléotide-fluorophore) ou bien à partir de composés selon l'invention (benzylnucléotide-*CH₂*-*NH*-*CO*-A-L2-fluorophore).

Ces exemples permettent de mettre en évidence la réactivité bien supérieure des substrats selon l'invention avec l'enzyme SNAP-tag et un de ces variants, en comparaison avec les substrats de l'art antérieur.

La mesure de la réactivité des substrats avec l'enzyme SNAP-tag est effectuée soit dans un modèle in vitro, soit dans un modèle sur cellules vivantes.

### Mode opératoire du test de réactivité enzymatique sur modèle in vitro :

Le test de réactivité des substrats selon l'invention avec l'enzyme SNAP-tag est basé sur un test in vitro de marquage d'une protéine recombinante. Ce marquage est mis en évidence par la détection d'un signal de FRET entre deux protéines, chacune marquée par un fluorophore membre d'un couple de partenaires de FRET.

En l'occurrence, on utilise les protéines FRB et FKBP qui interagissent en présence de rapamicyne.

La protéine FKBP est marquée par un premier fluorophore par la technologie SNAP-tag : pour cela une protéine de fusion GST-SNAPtag-FKBP est produite à partir du plasmide pSET-26b selon le protocole du kit commercial (SNAP express pSTE7-26b kit Covalys Biosciences AG Witterswil/Switzerland). Cette protéine de fusion est ensuite mise en contact avec le premier fluorophore conjugué à un dérivé de BG de référence ou à un dérivé de BG selon l'invention.

La protéine FRB est quant à elle couplée par des techniques classiques de conjugaison avec un composé fluorescent donneur (Cryptate d'europium TBP (KTBP), Cisbio Bioassay) ou accepteur (DY647, Dyomics) selon les cas, en suivant les protocoles fournis avec ces produits.

Le tableau ci-dessous explicite la nature des couples de fluorophores dans chacun des exemples 1 à 4 suivants :

| **Exemple** | **BG conjugué avec :** | **FRB fusionnée avec** : |
|---|---|---|
| 1 | DY647 | KTBP |
| 2 | ATTO647 | KTBP |
| 3 | 5-Ex-fluorescéine | KTBP |
| 4 | Tb(KR) | DY647 |
| 5 | DY647 | KTBP |
| 6 | Tb(KR) | DY647 |

Dans chaque exemple, 2 nmoles de protéine recombinante FRB marquée avec un cryptate d'europium ou DY647 (2^{ème} fluorophore), sont co-incubées à température ambiante avec 2 nmoles de protéine recombinante GST-SNAP-tag-FKBP et des concentrations croissantes de BG marquée par le premier fluorophore (BG-fluorophore de l'art antérieur ou BG-*CH₂*-*NH-CO*-A-L2-fluorophore selon l'invention).

Les lectures de fluorescence se font à 620 nm et à 665 nm sur RubyStar (BMG laboratory) après une excitation à 337 nm. Les lectures sont réalisées avant et après induction de l'interaction protéique FRB/FKBP par 100 nM de rapamycine. Cette interaction se traduit par l'émission d'un signal de TR-FRET. L'enzyme SNAP-tag ne pouvant fixer qu'un seul groupement fluorescent, l'augmentation du signal de TR-FRET est directement proportionnelle au pourcentage de protéines marquées dans le milieu réactionnel. Ce modèle *in vitro* permet d'établir des cinétiques de marquage enzymatique en fonction de l'excès de substrat engagé dans la réaction.

Les résultats sont indiqués soit en d665 soit en % de marquage suivant les formules suivantes : $d 665 = \left(signal à 665 nm avec la protéine GST-ST-FKBP\right) - \left(signal à 665 nm sans la protéine GST-ST-FKBP\right) .$
Le pourcentage de marquage est obtenu par comparaison avec le signal correspondant au marquage maximal qui est obtenu après 18 heures d'incubation. $% marquage = \left(d⁢665 à t mesure/d⁢665 18 h dʹincubation\right) x 100.$

### Mode opératoire du test de réactivité enzymatique sur modèle cellulaire.

Ce mode opératoire permet de tester la réactivité de substrats selon l'invention avec une AGT exprimée par des cellules COS 7 sous forme de protéine de fusion avec le récepteur transmembranaire au glutamate de type 2 (mGluR2).

Différents mutants d'AGT ont été analysés dans cette étude : SNAP-tag-ST26 ou CLIP-tag de la société Covalys ou le mutant N-AGT décrit par Gronemeyer et al (Protein Engineering, Design & Selection, vol. 19, no 7, pp. 309-316, 2006).

### Plasmides :

- Plasmide SNAP-tag-mGluR2, préparé à partir du plasmide pSET-26b selon le protocole du kit commercial (SNAP express pSTE7-26b, kit Covalys Biosciences)
- Plasmide pcdna3.1 : invitrogen Ce plasmide est utilisé comme contrôle négatif ; les cellules transfectées avec ce plasmide sont désignées cellules « mock » et permettent de déterminer le niveau de marquage non spécifique.

### Protocole :

La transfection transitoire des cellules est effectuée par électroporation à l'aide un électroporateur Biorad : 10 millions de cellules sont transfectées par 1 µg SNAP-tag-mGluR2 + 3µg pcdna3.1 dans un volume final de 300µl. Les paramètres d'électroporation sont : 280V & 1000µF.

Les cellules sont ensuite ensemencées à la densité de 150 000 cellules par puits dans des plaques Greiner (noire fond opaque) préalablement incubées avec de la poly-ornithine pendant 30 minutes à 37°C.

Les cellules sont cultivées dans du milieu DMEM + Rouge Phénol + 10% SVF + Penicilline/Streptomycine + acides aminés non essentiels à 37°C et 5% CO2. Après 24h d'expression, un premier lavage est effectué avec 100µl de ce même milieu.

Le marquage de la protéine de fusion AGT-mGluR2 s'effectue dans 100µl de milieu complet avec des concentrations croissantes de BG-fluorophore ou BG*-CH₂-NH-CO-*A*-*L2-fluorophore selon l'invention pendant 1h à 37°C. Avant la lecture, 4 lavages sont effectués avec 100µl de Tris-Krebs.

Pour les substrats porteurs des fluorophores ATTO647 (exemple 2), DY647 (exemples.1 et 5) et (exemple 3), la lecture est réalisée dans 100µl de Tris-Krebs en fluorescence directe à 682 sur Rubystar, et à 520 nm pour ceux porteurs de 5-Ex-fluorescéine (exemple 3).

Pour les substrats porteurs du complexe de terbium KR (exemples. 4 et 6), la lecture est réalisée dans 100µl de Tris-Krebs en fluorescence en temps résolu à 620 nm sur Rubystar.

### EXEMPLE 1 : BG-méthylbenzamide-DY647

Comparaison des réactivités entre les BG-DY647 & BG-méthylbenzamide-DY647 et l'enzyme SNAP-tag.

### Structure du substrat de référence BG-DY647 :

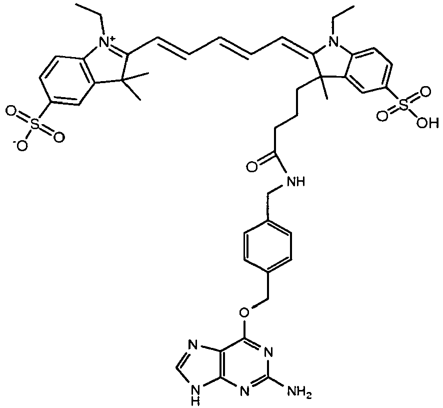

### Structure du substrat BG-méthylbenzamide-DY647 :

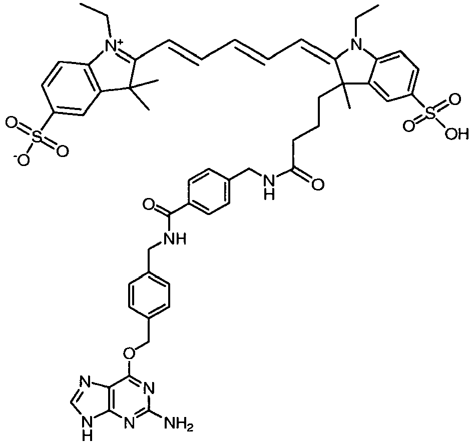

### • Résultats de réactivité enzymatique et de marquage protéique in vitro

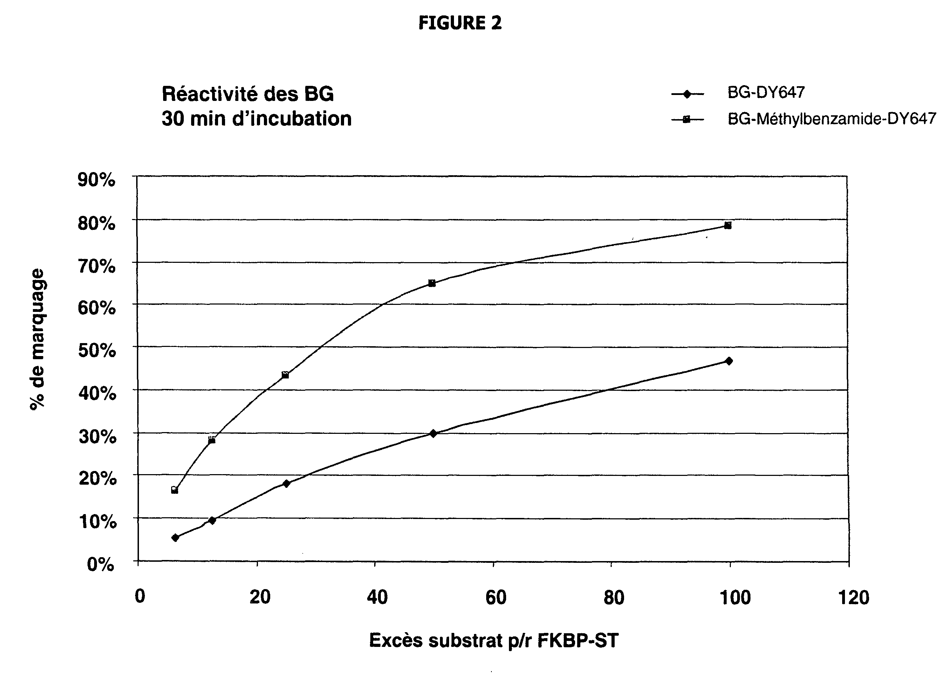

La **figure 2** représente l'évolution du pourcentage de marquage de l'enzyme SNAP-tag en fonction de la concentration en substrat. Ces résultats montrent que le marquage est fortement amélioré après 30 min d'incubation avec le substrat selon l'invention comportant un motif méthylbenzamide, et ce quelle que soit la concentration de substrat testée.

### • Résultats obtenu sur modèle cellulaire

### Effet avec l'enzyme SNAP-tag ST26

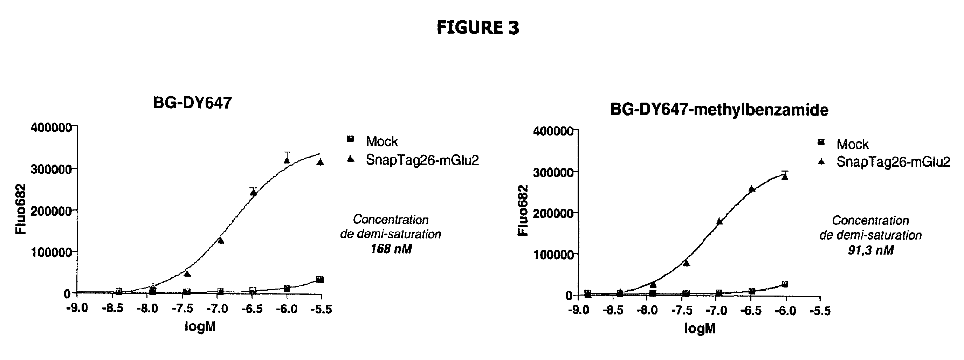

La concentration permettant la demi-saturation de l'enzyme est de 168 nM avec le composé de référence alors qu'elle est de seulement 91,3 nM avec le substrat selon l'invention (**figure 3**)**.** Ces résultats obtenus sur cellules vivantes confirment ceux obtenus dans le modèle in vitro. Cette faible concentration de demi-saturation avec les composés selon l'invention permet de travailler en faible concentration de substrat, ici inférieure à 100 nM.

Afin de valider l'effet du substrat selon l'invention sur un autre mutant de l'AGT, une caractérisation de marquage extracellulaire avec le mutant NAGT a été réalisée.

### Effet avec l'enzyme N-AGT

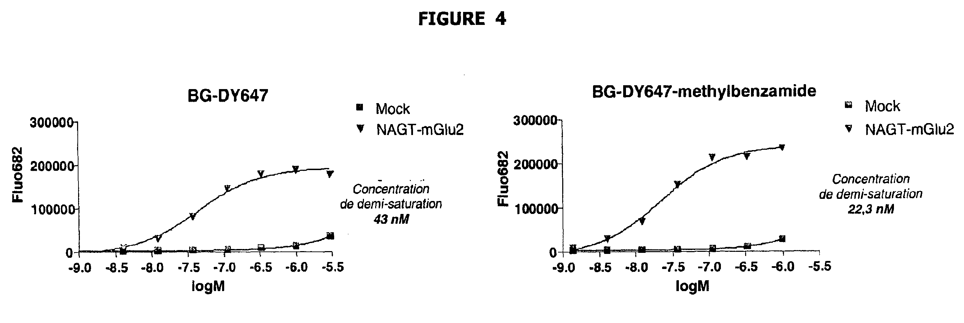

La figure 4 montre les résultats obtenus avec l'enzyme N-AGT, un mutant plus réactif que SNAP-tag, et là encore la concentration de demi-saturation est environ 2 fois moins élevée avec le composé selon l'invention (22,3 nM) par rapport au substrat de référence (43 nM). Cet exemple montre que la réactivité supérieure des substrats selon l'invention n'est pas spécifique de l'enzyme SNAP-tag ST-26 mais est également observée lorsque des variants de cette enzyme sont utilisés.

### EXEMPLE 2 : BG-Méthylbenzamide-ATTO647

Comparaison de la réactivité de BG-ATTO647N / BG-méthylbenzamide-ATTO647 N avec l'enzyme SNAP-tag.

### Structure du substrat de référence: BG-ATT0647N :

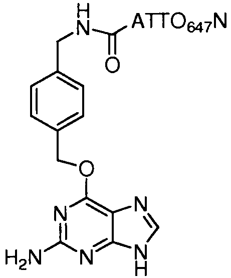

### Structure du substrat BG-Méthylbenzamide-ATTO647N :

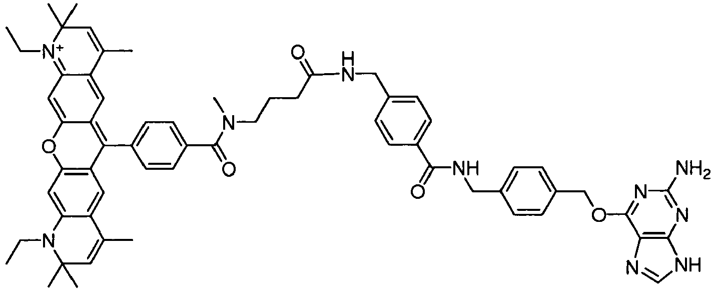

### • Résultats de réactivité enzymatique et de marquage protéique in vitro

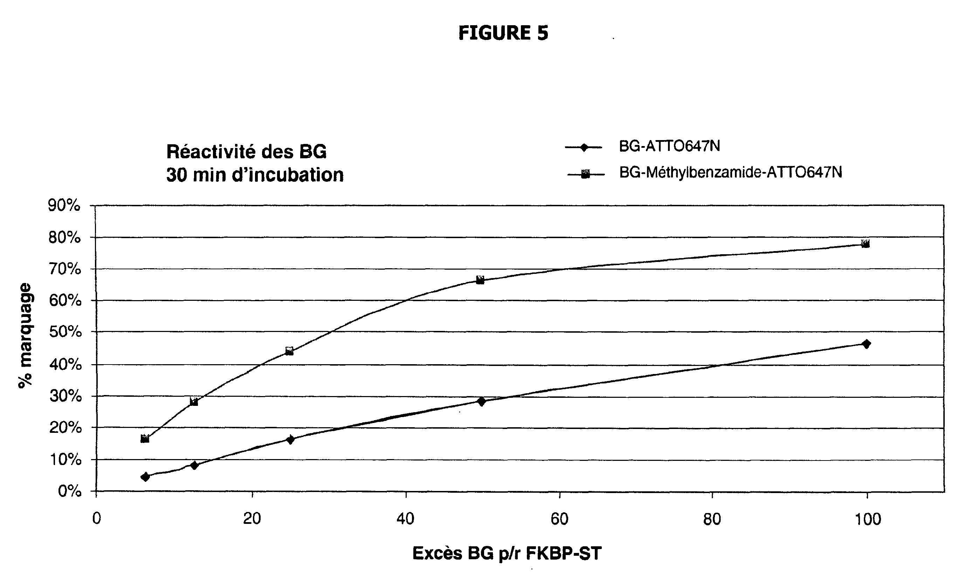

La **figure 5** représente l'évolution du pourcentage de marquage de l'enzyme SNAP-tag en fonction de la concentration en substrat après 30 min d'incubation : Là encore on observe que le pourcentage d'enzyme marquée par l'ATTO647 est bien meilleur lorsque le substrats selon l'invention sont utilisés, et cela quelle que soit la concentration de substrat.

Cet exemple montre que l'effet des substrats selon l'invention ne dépend pas de la nature de la molécule d'intérêt puisque des résultats similaires sont obtenus avec 2 fluorophores différents, DY647 (exemple 1) et ATTO647.

### EXEMPLE 3 : BG-méthylbenzamide-5-Ex-fluorescéine

Comparaison de la réactivité de BG-5-Ex-fluorescéine / BG-méthylbenzamide-5 Ex-fluorescéine avec l'enzyme SNAP-tag.

### Structure du substrat de référence BG-5-Ex-fluorescéine:

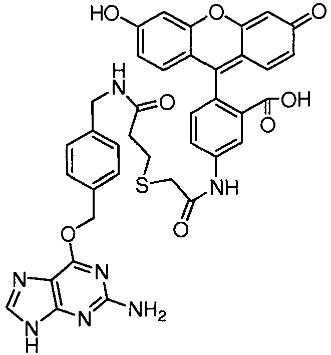

### Structure du substrat BG-méthylbenzamide- 5 fluorescéine-EX :

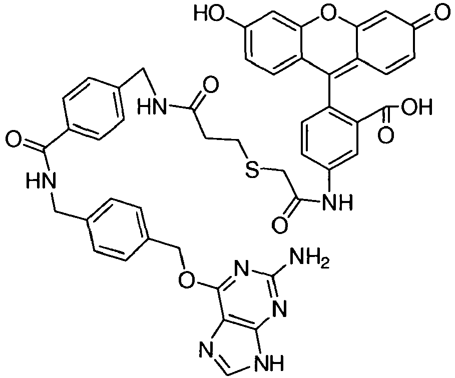

### • Résultats de réactivité enzymatique et de Marquage protéique in vitro

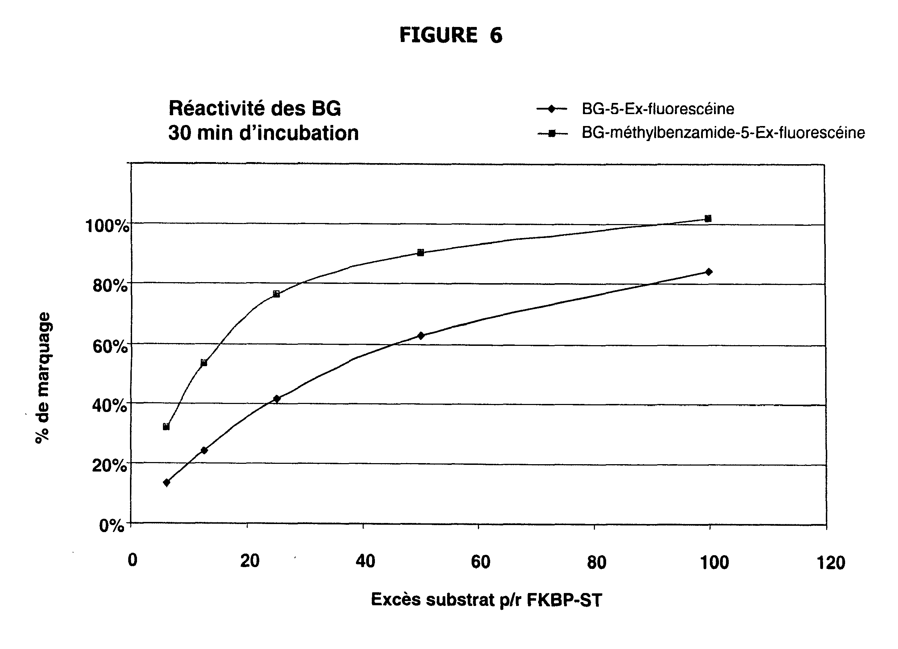

Les résultats présentés sur la **figure 6** montrent là-encore que la réactivité du substrat selon l'invention avec l'enzyme SNAP-tagST-26 est meilleure que celle du substrat de référence, et cela alors même que la réactivité du substrat BG-5-Ex-fluorescéine de référence peut être considérée comme correcte. Ces résultats confirment également que l'effet des substrats selon l'invention ne dépend pas de la nature de la molécule d'intérêt qui y est conjuguée.

### EXEMPLE 4 : BG-méthylbenzamide-Tb(KR)

Comparaison de la réactivité de BG-Tb(KR) / BG-méthylbenzamide-Tb(KR) avec l'enzyme SNAP-tag.

### Structure du substrat de référence : BG- Tb(KR) :

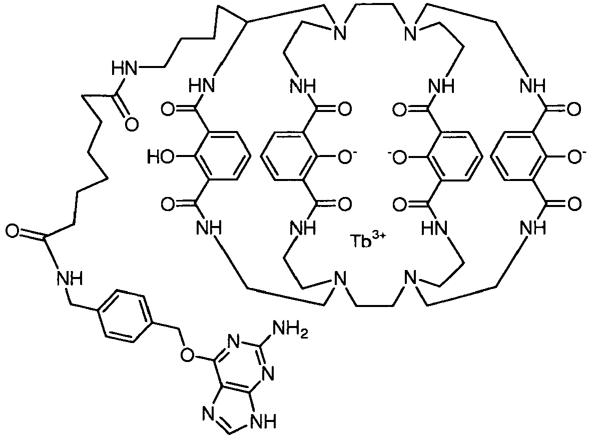

### Structure du substrat BG-méthylbenzamide-Tb(KR) :

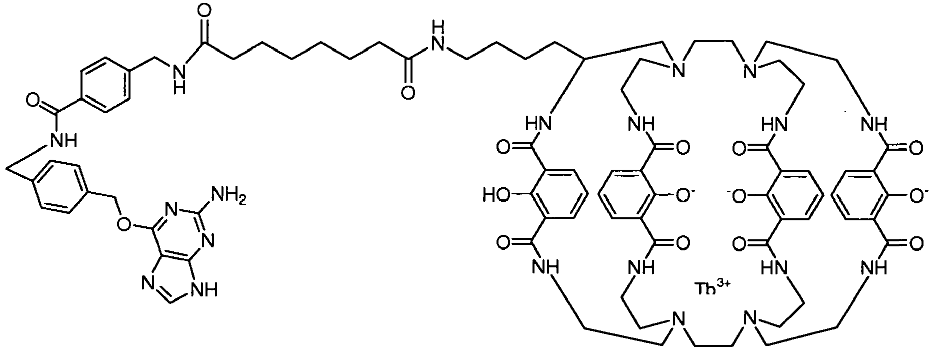

### • Résultats de réactivité enzymatique et de marquage protéique in vitro

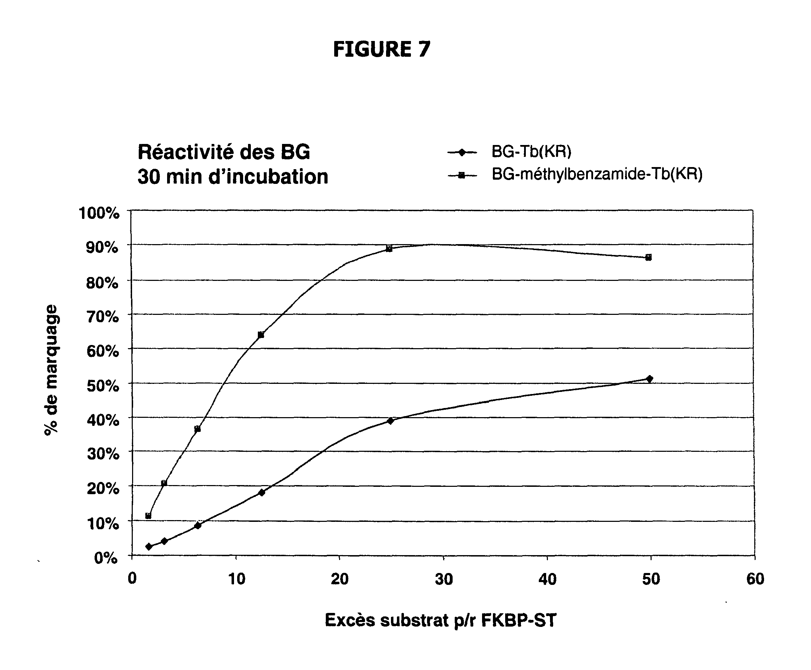

Les résultats présentés sur la **figure 7** montrent là encore une bien meilleure réactivité des substrats selon l'invention avec l'enzyme SNAP-tag par rapport au substrat de référence. On note qu'ici la molécule conjuguée n'est pas un fluorophore organique comme dans les exemples précédents mais un complexe constitué d'un polymacrocycle et d'une terre rare, beaucoup plus volumineux que les fluorophores testés dans les exemples précédents. Cet exemple illustre à nouveau le caractère universel des substrats selon l'invention qui peuvent être conjugués à une variété de molécules.

### • Résultats obtenu sur modèle cellulaire

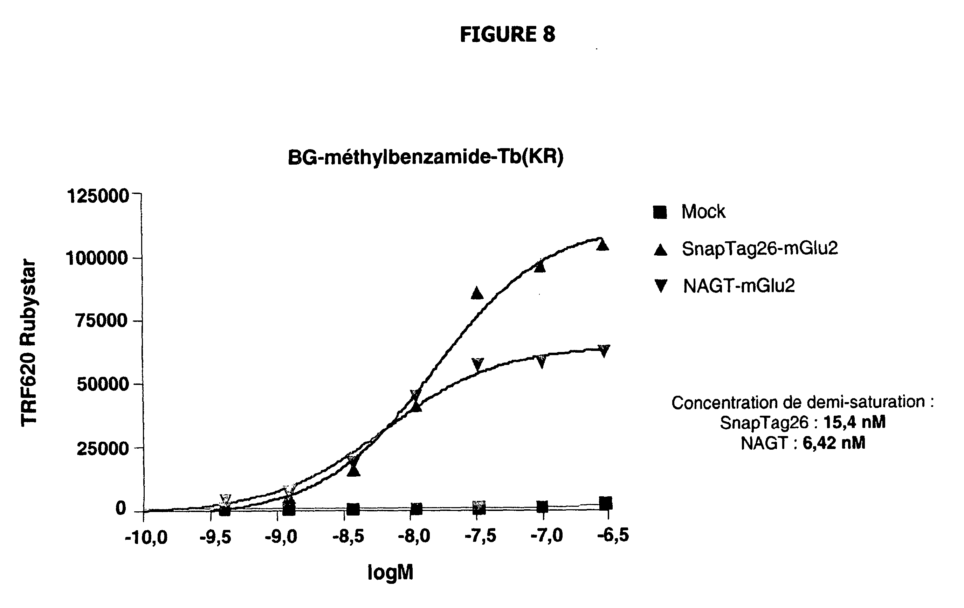

Les résultats obtenus sur le modèle in vitro sont confirmés lorsque le substrat BG-méthylbenzamide-Tb(KR) est utilisé pour marquer une protéine transmembranaire exprimée à la surface de cellules vivantes. Les concentrations de demi-saturation mesurées avec le substrat selon l'invention sont de 15,4 nM avec l'enzyme SNAP-tag-ST26, et de 6,4 nM avec le mutant N-AGT (**Figure 8**) alors qu'elle est de 60 nM avec un substrat de référence BG-Tb(KR) (SNAP-tag).

### EXEMPLE 5 : BG-aminonicotinamide-DY647

Comparaison de la réactivité de BG-DY647 / BG-Aminométhylnicotinamide-DY647 avec l'enzyme SNAP-tag.

### Structure du substrat de référence BG-DY647 :

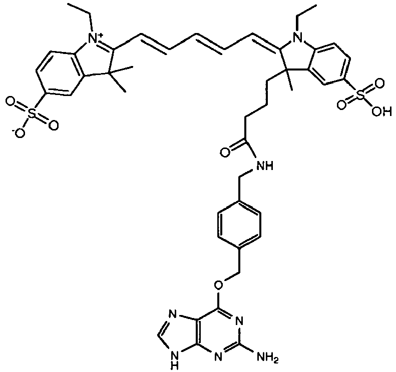

### Structure du substrat BG-aminomethylnicotinamide -DY647 :

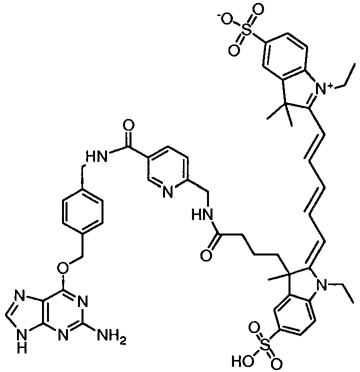

### • Résultats de réactivité enzymatique et de Marquage protéique in vitro

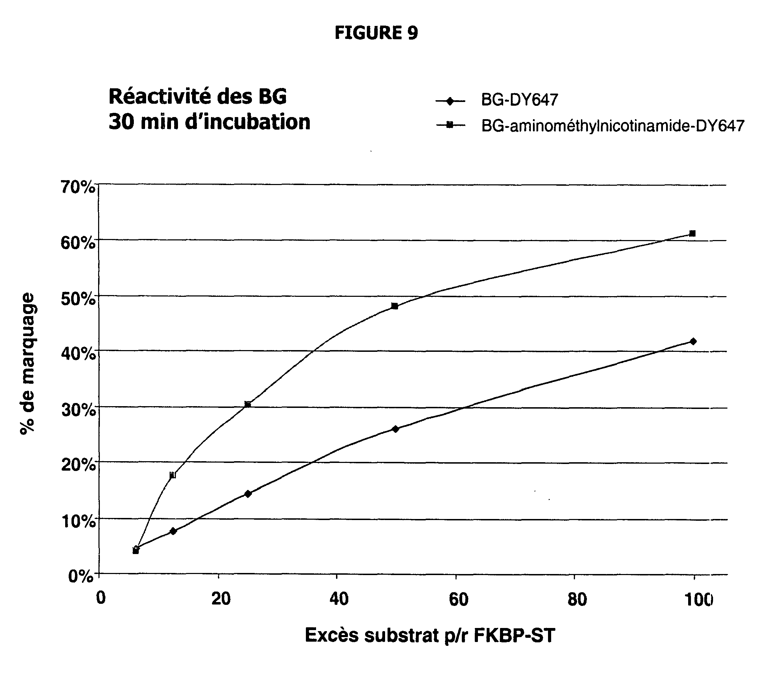

Les courbes de la **figure 9** montrent que le pourcentage d'enzyme marquée après 30 minutes d'incubation avec des substrats comportant un motif aminométhylnicotinamide est également bien meilleur que celui observé avec le substrat de référence, et cela quelle que soit la concentration en substrat.

Par ailleurs, le gain de réactivité observé avec le substrat comportant un motif aminométhylnicotinamide est du même ordre de grandeur que celui obtenu avec les substrats comportant un motif méthylbenzamide.

Ces résultats suggèrent qu'une certaine variabilité est possible au niveau du motif aromatique des substrats selon l'invention (motif A de la formule I)

### EXEMPLE 6 : BG-aminonicotinamide-Tb(KR)

Comparaison de la réactivité de BG- Tb(KR) / BG- Aminonicotinamide - Tb(KR) avec l'enzyme SNAP-tag.

### Structure du substrat de référence : BG-Tb(KR)

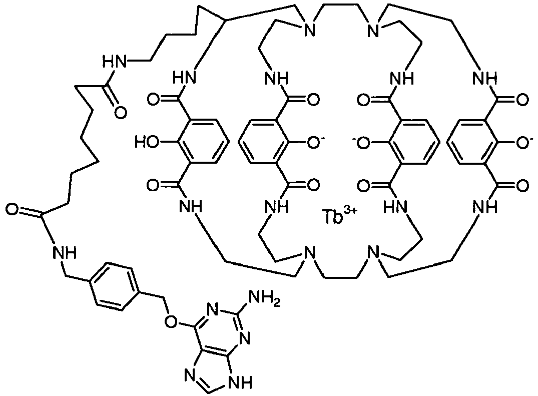

### Structure du substrat BG-aminomethylnicotinamide-Tb(KR) :

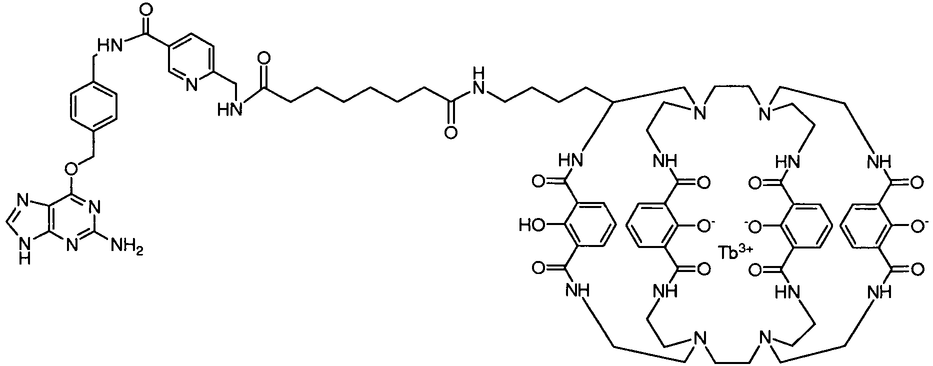

On a obtenu des résultats similaires à ceux obtenus avec les composés des exemples précédents.

### EXEMPLE 7: BC-méthylbenzamide-Tb(KR)

Comparaison de la réactivité de BC-Tb(KR) / BC-méthylbenzamide-Tb(KR) avec l'enzyme CLIP-tag.

Comme mentionné précédemment, l'enzyme CLIP-tag est un mutant de l'AGT réactif avec les dérivés de la benzylcytosine, et est commercialisée par la société Covalys, comme l'enzyme SNAP-tag.

### Structure du substrat de référence : BC-Tb(KR)

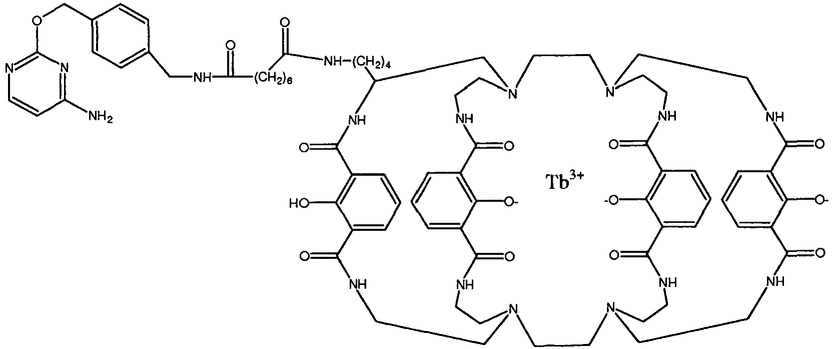

### Structure du substrat BC-méthylbenzamide-Tb(KR) :

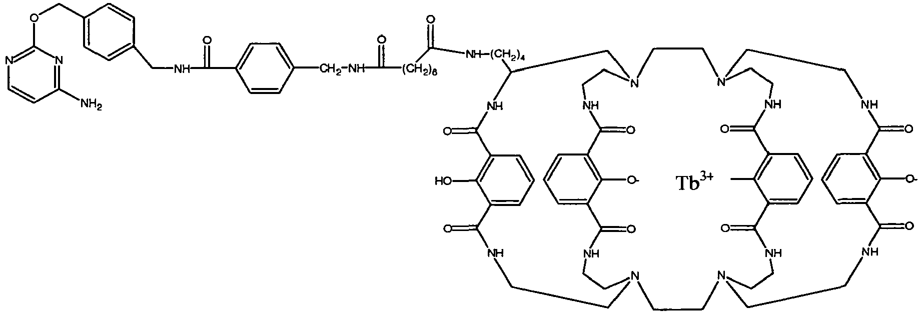

### • Résultats obtenu sur modèle cellulaire

Le modèle cellulaire utilisé dans cet exemple est différent de celui utilisé dans les exemples précédents :
Les cellules sont soit cotransfectées avec un plasmide codant pour la protéine de fusion CLIPtag-mGluR2 et un autre plasmide codant pour la protéine de fusion SNAPtag-mGluR2, soit transfectées avec un seul plasmide codant pour la protéine de fusion SNAPtag-mGluR2.

Chaque population de cellule est incubée en présence de 300 nM de BG-méthylbenzamide-DY647 et de concentrations croissantes de substrats de référence (BC-Tb(KR)) ou de substrat selon l'invention (BC-méthylbenzamide-Tb(KR)). La fluorescence est mesurée à 665 nm en mode TR-FRET sur Rubystar et le signal mesuré est représentatif d'un transfert d'énergie consécutif à la dimérisation de deux monomères de mGluR2 marqués respectivement par DY647 et Tb(KR).

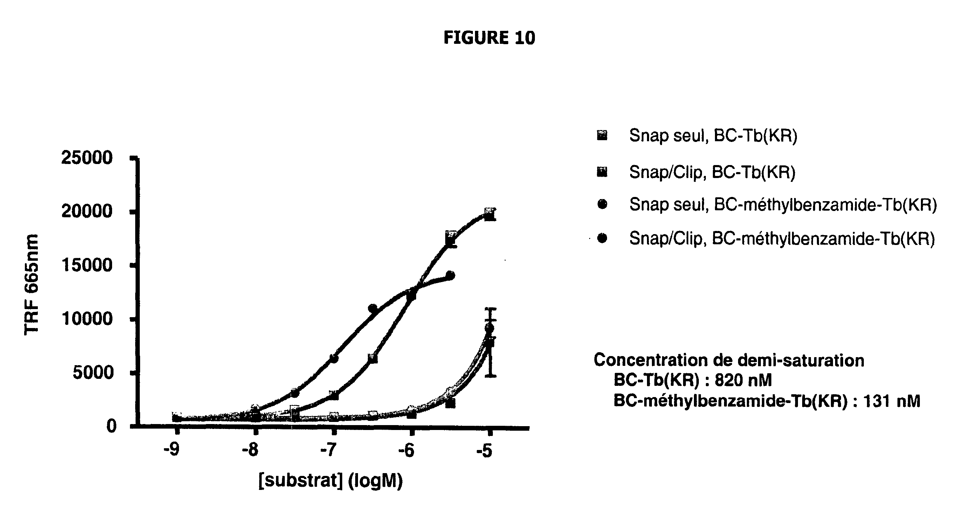

On peut observer sur la **figure 10** que la concentration de demi-saturation de CLIPtag par le dérivé de benzylcytosine selon l'invention est de 131 nM, alors qu'elle est de 820 nM avec le composé de référence. (Le signal observé dans les cellules exprimant SNAPtag-mGluR2 seulement correspond au signal de fixation non spécifique de BG-DY647 par CLIPtag)

Cet exemple confirme que l'effet inattendu des substrats selon l'invention n'est pas limité à SNAPtag / benzylguanine mais est également observé avec CLIPtag / benzylcytosine.

### EXEMPLE 8 : BC-méthylbenzamide-DY647

### Comparaison de la réactivité de BC-DY647 / BC-méthylbenzamide-DY647 avec l'enzyme CLIP-tag

On procède dans cet exemple comme dans l'exemple 7 mais les cellules sont incubées en présence de 300 nm de BG-méthylbenzamide-Tb(KR) et de concentrations croissantes de substrat de référence (BC-DY647) ou de substrat selon l'invention (BC-méthylbenzamide-DY647) dont les formules sont ci-dessous :

### Substrat de référence (BC-DY647)

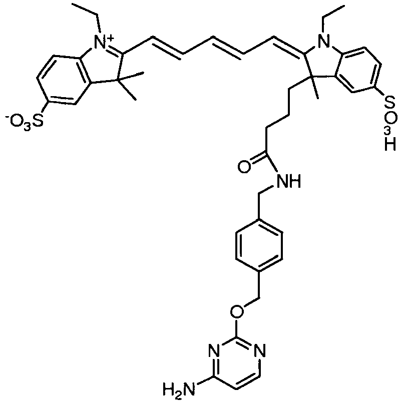

### Substrat BC-méthybenzamide-DY647 :

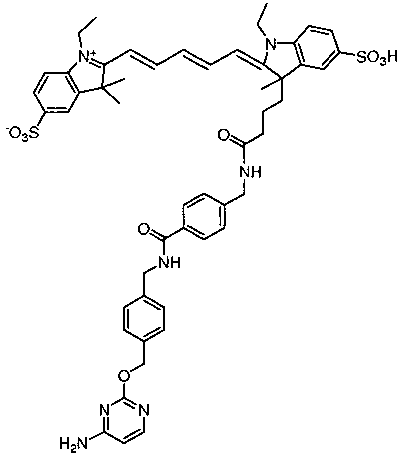

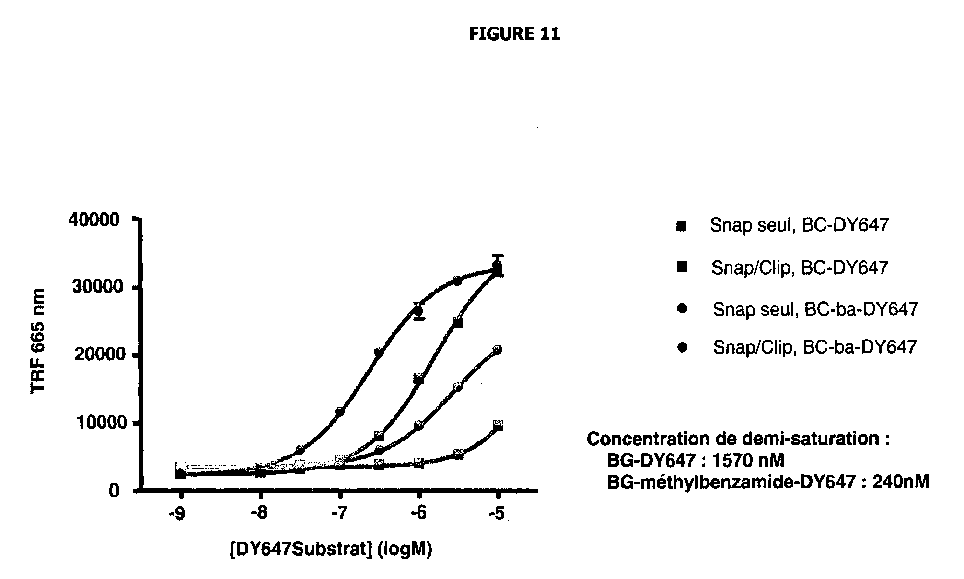

Les résultats présentés sur la **figure 11** confirment la meilleure réactivité du substrat selon l'invention (demi-saturation : 240 nM) par rapport au substrat de référence (demi-saturation : 1570 nM).

### EXEMPLE 9 : BG-cis-cyclohexane-DY647

### Comparaison de la réactivité de BG-DY647 / BG-cis-cyclohexane-DY647 avec l'enzyme SNAP-tag

### Structure du substrat de référence BG-DY647 (composé 1) :

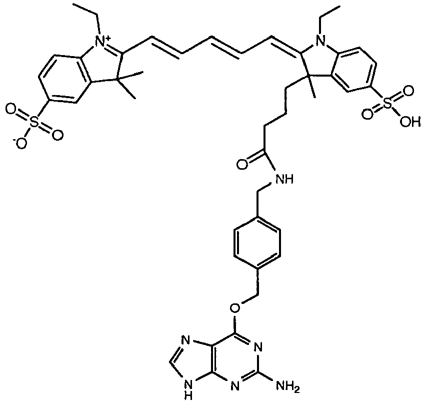

### Structure du substrat BG -cis-cyclohexane-DY647 (composé 29) :

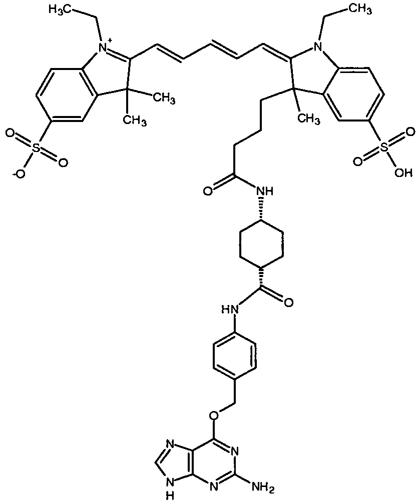

### • Résultats de réactivité enzymatique et de Marquage protéique in vitro

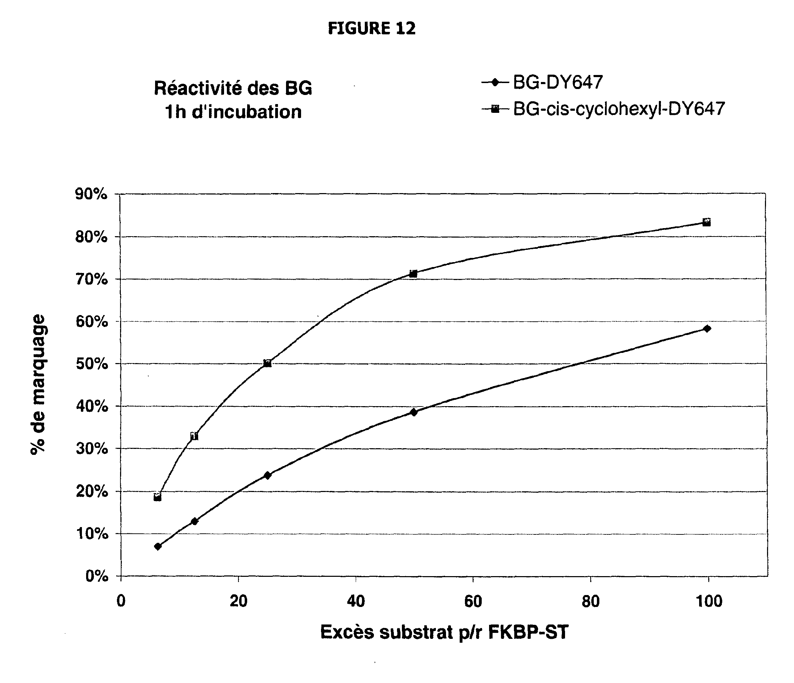

Les courbes de la figure **12** montrent que le pourcentage d'enzyme marquée après 1h d'incubation avec des substrats comportant un motif cis-cylohexane est bien meilleur que celui observé avec le substrat de référence, et cela quelle que soit la concentration en substrat

### EXEMPLE 10 : BG-méthylbenzamide-Atto465

### Comparaison de la réactivité de BG-Atto465/ Bg-méthylbenzamide-Atto465 avec l'enzyme SNAP-tag

### Structure du substrat de référence BG-Atto465 (composé 35) :

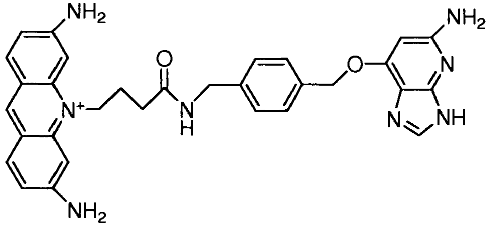

### Structure du substrat BG-méthylbenzamide-Atto465 (composé 36)

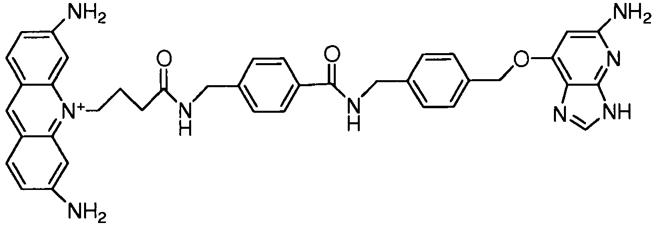

### • Résultats obtenu sur modèle cellulaire

Le marquage d'une protéine transmembranaire exprimée à la surface de cellules vivantes montre que le substrat BG-méthylbenzamide-Atto465 a une bien meilleure réactivité avec l'enzyme SNAP-tag que le substrat de référence. La concentration de demi saturation mesurée avec BG-méthylbenzamide-Atto465 est de 213 nM alors qu'elle est de 1983 nM avec le substrat BG-Atto465..

***Les synthèses des composés suivants sont décrites ci-après** :*
Composé 1 : benzylguanine-Dy647
Composé 2 : benzylguanine-5,6-carboxyfluorescéine
Composé 3 : benzylguanine-5-Ex-fluorescéine-Ex
Composé 4 : benzylguanine-ATTO647N
Composé 5 : benzylguanine-Tb(KR)
Composé 6 : Acide 4-((2,2,2-trifluoroacétamido)méthyl)benzoïque
Composé 7 : Trifluoroacétamidométhylbenzamide-BG
Composé 8 : Aminométhylbenzamide-BG
Composé 9 : Dy647-méthylbenzamide-BG
Composé 10 : ATTO647N-méthylbenzamide-BG
Composé 11 : 5,6-carboxyfluoresceine-méthylbenzamide-BG
Composé 12 : 5-fluorescéine-Ex-Benzylamide-BG
Composé 13 : Tb(KR)-méthylbenzamide-BG
Composé 14 : BG-SH
Composé 15 : BG-méthylbenzamide-SH
Composé 16 : acide 2-(trifluoroacétamidométhyl)nicotinique
Comaosé 17 : acide 2-(trifluoroacétamidométhyl)isonicotinique
Composé 18 : DY647-aminométhylisonicotinamide-benzylguanine
Composé 19 : DY647-aminométhylnicotinamide-benzylguanine
Composé 20 :Tb(KR)-aminométhylnicotinamide-benzylguanine
Composé 21 : benzylguanine-N-Fmoc-pipéridine
Composé 22 : benzylguanine-pipéridine
Composé 23 : DY647-pipéridine-benzylguanine
Composé 24 : benzylguanine-trans-4-(Fmoc-amino)-cyclohexane
Composé 25 : benzylguanine-trans-4-amino-cyclohexane
Composé 26 : DY647-trans-cyclohexane-benzylguanine
Composé 27 : benzylguanine-cis-4-(Fmoc-amino)-cyclohexane
composé 28 : benzylguanine-cis-4-amino-cyclohexane
Composé 29 : DY647-cis-cyclohexane-benzylguanine
Composé 30 : Benzylcytosine-DY647
Composé 31 : Benzylcytosine-Tb(KR)
Composé 32 : Aminométhylbenzamide-BC
Composé 33 : DY647-méthylbenzamide-BC :
Composé 34 : Tb(KR)-méthylbenzamide-BC:
Composé 35 : Atto465-BG:
Composé 36 : Atto465-méthylbenzamine-BG:

### Composé 1 : benzylguanine-DY647

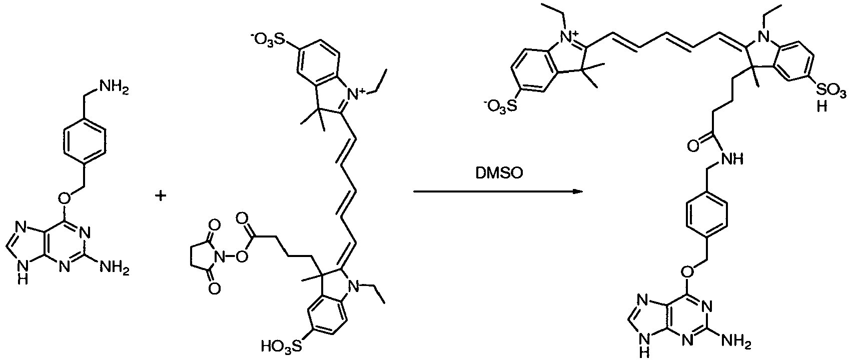

Dans un ballon de 10 ml on a introduit 4,2 mg (15,5 µmol) de 6-aminométhylbenzylguanine et 11,8 mg (15,5 µmol) de DY647-NHS. Les produits ont été partiellement solubilisés dans 4 ml de diméthylsulfoxyde anhydre.

Le mélange a été agité pendant une heure. La réaction a été suivie par HPLC sur une colonne Merck Lichrospher RP° 18 5 µm 125 x 4,6 avec un gradient d'acétonitrile dans de l'eau à 0,2% d'acide trifluoroacétique.

Une purification par HPLC préparative a été effectuée sur une colonne Vydac C18 10 µm 250 x 22 avec un gradient d'acétonitrile dans de l'eau à 0,2% d'acide trifluoroacétique.

Les fractions ont été collectées et concentrées sous pression réduite. Un solide bleu a été obtenu (8,5 µmol par mesure de densité optique, 55%) correspondant au produit souhaité. MS (ES⁺) m/z : [M+H⁺] 895,3.

### Composé 2 : benzylguanine-5,6-carboxyfluorescéine

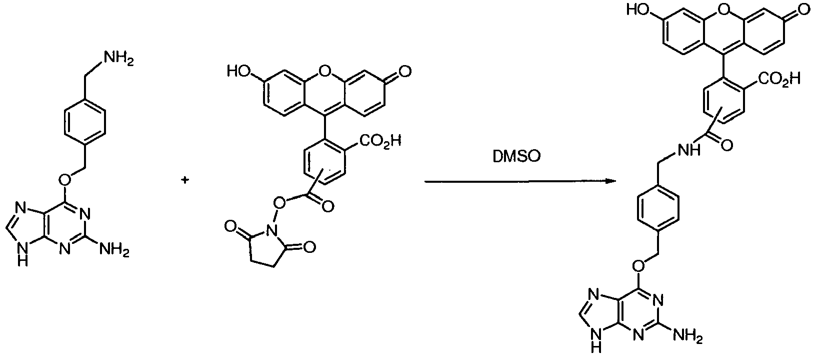

Dans un tube Eppendorf de 1,5 ml on a introduit 100 µl de solution à 10 mmol.1⁻¹ dans du diméthylsulfoxyde anhydre (1 µmol) de 6-aminométhylbenzylguanine et 100 µl de solution à 10 mmol.l⁻¹ dans du diméthylesulfoxyde anhydre (1 µmol) de 5,6-carboxyfluorescéine-NHS. Le mélange a été agité pendant une heure. La réaction a été suivie par HPLC sur une colonne Merck Lichrospher RP° 18 5 µm 125 x 4,6 avec un gradient d'acétonitrile dans de l'eau à 0,2% d'acide trifluoroacétique.

Une purification par HPLC préparative est effectuée sur une colonne Vydac C18 10 µm 250 x 22 avec un gradient d'acétonitrile dans de l'eau à 0,2% d'acide trifluoroacétique.

Les fractions sont collectées et concentrées sous pression réduite. On a obtenu un solide jaune (900 nmol par mesure de densité optique, 90%) correspondant au produit souhaité. MS (ES⁺) m/z : [M+H⁺] 629,1.

### Composé 3 : benzylguanine-5-Ex-fluorescéine.

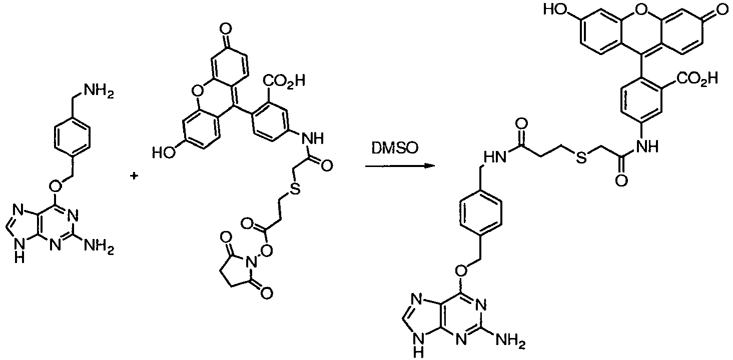

Dans un tube Eppendorf de 1,5 ml on a introduit 100 µl de solution à 10 mmol.l⁻¹ dans du diméthylsulfoxyde anhydre (1 µmol) de 6-aminométhylbenzylguanine et 100 µl de solution à 10 mmol.l⁻¹ dans du diméthylsulfoxyde anhydre (1 µmol) de 5-fluorescéine-Ex-NHS. Le mélange a été agité pendant une heure. La réaction a été suivie par HPLC sur une colonne Merck Lichrospher RP° 18 5 µm 125 x 4,6 avec un gradient d'acétonitrile dans de l'eau à 0,2% d'acide trifluoroacétique.

Une purification par HPLC préparative a été effectuée sur une colonne Vydac C18 10 µm 250 x 22 avec un gradient d'acétonitrile dans de l'eau à 0,2% d'acide trifluoroacétique.

Les fractions on été collectées et concentrées sous pression réduite. On a obtenu un solide jaune (650 nmol par mesure de densité optique, 65%) correspondant au produit souhaité. MS (ES⁺) m/z : [M+H⁺] 746,1.

### Composé 4 : benzylguanine-ATTO647N

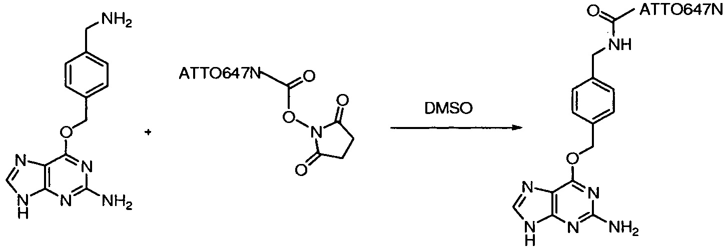

Dans un ballon de 10 ml on a introduit 1,8 mg (6,7 µmol) de 6-aminométhylbenzylguanine et 5 mg (6,6 µmol) d'ATTO647-NHS. Les produits ont été partiellement solubilisés dans 5 ml de diméthylsulfoxyde anhydre.

Le mélange a étéagité pendant une heure. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 x 22 avec un gradient d'acétonitrile dans de l'eau à 0,2% d'acide trifluoroacétique.

Les fractions ont été collectées et concentrées sous pression réduite. On a obtenu un solide bleu (3,6 µmol par mesure de densité optique, 54,5%) correspondant au produit souhaité. MS (ES⁺) m/z : [M⁺] 898.

### Composé 5 : benzylguanine-Tb(KR)

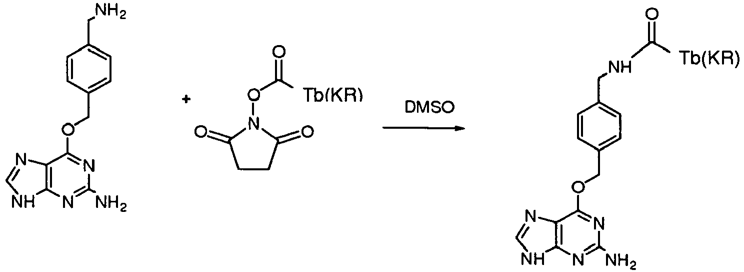

Dans un tube Eppendorf de1,5 ml on a introduit 100 µl de solution à 10 mmol.l⁻¹ dans du diméthylsulfoxyde anhydre (1 µmol) de 6-aminométhylbenzylguanine et 100 µl de solution à 10 mmol.l⁻¹ dans du diméthylsulfoxyde anhydre (1 µmol) de Tb(KR)-NHS. Le mélange a été agité pendant une heure. La réaction a été suivie par HPLC sur une colonne Merck Lichrospher RP°18 5 µm 125x 4,6 avec un gradient d'acétonitrile dans de l'eau à 25 mM d'acétate de triéthyl ammonium.

Une purification par HPLC préparative a été effectuée sur une colonne Vydac C18 10 µm 250 × 22 avec un gradient d'acétonitrile dans de l'eau à 25 mM d'acétate de triéthyl ammonium.

Les fractions ont été collectées et concentrées sous pression réduite. On a obtenu un solide blanc (650 nmol par mesure de densité optique, 65%) correspondant au produit souhaité. MS (ES⁺) m/z : [M+H⁺] 1685,6.

### Composé 6 : Acide 4-((2,2,2-trifluoroacétamido)méthyl)benzoïque

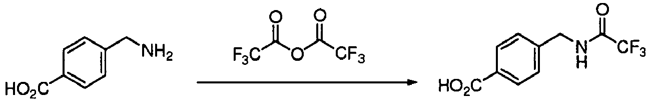

Dans un ballon de 100 ml on a pesé 2,38 g (15,7 mmol) d'acide 4-(aminométhyl) benzoïque. On a mis le ballon dans un bain d'eau et de glace et on a ajouté lentement 5,6 ml (59 mmol) d'anhydride trifluoroacétique. En fin d'addition, on a laissé le mélange réactionnel remonter à température ambiante et on a laissé sous agitation, le mélange réactionnel pendant deux heures.

On a ajouté 16,5 ml d'eau à 4°C. On a filtré le précipité sur fritté, rincé avec 2 × 3 ml d'eau à 4°C et fait séché sous vide en présence de pentoxyde de phosphore pendant 48 h.

On a obtenu un solide blanc (3,27 g - 13,2 mmol - 84%) correspondant au produit souhaité. MS (ES⁻) m/z : [M-H⁺] 246,2.

### Composé 7 : Trifluoroacétamidométhylbenzamide-BG

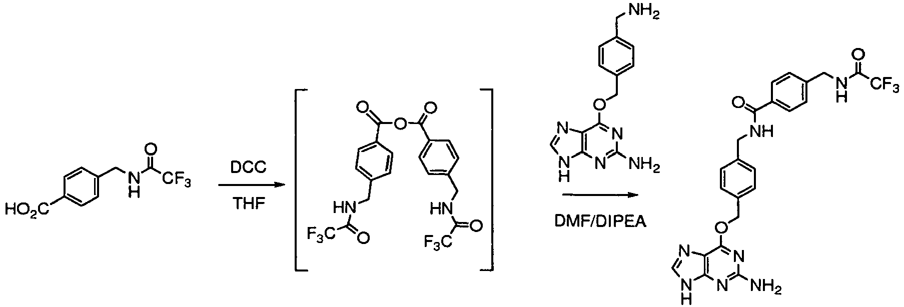

Dans un ballon de 10 ml, on a pesé 593 mg (2,4 mmol) d'acide trifluoroacétamidométhyl benzoïque. On a solubilisé cet acide dans 4 ml de tétrahydrofuranne anhydre et on a ajouté 324,4 mg (1,2 mmol) de 1,3-dicyclohexylecarbodiimide. On a laissé sous agitation, à température ambiante, pendant deux heures. Le précipité blanc de dicyclohexylurée obtenu a été filtré sur fritté.

Dans un ballon de 25 ml, on a pesé162,2 mg (0,6 mmol) de 6-aminobenzylguanine et on a ajouté 2 ml de diméthylformamide anhydre. Puis on a ajouté la solution d'anhydride trifluoroacétamidométhyle benzoïque dans le THF et 200 µl de diisopropyléthylamine. On a laissé le mélange réactionnel, sous agitation, à température ambiante pendant deux heures. La réaction a été suivie par HPLC sur une colonne Merck Lichrospher RP°18 5 µm 125x 4,6 avec un gradient d'acétonitrile dans de l'eau à 0,2% d'acide trifluoroacétique.

On a évaporé à sec, sous pression réduite, le mélange réactionnel jusqu'à l'obtention d'un solide jaune. On a trituré dans 5 ml d'acétonitrile et filtré sur fritté. On a ensuite rincé avec 2 × 1 ml d'acétonitrile et mis à sécher sous vide pendant 48 h.

On a obtenu un solide beige (289 mg - 0,58 mmol - 96%) correspondant au produit souhaité. MS (ES⁺) m/z : [M+H⁺] 500,1.

### Composé 8 : Aminométhylbenzamide-BG

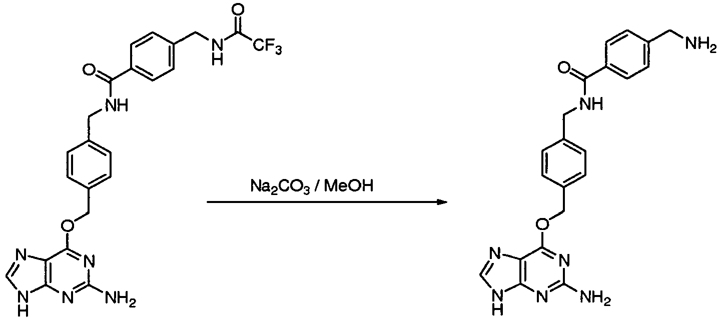

Dans un ballon de 50 ml, on a pesé 25 mg (50 µmol) de N-(4-((2-amino-9H-purin-6-yloxy)méthyl)benzyl)-4-((2,2,2- trifluoroacétamido) méthyl)benzamide. On a ajouté 10 ml de méthanol, 2 ml d'une solution aqueuse saturée en Na₂CO₃ et 4 ml d'eau. On a laissé sous agitation une nuit à température ambiante. La déprotection a été suivie par HPLC sur une colonne Merck Lichrospher RP°18 5 µm 125 × 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide blanc (17,5 mg - 43 µmol - 87%) correspondant au produit souhaité. MS (ES⁺) m/z : [M+H⁺] 404,3.

### Composé 9 : DY647-méthylbenzamide-BG

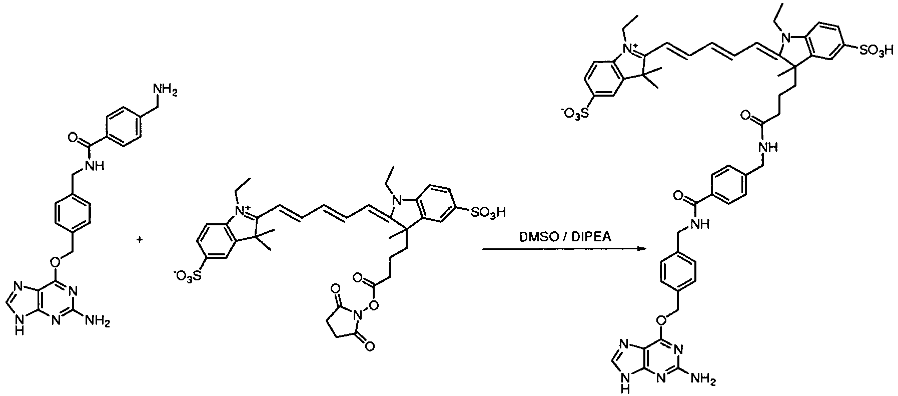

On a repris 2 µmol d'aminométhylbenzamide-BG dans 100 µl de diméthylsulfoxyde anhydre. On a ajouté 2 µmol de DY647-NHS en solution dans 300 µl de diméthylsulfoxyde anhydre et 2 µl de diisopropyléthylamine. On a laissé sous agitation pendant deux heures, à température ambiante.

La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (1 µmol par mesure de densité optique, 50%) correspondant au produit souhaité. MS (ES⁺) m/z : [M+H⁺] 1028.4.

### Composé 10 : ATTO647N-méthylbenzamide-BG

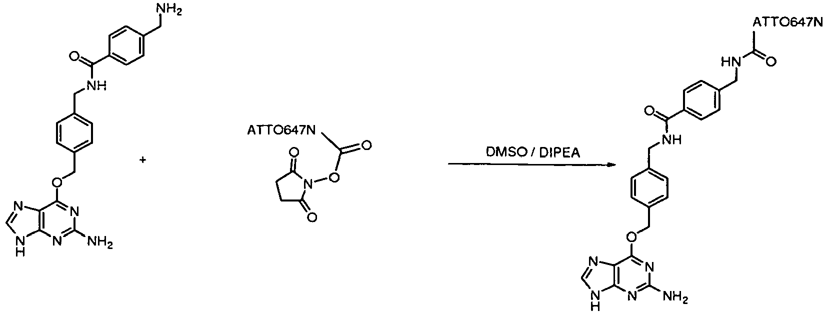

On a repris 200 nmol d'aminométhylbenzamide-BG dans 100 µl de diméthylsulfoxyde anhydre. On a ajouté 200 nmol de ATTO647N-NHS en solution dans 20 µl de diméthylsulfoxyde anhydre et 1 µl de diisopropyléthylamine. On a laissé le mélange réactionnel sous agitation pendant deux heures, à température ambiante.

La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (149 nmol par mesure de densité optique, 74%) correspondant au produit souhaité. MS (ES⁺) m/z : [M⁺] 1031,5.

### Composé 11 : 5,6-carboxyfluoresceine-méthylbenzamide-BG

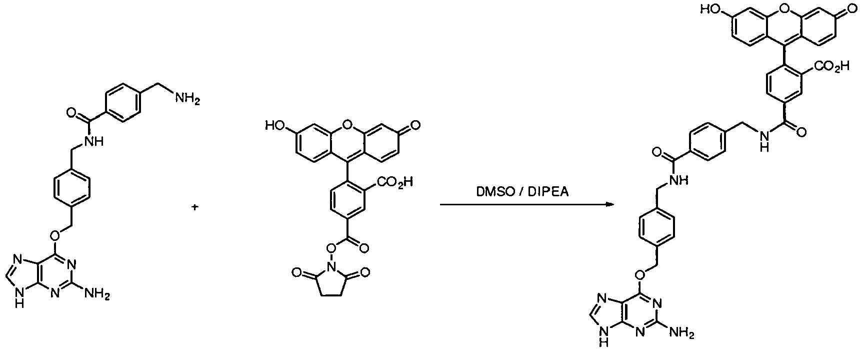

On a repris 1 µmol d'aminométhylbenzamide-BG dans 100 µl de diméthylsulfoxyde (DMSO) anhydre. On a ajouté 1 µmol de 5,6-carboxyfluoresceine-NHS en solution dans 50 µl de diméthylsulfoxyde anhydre et 2 µL de diisopropyléthylamine. On a laissé le mélange réactionnel sous agitation pendant une heure, à température ambiante.

La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide jaune (600 nmol par mesure de densité optique, 60%) correspondant au produit souhaité. MS (ES⁺) m/z : [M+H⁺] 762,1.

### Composé 12 : 5-Ex-luoresceine-Benzylamide-BG

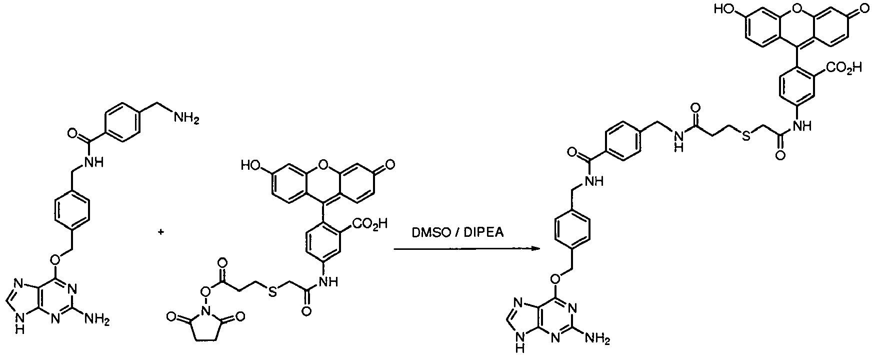

On a repris 1 µmol d'aminométhylbenzamide-BG dans 100 µl de diméthylsulfoxyde anhydre. On a ajouté 1 µmol de 5-fluoresceine-Ex-NHS en solution dans 40 µl de diméthylsulfoxyde anhydre et 2 µl de diisopropyle amine. On a laissé le mélange réactionnel sous agitation pendant une heure, à température ambiante.

La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide jaune (400 nmol par mesure de densité optique, 40%) correspondant au produit souhaité. MS (ES⁺) m/z : [M+H⁺] 879,3.

### Composé 13 : Tb(KR)-méthylbenzamide-BG

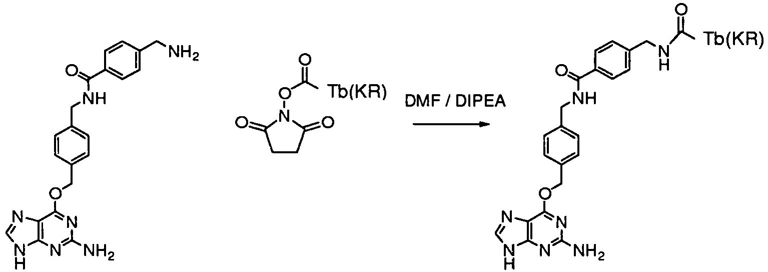

Dans un ballon de 10 ml, on a pesé 2,3 mg (8,5 µmol) d'aminométhylbenzamide-BG dans 2 ml de diméthylformamide anhydre. On a ajouté 6 µmol de Tb(KR)-NHS en solution dans 2 ml de diméthylformamide anhydre et 20 µl de diisopropyle amine. On a laissé le mélange réactionnel sous agitation pendant deux heures, à température ambiante.

La réaction a été suivie par HPLC sur une colonne Merck Lichrospher RP°18 5 µm 125x 4,6 avec un gradient d'acétonitrile dans de l'eau 25 mM acétate de triéthyle ammonium pH 6. On a évaporé le DMF sous pression réduite et on a repris le résidu dans de l'eau.

Une purification par HPLC préparative a été effectuée sur une colonne Vydac C18 10 µm 250 × 22 avec un gradient d'acétonitrile dans l'eau 25 mM acétate de triéthyle ammonium pH 6.

Les fractions ont été collectées et concentrées sous pression réduite. On a obtenu un solide blanc (2,2 µmol par mesure de densité optique, 36%) correspondant au produit souhaité. MS (ES⁺) m/z : [M+H⁺] 1819,3.

### Composé 14 : Préparation du BG-SH

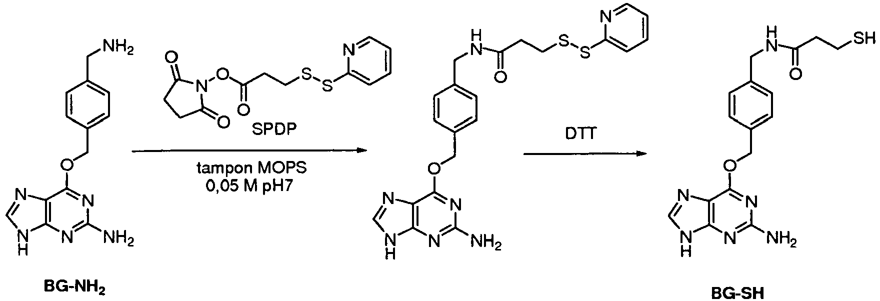

Dans un ballon de 10 ml on a pesé 0,8 mg (3 µmol) de 6-aminométhylbenzylguanine que l'on a solubilisé dans 200 µl de diméthylformamide anhydre et 1,5 ml de tampon MOPS 0,05 M pH 7. On a jouté 1,1 mg (3,6 µmol) de SPDP. On a laissé le mélange réactionnel, sous agitation, pendant une heure. La réaction a été suivie par HPLC sur une colonne Merck Lichrospher RP°18 5 µm 125x 4,6 avec un gradient d'acétonitrile dans de l'eau à 0,2% d'acide trifluoroacétique.

On a ajouté au mélange réactionnel 2,2 mg (14,4 µmol) de DTT et laissé celui-ci sous agitation pendant deux heures. La réaction a été suivie par HPLC.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide blanc (2,1 µmol par mesure de densité optique, 70%) correspondant au produit souhaité. MS (ES⁺) m/z : [M+H⁺] 358,9.

### Composé 15 : BG-méthylbenzamide-SH

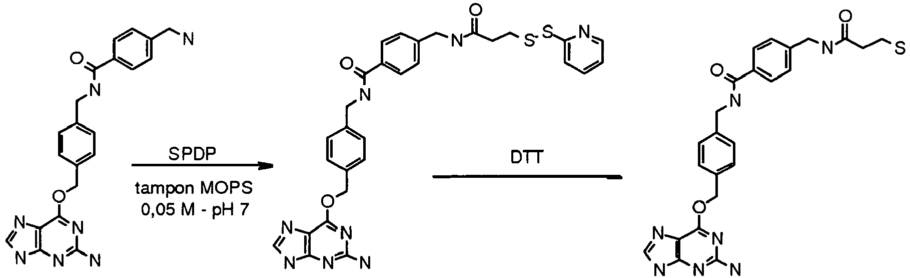

Dans un ballon de 10 ml, on a repris 4,5 µmol d'aminométhylbenzamide-benzylguanine dans 200 µl de diméthyleformamide anhydre et 1,5 ml de tampon MOPS 0,05 M pH 7. On a ajouté 1,7 mg (5,4 µmol) de SPDP. On a laissé le mélange réactionnel, sous agitation, pendant une heure. La réaction a été suivie par HPLC sur une colonne Merck Lichrospher RP°18 5 µm 125 × 4,6 avec un gradient d'acétonitrile dans de l'eau à 0,2% d'acide trifluoroacétique.

On a ajouté au mélange réactionnel 2,5 mg (16,2 µmol) de DTT et on l'a laissé sous agitation pendant deux heures. La réaction a été suivie par HPLC.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide blanc (3 µmol par mesure de densité optique, 66%) correspondant au produit souhaité. MS (ES⁺) m/z : [M+H⁺] 492,2.

### Composé 16 : acide 2-(trifluoroacétamidométhyl)nicotinique

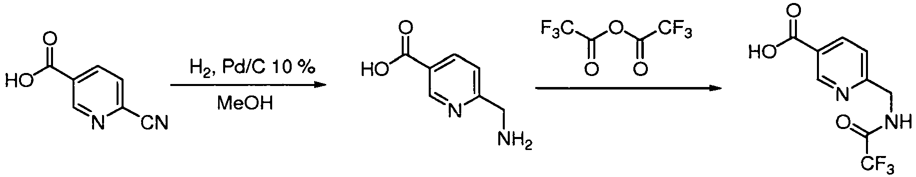

Dans un ballon de 100 ml, on a pesé 500 mg (3,58 mmol) d'acide 6-cyanopyridine-3-carboxylique et solubiliser dans 50 ml de méthanol. On a ensuite ajouté 30 mg de palladium à 10 % sur du charbon et de l'hydrogène sous une atmosphère pendant quatre heures. La réaction est suivie par HPLC sur une colonne Merck Lichrospher RP°18 5 µm 125 × 4,6 avec un gradient d'acétonitrile dans de l'eau à 0,2% d'acide trifluoroacétique. Le produit formé a précipité.

On a ajouté 40 ml d'eau déminéralisée au mélange réactionnel, puis on a filtré sur de la célite le palladium et concentré le mélange réactionnel sous pression réduite dans un ballon de 50 ml. On a mis le ballon dans un bain de glace/eau et on a ajouté lentement 4 ml d'anhydride trifluoroacétique. On a laissé le mélange réactionnel remonter à température ambiante et sous agitation pendant deux heures. La réaction a été suivie par HPLC.

On a ajouté au mélange réactionnel 20 ml d'éther et on l'a mis dans un bain glace/eau pendant 20 min. On a filtré sur fritté le précipité et on l'a mis à sécher sous vide pendant 48 h.

On a obtenu un solide blanc (450 mg - 1,81 mmol - 54%) correspondant au produit souhaité. MS (ES⁺) m/z : [M+H⁺] 249.

### Composé 17 : acide 2-(trifluoroacétamidométhyl)isonicotinique

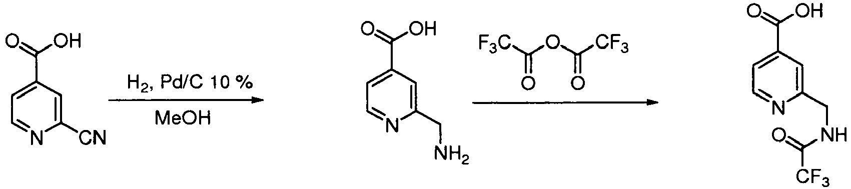

Dans un ballon de 50 ml, on a pesé 100 mg (675 µmol) d'acide 6-cyanopyridine-4-carboxylique et on l'a solubilisé dans 30 ml de méthanol. On a ensuite ajouté 10 mg de palladium à 10 % sur du charbon et de l'hydrogène sous une atmosphère pendant quatre heures. La réaction a été suivie par HPLC sur une colonne Merck Lichrospher RP°18 5 µm 125 x 4,6 avec un gradient d'acétonitrile dans de l'eau à 0,2% d'acide trifluoroacétique. Le produit formé a précipité.

On a ajouté 20 ml d'eau déminéralisée au précipité, filtré sur de la célite le palladium et concentré sous pression réduite dans un ballon de 50 ml. On a mis le ballon dans un bain de glace/eau et ajouté lentement 3 ml d'anhydride trifluoroacétique. On a laissé la température du mélange réactionnel remonter à la température ambiante et on l'a maintenu sous agitation pendant deux heures. La réaction a été suivie par HPLC.

On a ajouté au mélange réactionnel 10 ml d'éther et on l'a mis dans un bain glace/eau pendant 20 min. On a filtré sur fritté le précipité et on l'a mis à sécher sous vide pendant 48 h.

On a obtenu un solide blanc (110 mg - 443 µmol - 67%) correspondant au produit souhaité. MS (ES⁺) m/z : [M+H⁺] 249.

### Composé 18 : DY647-aminométhylisonicotinamide-benzylguanine

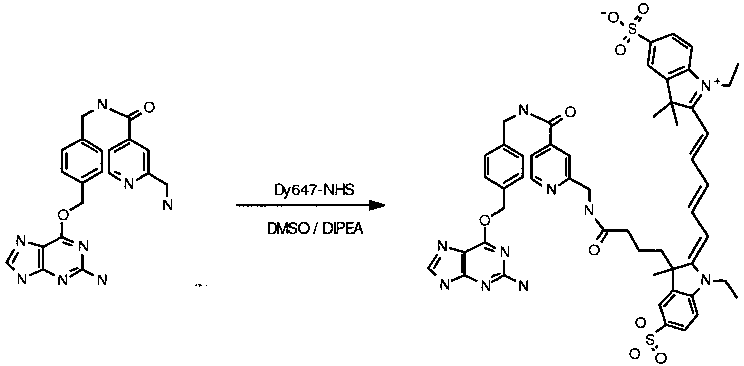

Dans un tube eppendorf de 1,5 ml contenant 1 µmol d'aminométhylisonicotinamide-benzylguanine on a ajouté une solution de 1 µmol de Dy647-NHS dans 100 µl de diméthylsulfoxyde anhydre et 2 µl de diisopropyléthylamine. On a laissé le mélange réactionnel sous agitation pendant une heure. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (470 nmol par mesure de densité optique, 47%) correspondant au produit souhaité. MS (ES⁺) m/z : [M+H⁺] 1029,3.

### Composé 19 : DY647-aminométhylnicotinamide-benzylguanine

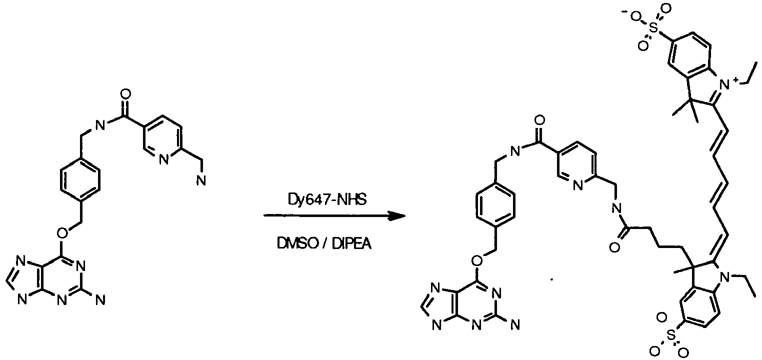

Dans un tube eppendorf de 1,5 ml contenant 1 µmol d'aminométhylnicotinamide-benzylguanine ajouter une solution de 1 µmol de Dy647-NHS dans 100 µl de diméthylsulfoxyde anhydre et 2 µl de diisopropyléthylamine. On a laissé le mélange réactionnel sous agitation pendant une heure. La réaction a été suivie par HPLC sur une colonne Merck Lichrospher RP°18 5 µm 125 × 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (540 nmol par mesure de densité optique, 54%) correspondant au produit souhaité. MS (ES⁺) m/z : [M+H⁺] 1029,3.

### Composé 20 : Tb(KR)-aminométhylnicotinamide-benzylguanine :

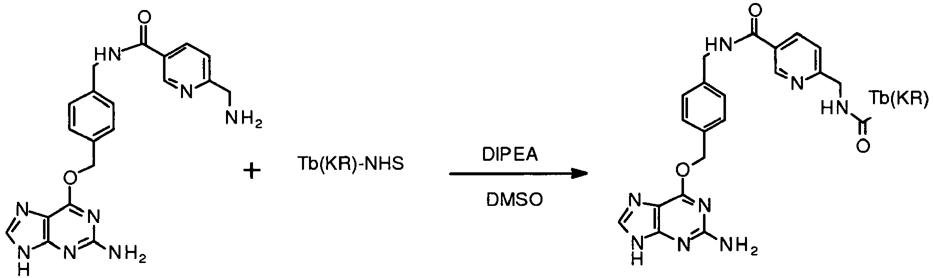

Dans un tube eppendorf de 1,5 ml contenant 1 µmol d'aminométhylnicotinamide-benzylguanine on a ajouté une solution de 1 µmol de Tb(KR)-NHS dans 100 µl de diméthylsulfoxyde anhydre et 2 µl de diisopropyléthylamine. On a laissé le mélange réactionnel sous agitation pendant une heure. La réaction a été suivie par HPLC sur une colonne Merck Lichrospher RP°18 5 µm 125 × 4,6 avec un gradient d'acétonitrile dans de l'eau 25 mM acétate de triéthyle ammonium pH 6.

Une purification par HPLC préparative a été effectuée sur une colonne Vydac C18 10 µm 250 × 22 avec un gradient d'acétonitrile dans de l'eau à 25 mM d'acétate de triéthylammonium pH 6.

Les fractions ont été collectées et concentrées sous pression réduite. On a obtenu un solide bleu (400 nmol par mesure de densité optique, 40%) correspondant au produit souhaité. MS (ES⁺) m/z : [M+H⁺] 1820,3 - [M+2H⁺] / 2 = 910,5.

### Composé 21 : benzylguanine-N-Fmoc-pipéridine

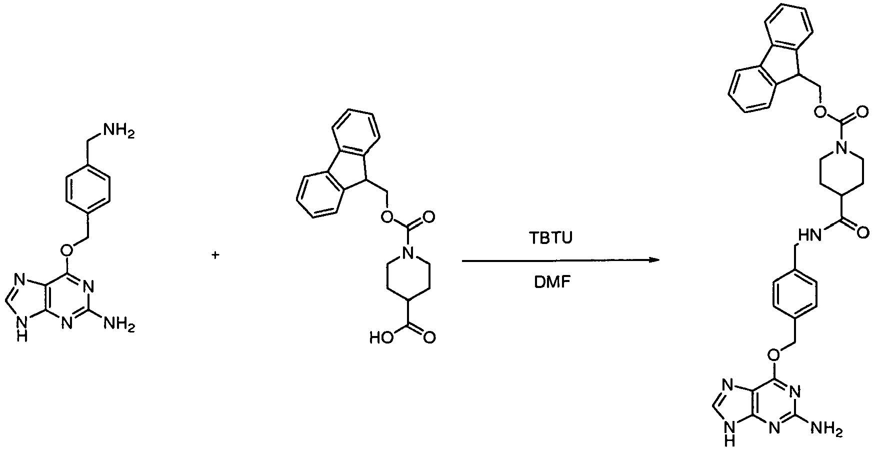

Dans un ballon de 50 ml, on a pesé 48,1 mg (178 µmol) de 6-aminométhylbenzylguanine et on l'a solubilisé partiellement dans 10 ml de diméthylformamide anhydre. On a ajouté 62,5 mg (178 µmol) de 4-carboxy-N-Fmoc-pipéridine et 57,1 mg (178 µmol) de TBTU. On a laissé le mélange réactionnel sous agitation pendant deux heures. La réaction a été suivie par HPLC sur une colonne Merck Lichrospher RP°18 5 µm 125 × 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide blanc (38 mg, 35%) correspondant au produit souhaité. MS (ES⁺) m/z : [M+H⁺] 604,5.

### Composé 22 : benzylguanine-pipéridine

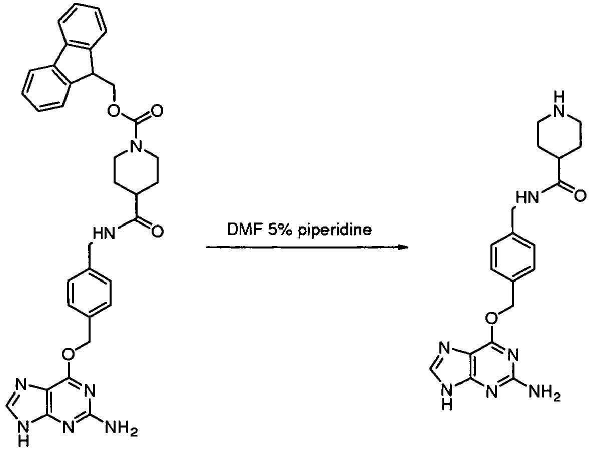

Dans un tube eppendorf de 1,5 ml, on a pesé 1,8 mg (3 µmol) de benzylguanine-N-Fmoc-pipéridine et on l'a solubilisé dans 190 µl de diméthylformamide anhydre. On a ajouté 10 µl de pipéridine et On a laissé le mélange réactionnel sous agitation pendant une heure. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide blanc correspondant au produit souhaité. Il a été directement utilisé dans la réaction suivante sans mesure de rendement. MS (ES⁺) m/z : 382,3.

### Composé 23 : DY647-pipéridine-benzylguanine

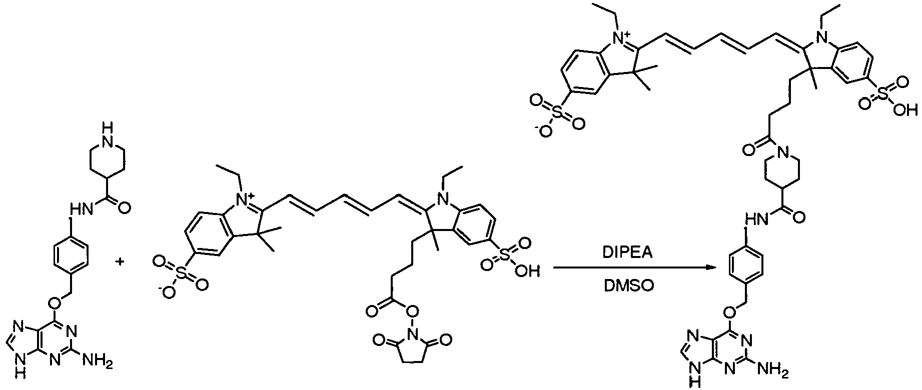

Dans un ballon de 25 ml contenant 1,8 µmol de benzylguanine-pipéridine, on a ajouté 800 µl de diméthylsulfoxyde anhydre, 4µl de diisopropyléthylamine et 0,14 mg (1,8 µmol) de Dy647-NHS. On a laissé le mélange réactionnel sous agitation pendant une heure. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (700 nmol par mesure de densité optique - 40%) correspondant au produit souhaité. MS (ES⁺) m/z : 1006,75.

### Composé 24 : benzylguanine-trans-4-(Fmoc-amino)-cyclohexane :

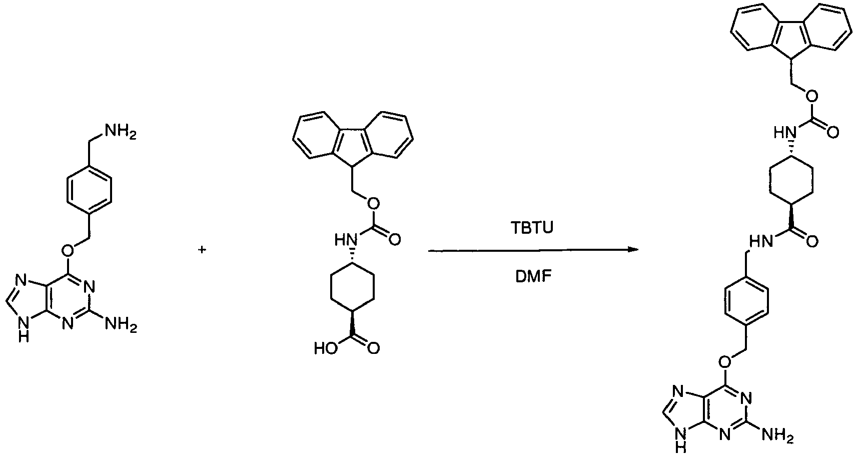

Dans un ballon de 50 ml, on a pesé 51 mg (189 µmol) de 6-aminométhylbenzylguanine et on l'a solubilisé partiellement dans 20 ml de diméthylformamide anhydre. On a ajouté 69 mg (189 µmol) de trans-4-(Fmoc-amino)-carboxycyclohexane et 60,7 mg (189 µmol) de TBTU. On a laissé le mélange réactionnel sous agitation pendant deux heures. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 x 22 avec un gradient d'acétonitrile dans de l'eau à 0,2% d'acide trifluoroacétique.

Les fractions ont été collectées et concentrées sous pression réduite. On a obtenu un solide blanc (16 mg, 13,7%) correspondant au produit souhaité. MS (ES⁺) m/z : [M+H⁺] 618 ,6.

### Composé 25 : benzylguanine-trans-4-amino-cyclohexane

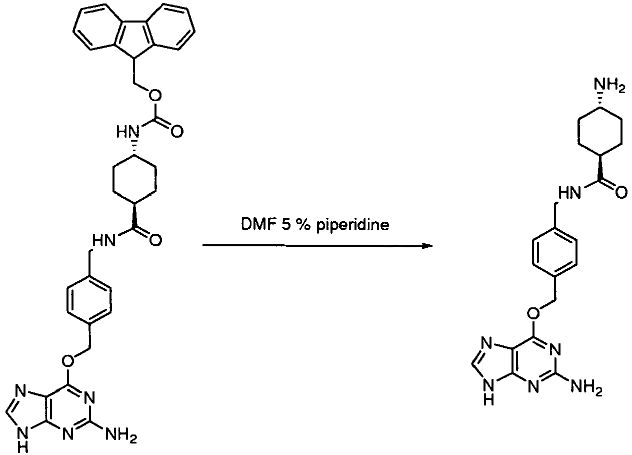

Dans un tube eppendorf de 1,5 ml, on a pesé 1,2 mg (2 µmol) de benzylguanine-trans-4-(Fmoc amino)-cyclohexane et on l'a solubilisé dans 190 µl de diméthylformamide anhydre. Ajouter 10 µl de pipéridine et on a laissé le mélange réactionnel sous agitation pendant une heure. La réaction a été suivie par HPLC sur une colonne Merck Lichrospher RP°18 5 µm 125 × 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide blanc correspondant au produit souhaité. Il a été directement utilisé dans la réaction suivante sans mesure de rendement. MS (ES⁺) m/z : 396,3.

### Composé 26 : DY647-trans-cyclohexane-benzylguanine

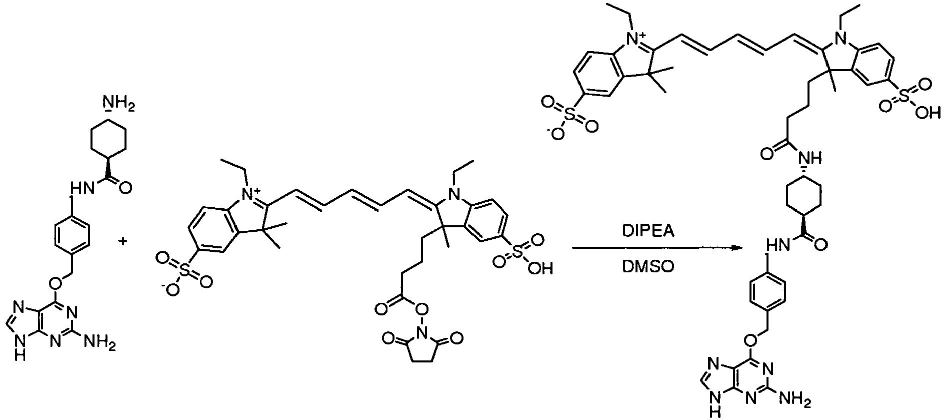

Dans un ballon de 25 ml contenant 2 µmol de benzylguanine-trans-4-amino-cyclohexane on a ajouté 800 µl de diméthylsulfoxyde anhydre, 4µl de diisopropyléthylamine et 0,14 mg (1,8 µmol) de DY647-NHS. On a laissé le mélange réactionnel sous agitation pendant une heure. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (600 nmol par mesure de densité optique - 33%) correspondant au produit souhaité. MS (ES⁺) m/z : 1020,8.

### Composé 27 : benzylguanine-cis-4-(Fmoc-amino)-cyclohexane

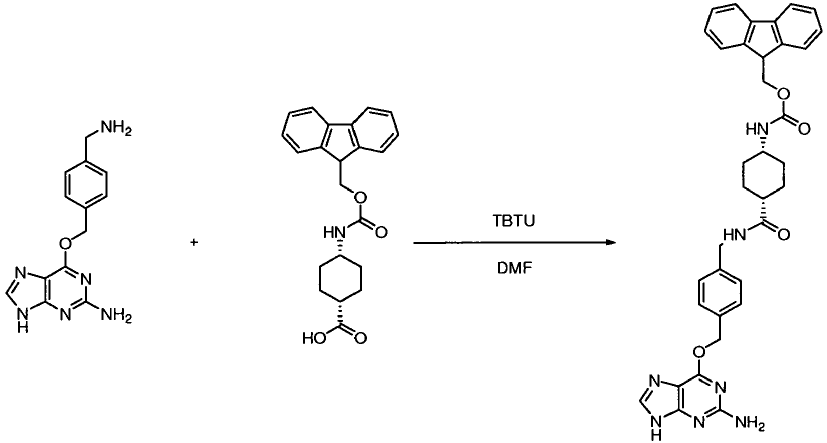

Dans un ballon de 50 ml, on a pesé 61,3 mg (226 µmol) de 6-aminométhylbenzylguanine et on l'a solubilisé partiellement dans 20 ml de diméthylformamide anhydre. On a ajouté 82,7 mg (226 µmol) cis-4-(Fmoc-amino)-carboxy-cyclohexane et 72,8 mg (226 µmol) de TBTU. On a laissé le mélange réactionnel sous agitation pendant deux heures. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 x 22 avec un gradient d'acétonitrile dans de l'eau à 0,2% d'acide trifluoroacétique.

Les fractions ont été collectées et concentrées sous pression réduite. On a obtenu un solide blanc (28 mg, 20%) correspondant au produit souhaité. MS (ES⁺) m/z : [M+H⁺] 618 ,6.

### Composé 28 : benzylguanine-cis-4-amino-cyclohexane

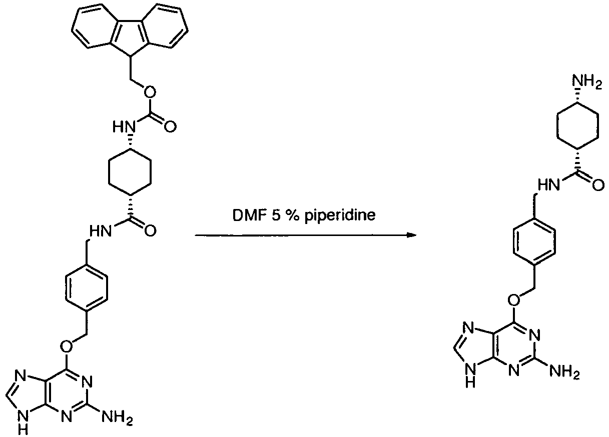

Dans un tube eppendorf de 1,5 ml, on a pesé 0,7 mg (1,1 µmol) de benzylguanine-cis-4-(Fmoc-amino)-cyclohexane et on l'a solubilisé dans 190 µl de diméthylformamide anhydre.

On a ajouté 10 µl de pipéridine et on a laissé le mélange réactionnel sous agitation pendant une heure. La réaction a été suivie par HPLC sur une colonne Merck Lichrospher RP°18 5 µm 125 × 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide blanc, correspondant au produit souhaité. Il a été directement utilisé dans la réaction suivante sans mesure de rendement. MS (ES⁺) m/z : [M+H⁺] 396,3.

### Composé 29 : DY647-cis-cyclohexane-benzylguanine

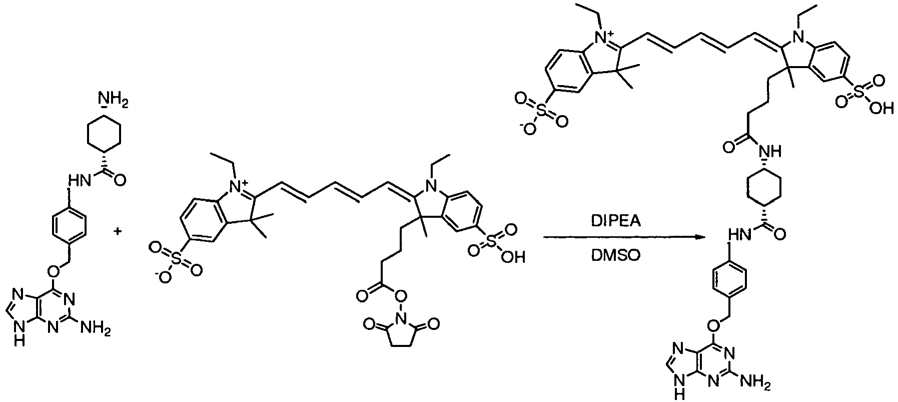

Dans un ballon de 25 ml contenant 1 µmol de benzylguanine-cis-4-amino-cyclohexane, on a ajouté 800 µl de diméthylsulfoxyde anhydre, 4µl de diisopropyléthylamine et 0,7 mg (1 µmol) de DY647-NHS et on a laissé le mélange réactionnel sous agitation pendant une heure. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (400 nmol par mesure de densité optique - 40%) correspondant au produit souhaité. MS (ES⁺) m/z : 1020,8.

### Composé 30 : Benzylcytosine-DY647

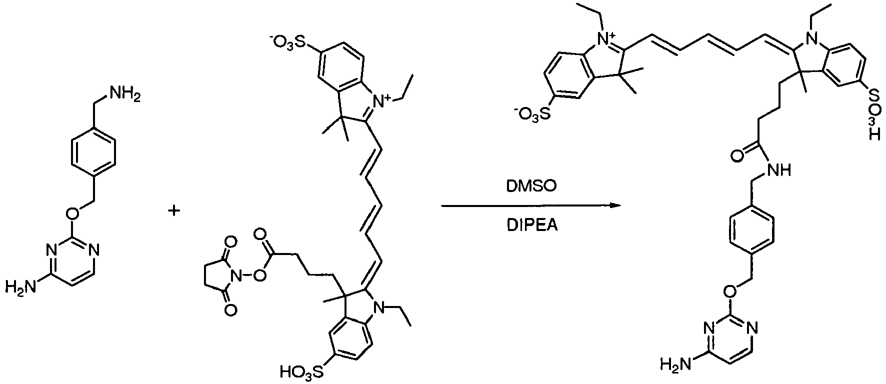

Dans un tube eppendorf de 1,5 ml, on a ajouté 1 µmol d'aminométhylbenzylcytosine en solution dans 26 µl de diméthylsulfoxyde anhydre, 1 µmol de DY647-NHS en solution dans 76 µl de diméthylsulfoxyde anhydre et 4 µl de diisopropyléthylamine.

Le mélange réactionnel a été agité pendant une heure. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (600 nmol par mesure de densité optique, 60%) correspondant au produit souhaité. MS (ES+) m/z : [M+H+] 855,6 [M+Na+] 877,6.

### Composé 31 : Benzylcytosine-Tb(KR)

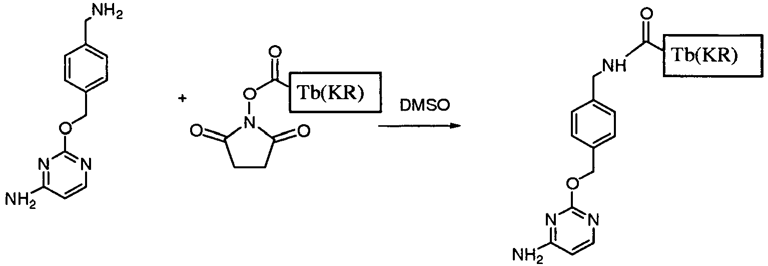

Dans un tube eppendorf de 1,5 ml, on a ajouté 1 µmol d'aminométhylbenzylcytosine en solution dans 26 µl de diméthylsulfoxyde anhydre, 1 µmol de Tb(KR)-NHS en solution dans 110 µl de diméthylsulfoxyde anhydre et 4 µl de diisopropyléthylamine.

Le mélange réactionnel a été agité pendant deux heures. La réaction a été suivie par HPLC sur une colonne Merck Lichrospher RP°18 5 µm 125 × 4,6 avec un gradient d'acétonitrile dans de l'eau à 25 mM d'acétate de triéthylammonium.

Une purification par HPLC préparative a été effectuée sur une colonne Vydac C18 10 µm 250 × 22 avec un gradient d'acétonitrile dans de l'eau à 25 mM d'acétate de triéthylammonium.

Les fractions ont été collectées et concentrées sous pression réduite. On a obtenu un solide blanc (350 nmol par mesure de densité optique, 35%) correspondant au produit souhaité. MS (ES+) m/z : [M+H+] 1645,2 [M+2H+]/2 823,4.

### Composé 32 : Aminométhylbenzamide-BC

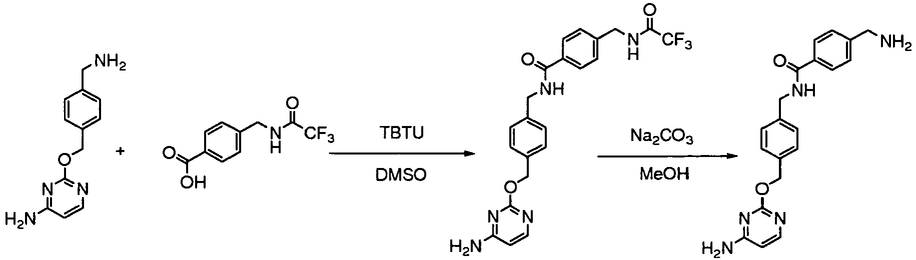

Dans un tube eppendorf de 2 ml, on a pesé 6 mg (26 µmol) d'aminométhylbenzylcytosine et 6,4 mg (26 µmol) d'acide trifluoroacétamidométhyl benzoïque. On a solubilisé le mélange dans 600 µl de diméthylsulfoxyde anhydre et on a ajouté 9,2 mg (28,6 µmol) de TBTU et 8 µl de diisopropyléthylamine. Le mélange réactionnel a été agité pendant deux heures. La réaction a été suivie par HPLC sur une colonne Merck Lichrospher RP°18 5 µm 125x 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

On a évaporé à sec sous pression réduite le mélange réactionnel jusqu'à obtention d'un solide blanc ;a été repris dans 2 ml de méthanol, on a ajouté 2 ml de solution aqueuse saturée en Na2CO3 et 4 ml d'eau. Le mélange a été agité toute une nuit. La déprotection a été suivie par HPLC.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide blanc (8,5 µmol par mesure de densité optique, 32%) correspondant au produit souhaité. MS (ES+) m/z : [M+H+] 364,4.

### Composé 33 : DY647-méthylbenzamide-BC :

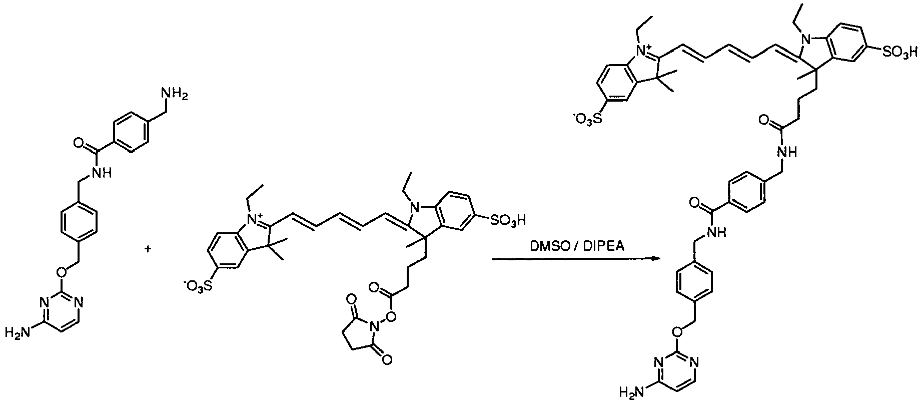

Dans un tube eppendorf de 1,5 ml contenant 1 µmol d'aminométhylbenzamide-BC, on a ajouté 1 µmol de DY647-NHS en solution dans 140 µl de diméthylsulfoxyde anhydre et 4 µl de diisopropyléthylamine.

Le mélange réactionnel a été agité pendant une heure. La réaction a été suivie par HPLC sur une colonne Merck Lichrospher RP°18 5 µm 125 × 4,6 avec un gradient d'acétonitrile dans de l'eau à 0,2% d'acide trifluoroacétique.

Une purification par HPLC préparative a été effectuée sur une colonne Vydac C18 10 µm 250 × 22 avec un gradient d'acétonitrile dans de l'eau à 0,2% d'acide trifluoroacétique.

Les fractions ont été collectées et concentrées sous pression réduite. On a obtenu un solide bleu (400 nmol par mesure de densité optique, 40%) correspondant au produit souhaité. MS (ES+) m/z : [M+H+] 988,6 [M+Na+] 1010,6 [M+K+] 1026,6.

### Composé 34 : Tb(KR)-méthylbenzamide-BC:

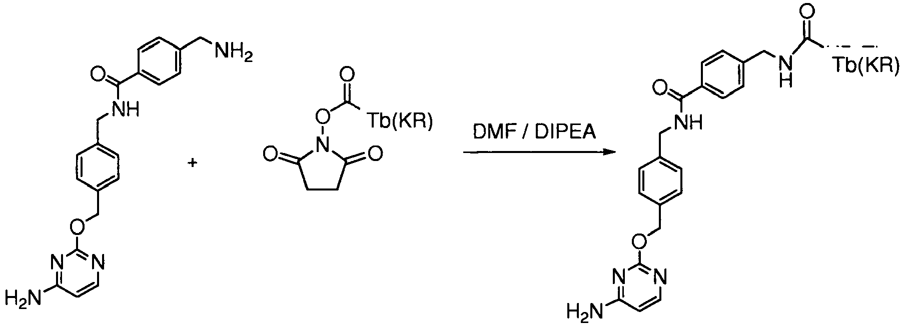

Dans un tube eppendorf de 1,5 ml contenant 1 µmol d'aminométhylbenzamide-BC, on a ajouté 1 µmol de Tb(KR)-NHS en solution dans 120 µl de diméthylsulfoxyde anhydre et 4 µl de diisopropyléthylamine.

Le mélange réactionnel a été agité pendant une heure. La réaction a été suivie par HPLC sur une colonne Merck Lichrospher RP°18 5 µm 125x 4,6 avec un gradient d'acétonitrile dans de l'eau à 25 mM d'acétate de triéthylammonium.

Une purification par HPLC préparative a été effectuée sur une colonne Vydac C18 10 µm 250 × 22 avec un gradient d'acétonitrile dans de l'eau à 25 mM d'acétate de triéthyle ammonium.

Les fractions ont été collectées et concentrées sous pression réduite. On a obtenu un solide blanc (270 nmol par mesure de densité optique, 27%) correspondant au produit souhaité. MS (ES+) m/z : [M+H+] 1779,3 [M+2H+]/2 890.

### Composé 35 : Atto465-BG

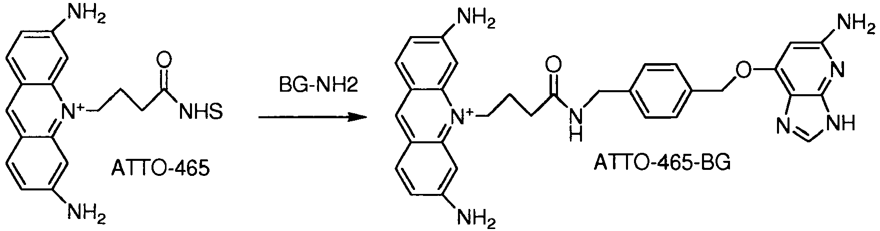

Dans un tube eppendorf de 1,5 ml on a introduit une solution de BG-NH2 (0.3 µmoles dissoutes dans 100µl de DMSO anhydre), une solution d'ATTO-465-NHS (0.254 µmoles dissoutes dans 100µl de DMSO anhydre), ainsi que 3 µl de DIPEA. On a laissé le mélange réactionnel sous agitation à température ambiante pendant 1,5 heures. La réaction a été suivie par HPLC sur une colonne Xbridge C18, 3,5 µm, 4,6x100 mm avec un gradient d'acétonitrile dans de l'eau à 0,2% d'acide trifluoroacétique.

Une purification par HPLC préparative a été effectuée sur une colonne Xbridge C18, OBDTM, 19x100 mm avec un gradient d'acétonitrile dans de l'eau à 0,2% d'acide trifluoroacétique.

Les fractions ont été collectées et concentrées sous pression réduite. On a obtenu un solide jaune-orange (0,128 µmoles, rendement de 50%) correspondant au produit souhaité. MS (ES+) m/z : [M+H⁺] 548

### Composé 36 : Atto465-méthylbenzamine-BG:

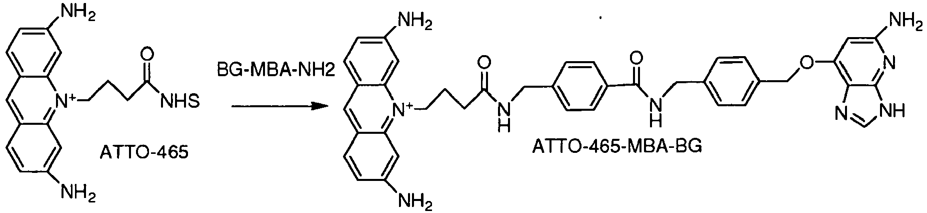

Dans un tube eppendorf de 1,5 ml on a introduit une solution de BG-MBA-NH2 (0.3 µmoles de composé 8 dissoutes dans 100µl de DMSO anhydre), une solution d'ATTO-465-NHS (0.254 µmoles dissoutes dans 100µl de DMSO anhydre), ainsi que 3 µl de DIPEA. On a laissé le mélange réactionnel sous agitation à température ambiante pendant 1,5 heures. La réaction a été suivie par HPLC sur une colonne Xbridge C18, 3,5 µm, 4,6x100 mm avec un gradient d'acétonitrile dans de l'eau à 0,2% d'acide trifluoroacétique.

Une purification par HPLC préparative a été effectuée sur une colonne XbridgeTM C18, OBDTM, 19x100 mm avec un gradient d'acétonitrile dans de l'eau à 0,2% d'acide trifluoroacétique.

Les fractions ont été collectées et concentrées sous pression réduite. On a obtenu un solide jaune-orange (0,205 µmoles, rendement de 80%) correspondant au produit souhaité. MS (ES+) m/z : [M+H+] 681

## Revendications

1. Composé de formule (I') : 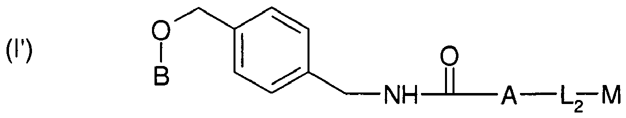 dans laquelle :
B représente un groupe de formule (II') ou (III') suivante : 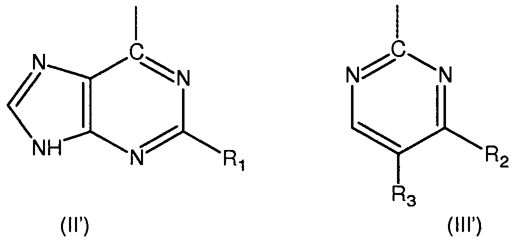 dans lesquelles les lignes pointillées représentent les liaisons avec l'oxygène de la formule (I),
R₁ est choisi parmi : H, NH₂, un groupe OH ou un groupe oxo,
R₂ est choisi parmi : NH₂, OH ou oxo,
R₃ est choisi parmi : H ou un groupe CH₃;
L₂ est choisi parmi les groupes suivants : 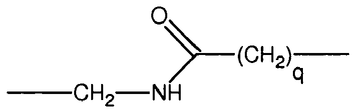 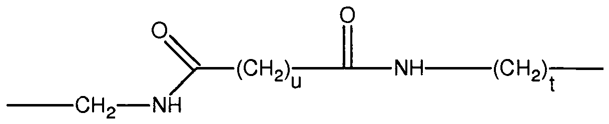 dans lesquels q, u, t dont des nombres entiers compris entre 1 et 10 ;
M est un composé luminescent, un fluorophore ou un groupe réactif choisi parmi les groupes suivants : un acrylamide, une amine activée, un ester activé, un aldéhyde, un halogénure d'alkyle, un anhydride, une aniline, un azide, une aziridine, un acide carboxylique, un diazoalcane, une haloacétamide, une halotriazine, une hydrazine), un imido ester, un isocyanate, un isothiocyanate, un maléimide, un halogénure de sulfonyle, ou un thiol, une cétone, une amine, un halogénure d'acide, un hydroxysuccinimidyl ester, un hydroxysulfosuccinimidyl ester, un azidonitrophényl, un azidophényl, une 3-(2-pyridyl dithio)-propionamide, glyoxal et en particulier les groupes de formule : 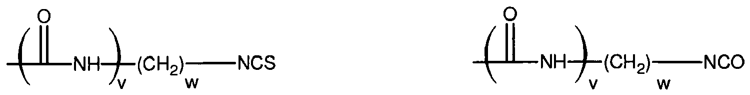 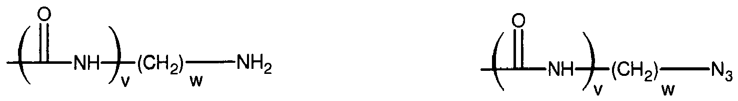 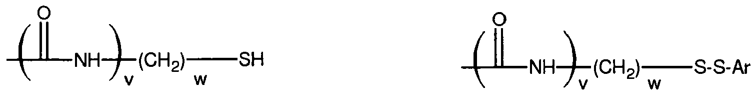 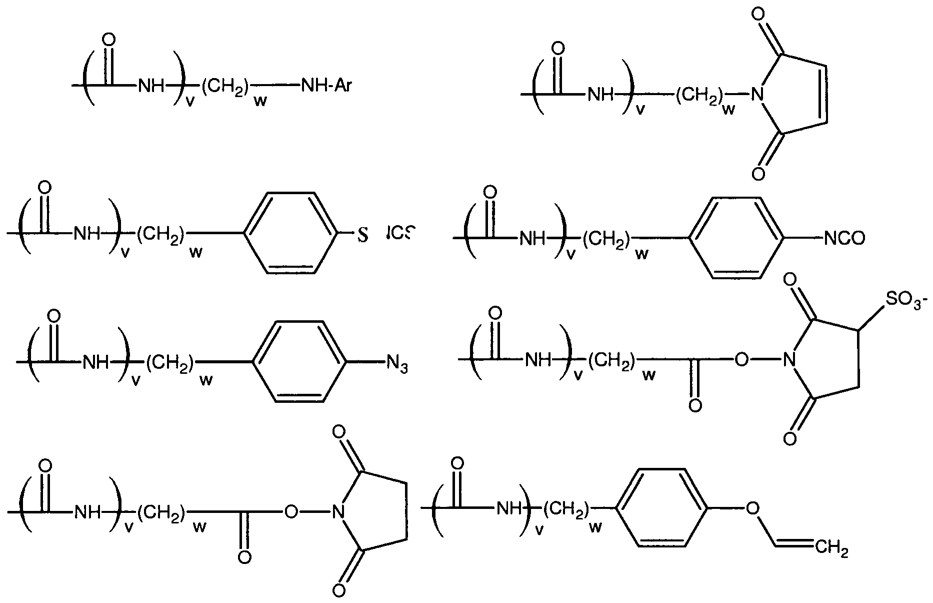 dans lesquels w varie de 0 à 8 et v est égal à 0 ou 1, et Ar est un hétérocycle à 5 ou 6 chaînons liés par des liaisons saturées ou insaturées, comprenant 1 à 3 hétéroatomes, éventuellement substitué par un atome d'halogène ;
A est choisi parmi les groupes divalents suivants :

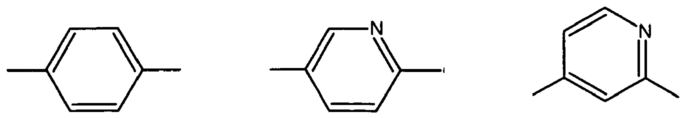

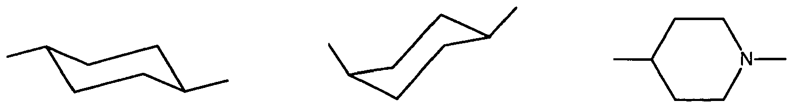

2. Composé selon la revendication 1, **caractérisé en ce que** B est un groupe de formule (II') et **en ce que** R₁ est un groupe NH₂.

3. Composé selon la revendication 1, **caractérisé en ce que** B est un groupe de formule (III') et **en ce que** R₂ est un groupe NH₂ et R₃ est H.

4. Composé selon l'une quelconque des revendications 1 à 3, **caractérisé en ce que** M est choisi parmi les composés luminescents ou les fluorophores suivants :
- les *fluorophores organiques* tel que les cyanines et leurs dérivés, la fluorescéine et ses dérivés, la coumarine et ses dérivés, la rhodamine et ses dérivés, la carbopyronine et ses dérivés, l'oxazine et ses dérivés, les AlexaFluors, le crystal violet, et ses dérivés, les perylènebisimide et ses dérivés, les squaraines, les BODIPYs, le NBD (nitrobenzoxadiazole) et ses dérivés, le DABCYL (acide 4-((4-(diméthylamino) phényl) azo) benzoïque) et ses dérivés ;
- les *fluorophores protéiques* tels que la GFP et ses variants, les protéines fluorescentes extraites de coraux, les phycobiliprotéines, telles que la B-phycoérythrine, la R-phycoérythrine, la C-phycocyanine, les allophycocyanines, en particulier celle connues sous la dénomination XL665 ;
- les *complexes de lanthanides fluorescents* tels que les cryptates de lanthanides, les chélates de lanthanides (en particulier les chélates et cryptates d'europium, de terbium, de samarium, de dysprosium, de néodymium).

5. Composé selon l'une quelconque des revendications 1 à 4, **caractérisé en ce que** M est un groupe réactif choisi parmi les groupes suivants : un acide carboxylique, une amine, un succinimidyl ester d'acide carboxylique, un haloacétamide, une hydrazine, un isothiocyanate, un group maléimide, une amine aliphatique.

6. Composé selon l'une quelconque des revendications 1 à 4, **caractérisé en ce qu'**il répond à l'une des formules suivantes :

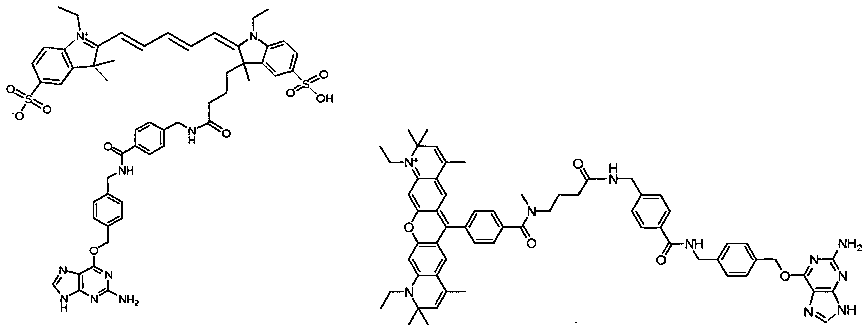

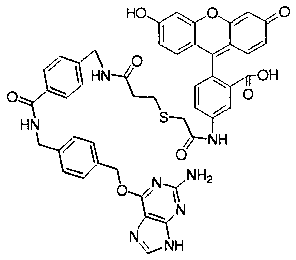

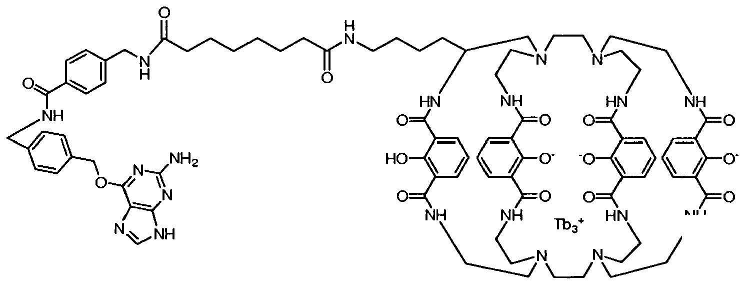

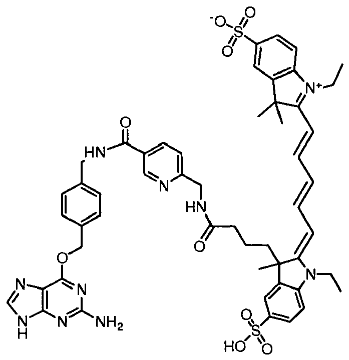

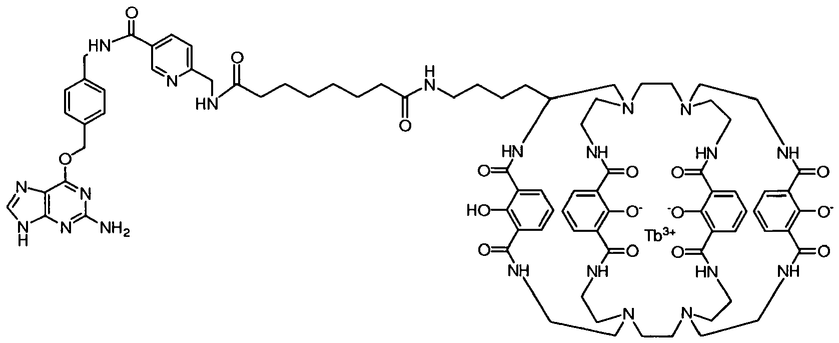

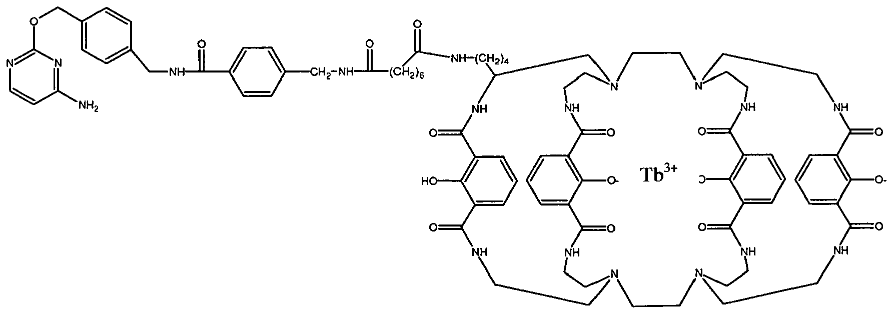

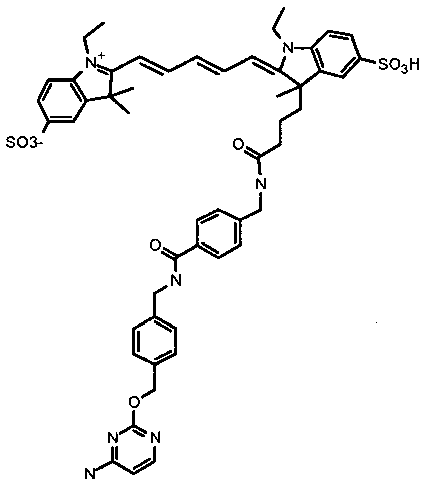

7. Composé selon l'une quelconque des revendications 1 à 4, **caractérisé en ce qu'**il répond à l'une des formules suivantes :

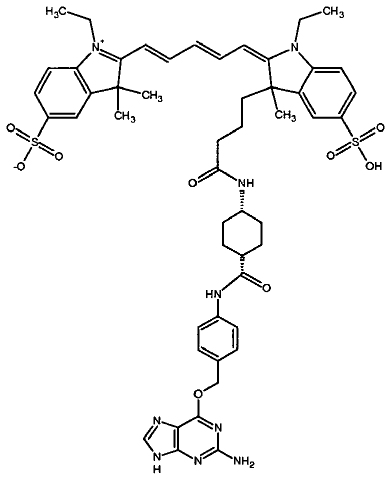

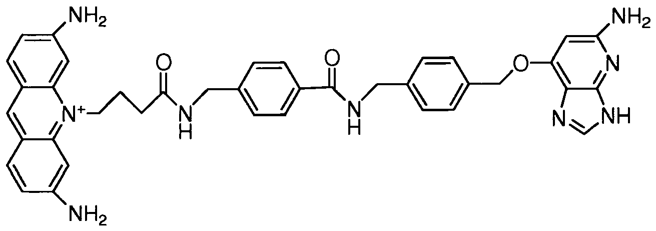

8. Méthode de marquage d'une protéine d'intérêt par un groupe M, ladite protéine d'intérêt étant exprimée sous forme de protéine de fusion avec une enzyme AGT, **caractérisée en ce qu'**elle comprend une étape de mise en contact de ladite protéine de fusion avec un composé selon l'une quelconque des revendications 1 à 4, 6 ou 7.

## Patentansprüche

1. Verbindung der Formel (I) 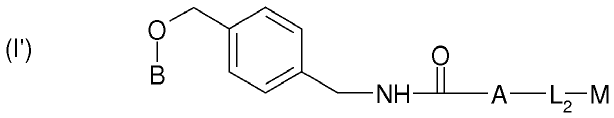 worin:
B eine Gruppe der folgenden Formel (II') oder (III') darstellt: 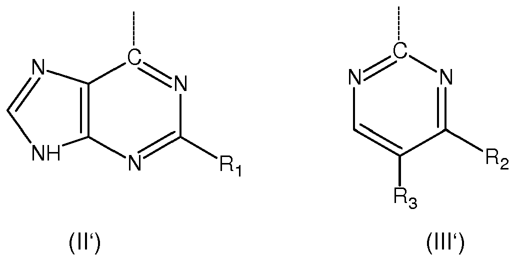 worin die gestrichelten Linien die Verbindungen mit dem Sauerstoff der Formel (I) darstellen,
R₁ ausgewählt ist aus: H, NH₂, einer OH-Gruppe oder einer Oxo-Gruppe,
R₂ ausgewählt ist aus: NH₂, OH oder Oxo,
R₃ ausgewählt ist aus: H oder einer CH₃-Gruppe;
L₂ aus den folgenden Gruppen ausgewählt ist: 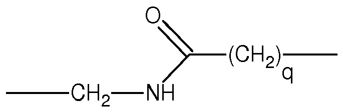 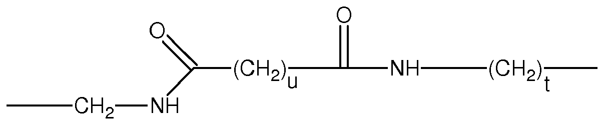 worin q, u, t ganze Zahlen zwischen 1 und 10 sind;
M eine lumineszierende Verbindung, ein Fluorophor oder ein reaktive Gruppe, ausgewählt aus den folgenden Gruppen ist: ein Acrylamid, ein aktiviertes Amin, ein aktivierter Ester, ein Aldehyd, ein Alkylhalogenid, ein Anhydrid, ein Anilin, ein Azid, ein Aziridin, eine Carbonsäure, Diazoalkan, ein Haloacetamid, ein Halotriazin, ein Hydrazin, ein Imidoester, ein Isocyanat, ein Isothiocyanat, ein Maleimid, ein Sulfonylhalogenid, oder ein Thiol, ein Keton, ein Amin, ein Säurehalogenid, ein Hydroxysuccinimidylester , ein Hydroxysulfosuccinimidylester, ein Azidonitrophenyl, ein Azidophenyl, ein 3-(2-Pyridyldithio)propionamid, Glyoxal, und insbesondere den Gruppen der Formel: 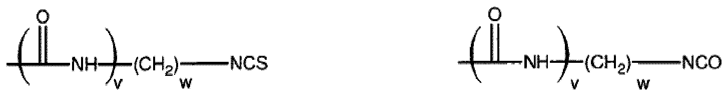 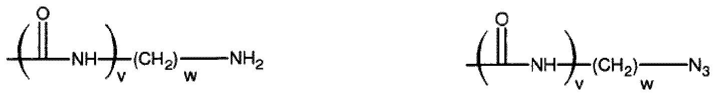 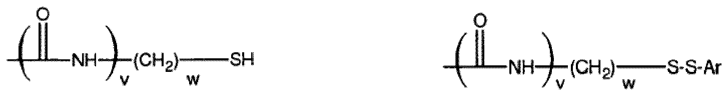 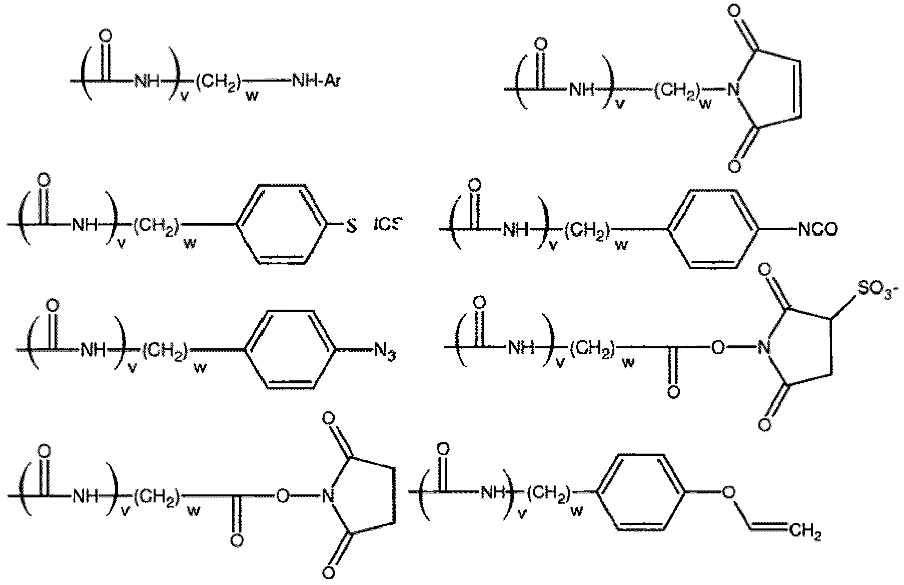 worin w von 0 bis 8 variiert und v gleich 0 oder 1 ist und Ar ein Heterocyclus mit 5 oder 6. durch gesättigte oder ungesättigte Verbindungen verbundenen Gliedern, umfassend 1 bis 3 Heteroatome, eventuell durch ein Halogenatom substituiert, ist;
A aus den folgenden zweiwertigen Gruppen ausgewählt ist:

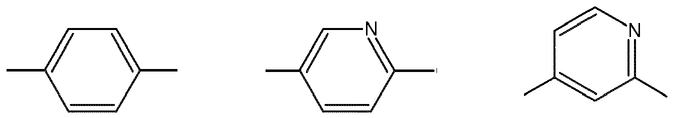

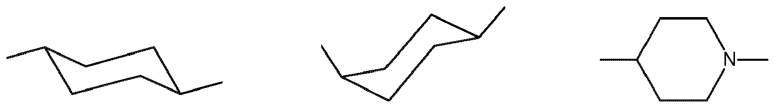

2. Verbindung nach Anspruch 1, **dadurch gekennzeichnet, daß** B eine Gruppe der Formel (11') ist und daß R₁ eine NH₂-Gruppe ist.

3. Verbindung nach Anspruch 1, **dadurch gekennzeichnet, daß** B eine Gruppe der Formel (III') ist und daß R₂ eine NH₂-Gruppe ist und R₃ H ist.

4. Verbindung nach einem der Ansprüche 1 bis 3, **dadurch gekennzeichnet, daß** M aus den folgenden lumineszierenden Verbindungen oder Fluorophoren ausgewählt ist:
- den organischen Fluorophoren, wie Cyaninen und ihren Derivaten, Fluorescein und seinen Derivaten, Cumarin und seinen Derivaten, Rhodamin und seinen Derivaten, Carbopyronin und seinen Derivaten, Oxazin und seinen Derivaten, AlexaFluors, Kristallviolett und seinen Derivaten, Perylenbisimid und seinen Derivaten, Squarainen, BODIPYs, NBD (Nitrobenzoxadiazol) und seinen Derivaten, DABCYL (4-((4-(Dimethylamino)phenyl)azo)benzoesäure und ihren Derivaten;
- den Proteinfluorophoren, wie GFP und seinen Varianten, den aus Korallen extrahierten fluoreszierenden Proteinen, Phycobiliproteinen, wie B-Phycoerythrin, R-Phycoerythrin, C-Phycocyanin, Allophycocyaninen, insbesondere das unter der Bezeichnung XL665 bekannte;
- den fluoreszierenden Lanthanid-Komplexen, wie Lanthanid-Kryptaten, Lanthanid-Chelaten (insbesondere Europium-, Terbium-, Samarium-, Dysprosium-, Neodymium-Chelaten und -Kryptaten).

5. Verbindung nach einem der Ansprüche 1 bis 4, **dadurch gekennzeichnet, daß** M eine reaktive Gruppe, ausgewählt aus den folgenden Gruppen ist: eine Carbonsäure, ein Amin, ein Carbonsäuresuccinimidylester, ein Haloacetamid, ein Hydrazin, ein Isothiocyanat, eine Maleimidgruppe, ein aliphatisches Amin.

6. Verbindung nach einem der Ansprüche 1 bis 4, **dadurch gekennzeichnet, daß** sie einer der folgenden Formeln entspricht:

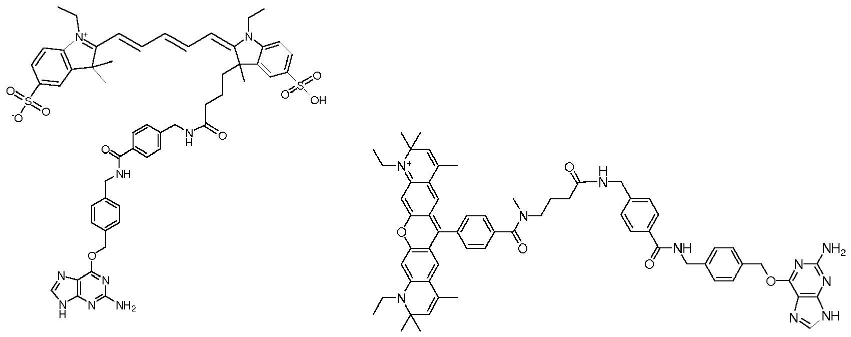

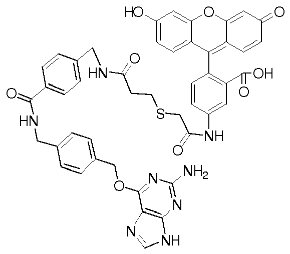

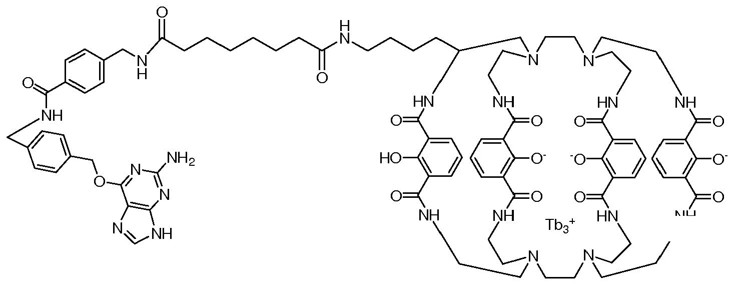

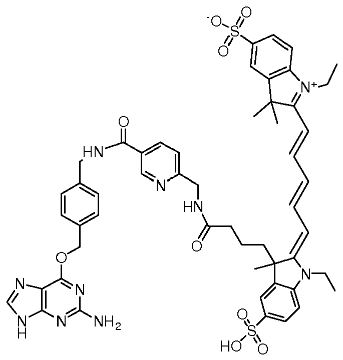

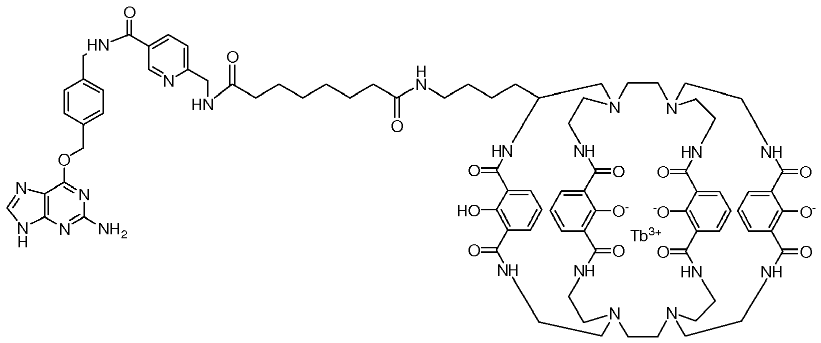

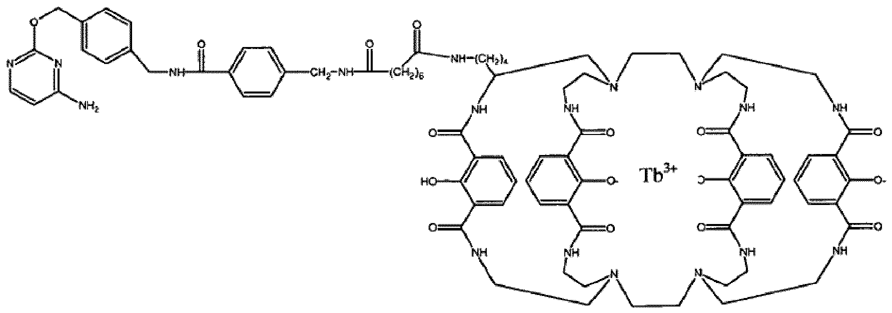

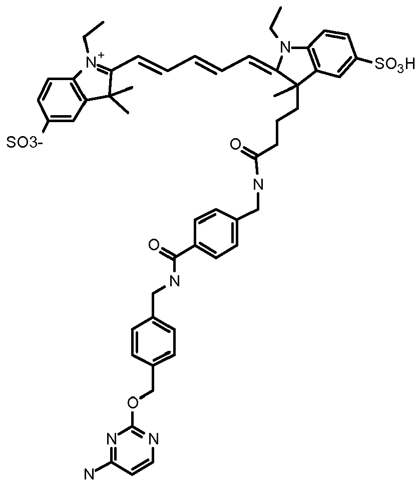

7. Verbindung nach einem der Ansprüche 1 bis 4, **dadurch gekennzeichnet, daß** sie einer der folgenden Formeln entspricht:

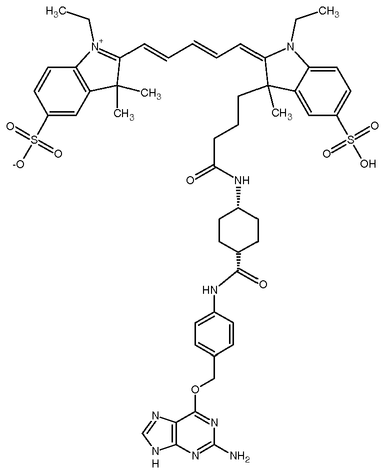

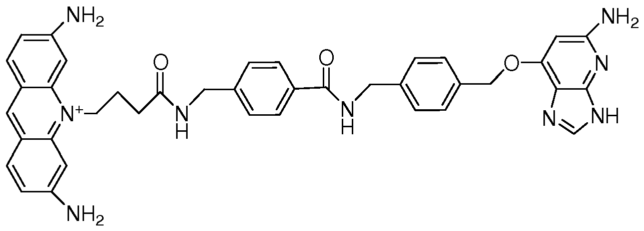

8. Verfahren zur Markierung eines Proteins von Interesse mit einer Gruppe M, wobei das Protein von Interesse in Form eines Fusionsproteins mit einem Enzym AGT exprimiert ist, **dadurch gekennzeichnet, daß** es einen Schritt zum Kontaktieren des Fusionsproteins mit einer Verbindung nach einem der Ansprüche 1 bis 4, 6 oder 7 umfaßt.

## Claims

1. A compound of formula (I'): 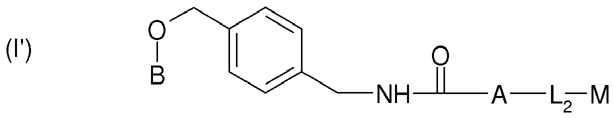 in which:
B represents a group of the following formula (II') or (III'): 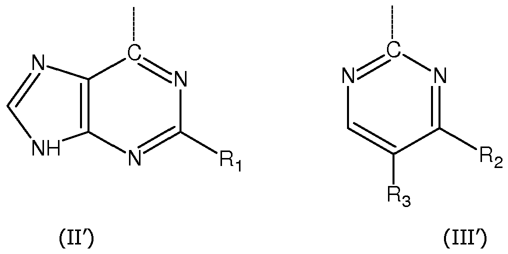 in which the dotted lines represent the bonds with the oxygen of the formula (I'),
R₁ is chosen from: H, NH₂, an OH group or an oxo group,
R₂ is chosen from: NH₂, OH or oxo,
R₃ is chosen from: H or a CH₃ group;
L₂ is chosen from the following groups: 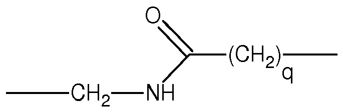 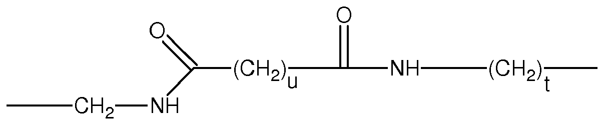 in which q, u, t are integers between 1 and 10;
M is a luminescent compound, a fluorophore or a reactive group chosen from the following groups: an acrylamide, an activated amine, an activated ester, an aldehyde, an alkyl halide, an anhydride, an aniline, an azide, an aziridine, a carboxylic acid, a diazoalkane, a haloacetamide, a halotriazine, a hydrazine, an imido ester, an isocyanate, an isothiocyanate, a maleimide, a sulfonyl halide, or a thiol, a ketone, an amine, an acid halide, a hydroxysuccinimidyl ester, a hydroxysulfosuccinimidyl ester, an azidonitrophenyl, an azidophenyl, a 3-(2-pyridyldithio)-propionamide, glyoxal and in particular the groups of formula: 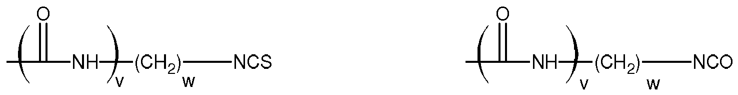 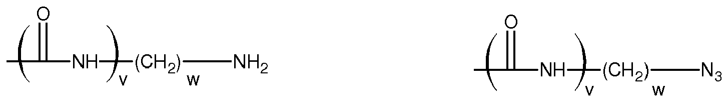 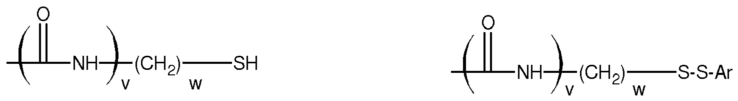 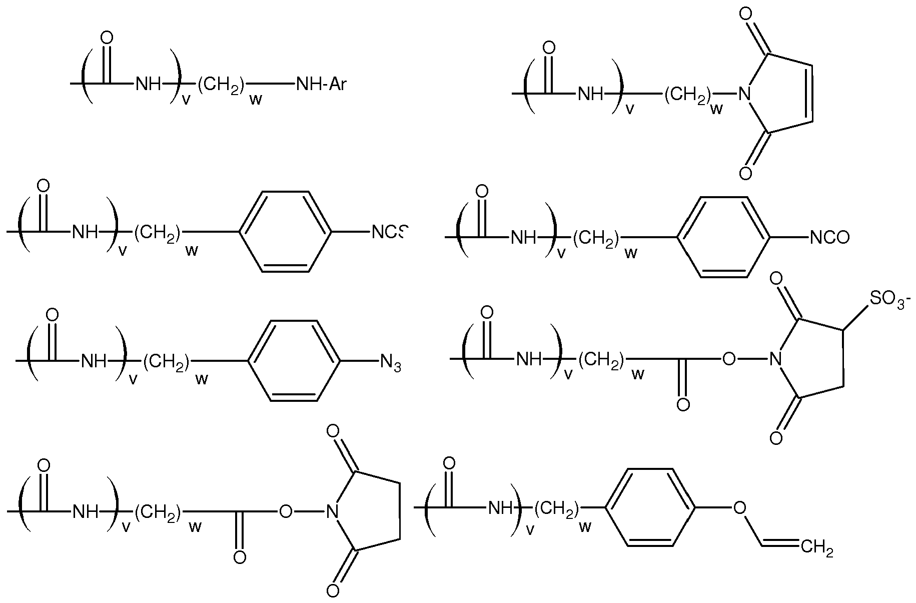 in which w varies from 0 to 8 and v is equal to 0 or 1, and Ar is a heterocycle having 5 or 6 ring members bonded by saturated or unsaturated bonds, comprising 1 to 3 heteroatoms, optionally substituted by a halogen atom;
A is chosen from the following divalent groups:

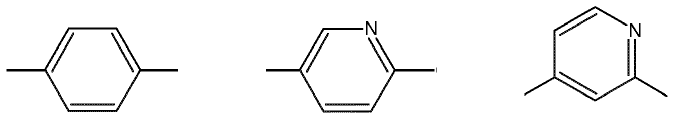

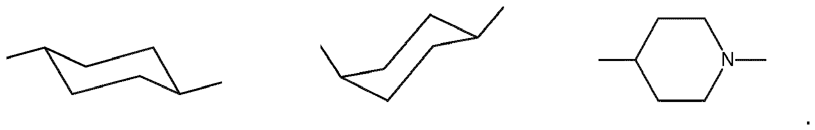

2. The compound as claimed in claim 1, **characterized in that** B is a group of formula (II') and **in that** R₁ is an NH₂ group.

3. The compound as claimed in claim 1, **characterized in that** B is a group of formula (III') and **in that** R₂ is an NH₂ group and R₃ is H.

4. The compound as claimed in claim 8, **characterized in that** M is chosen from the following luminescent compounds or fluorophores:
- *organic fluorophores* such as cyanines and their derivatives, fluorescein and its derivatives, coumarin and its derivatives, rhodamine and its derivatives, carbopyronine and its derivatives, oxazine and its derivatives, AlexaFluors, Crystal Violet and its derivatives, perylene bisimide and its derivatives, squaraines, BODIPYs, NBD (nitrobenzoxadiazole) and its derivatives, DABCYL (4-((4-(dimethylamino)-phenyl)azo)benzoic acid) and its derivatives;
- *protein fluorophores* such as GFP and its variants, fluorescent proteins extracted from corals, phycobiliproteins, such as B-phycoerythrin, R-phycoerythrin, C-phycocyanin, allophycocyanins, in particular those known under the name XL665;
- *fluorescent lanthanide complexes* such as lanthanide cryptates, lanthanide chelates (in particular the chelates and cryptates of europium, terbium, samarium, dysprosium, neodymium).

5. The compound as claimed in any one of claims 1 to 4, **characterized in that** M is a reactive group chosen from the following groups: a carboxylic acid, an amine, a succinimidyl ester of carboxylic acid, a haloacetamide, a hydrazine, an isothiocyanate, a maleimide group, an aliphatic amine.

6. The compound as claimed in any one of claims 1 to 4, **characterized in that** it has one of the following formulae:

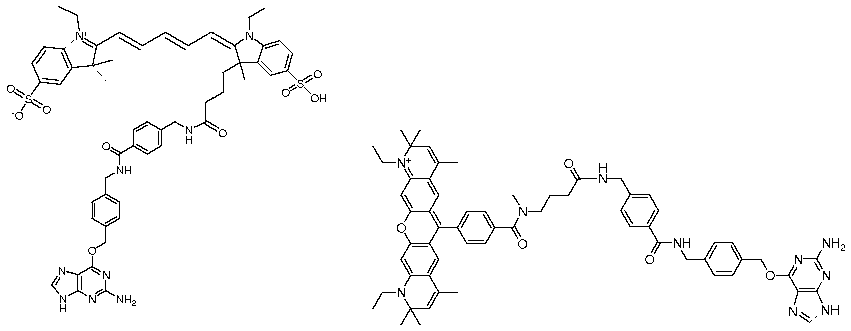

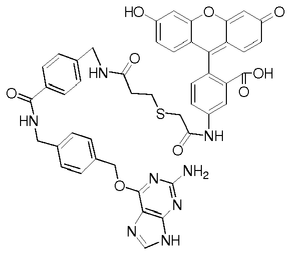

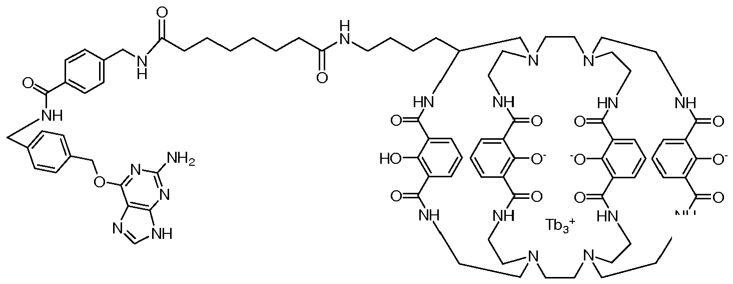

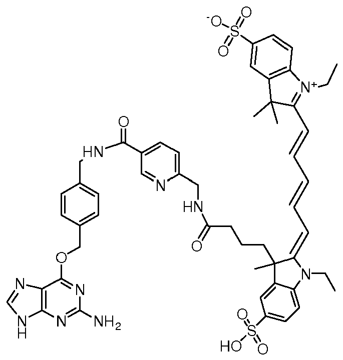

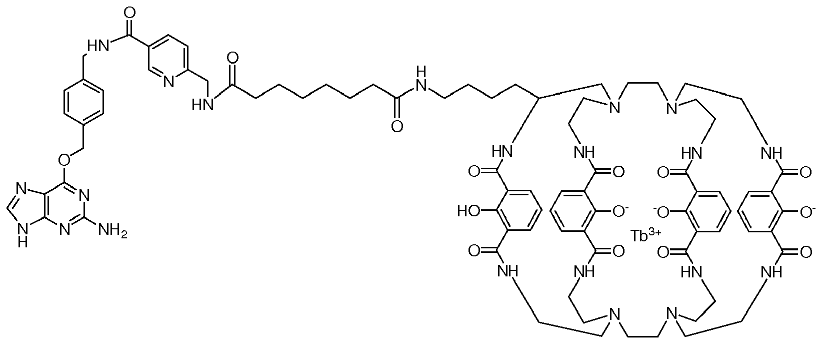

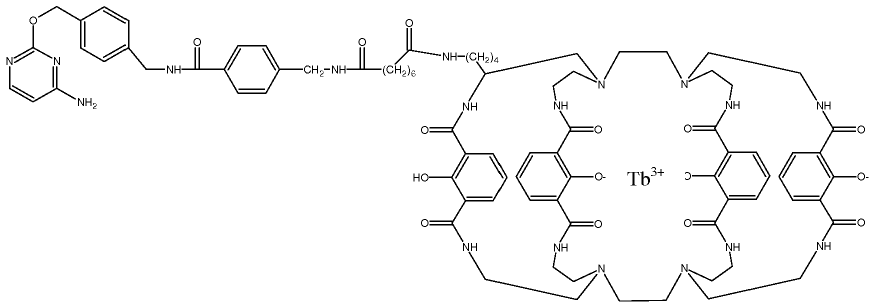

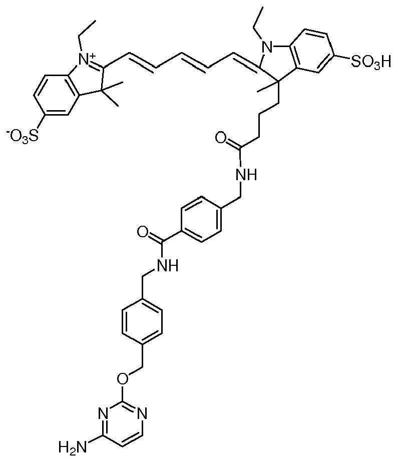

7. The compound as claimed in any one of claims 1 to 4, **characterized in that** it has one of the following formulae:

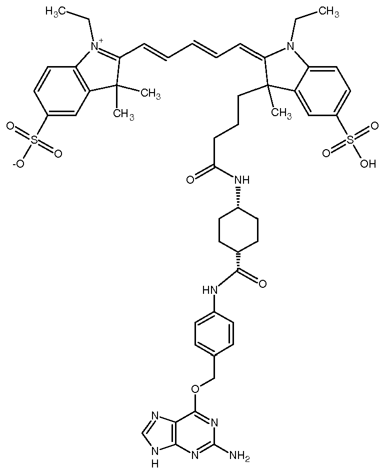

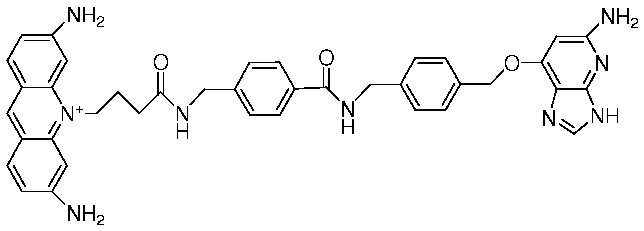

8. A method of labeling a protein of interest with a group M, said protein of interest being expressed in the form of a fusion protein with an AGT enzyme, **characterized in that** it comprises a step of bringing said fusion protein into contact with a compound as claimed in any one of claims 1 to 4 , 6 or 7.
